# EUROPEAN PATENT SPECIFICATION

(11) **EP 1 212 366 B1**
(45) Date of publication and mention of the grant of the patent: **12.09.2007**
(21) Application number: 00948934.5
(22) Date of filing: 25.07.2000
(51) Int. Cl.: C07K 19/00, A61K 39/395, A61P 35/00, C07K 16/28, C07K 14/485

(54) **THERAPEUTIC COMPOUNDS COMPRISED OF ANTI-FC RECEPTOR BINDING AGENTS**
THERAPEUTISCHE VERBINDUNGEN BESTEHEND AUS FC REZEPTOR BINDENDEN WIRKSTOFFEN
COMPOSES THERAPEUTIQUES CONSTITUES D'AGENTS DE LIAISON DU RECEPTEUR ANTI FC

(30) Priority: 30.07.1999 US 364088; 10.03.2000 US 523279
(43) Date of publication of application: 12.06.2002
(73) Proprietor: MEDAREX, INC., Annandale, NJ 08801-0953 (US)
(72) Inventor: DEO, Yashwant, M., Audubon, PA 19403 (US); GOLDSTEIN, Joel, Piscataway, NJ 08854 (US); GRAZIANO, Robert, Frenchtown, NJ 08825 (US); KELER, Tibor, Ottsville, PA 18942 (US)
(74) Representative: Lock, Graham James
(86) International application number: PCT/US2000/020158
(87) International publication number: WO 2001/009186

(56) References cited:
- WO-A-91/05805
- WO-A-98/02463
- WO-A-98/23646
- DEO Y M ET AL: "FCALPHAR DIRECTED BISPECIFIC MOLECULES (BSM) MEDIATE LYSIS AND PHAGOCYTOSIS OF TUMOR CELLS" PROCEEDINGS OF THE ANNUAL MEETING OF THE AMERICAN ASSOCIATION FOR CANCER RESEARCH,US,PHILADELPHIA, AACR, vol. 38, 1 March 1997 (1997-03-01), page 30 XP002046043
- ELSAESSER D ET AL: "Preclinical studies combining bispecific antibodies with cytokine-stimulated effector cells for immunotherapy of renal cell carcinoma." ANTICANCER RESEARCH, vol. 19, no. 2C, March 1999 (1999-03), pages 1525-1528, XP000944311 ISSN: 0250-7005
- WEINER L M ET AL: "PHASE I TRIAL OF 2B1, A BISPECIFIC MONOCLONAL ANTIBODY TARGETING C-ERBB-2 AND FCGAMMARIII" CANCER RESEARCH,US,AMERICAN ASSOCIATION FOR CANCER RESEARCH, BALTIMORE, MD, vol. 55, no. 20, 15 October 1995 (1995-10-15), pages 4586-4593, XP002046040 ISSN: 0008-5472
- J POSEY ET AL: "PILOT TRIAL OF MDX-H210 AND GM-CSF FOR PATIENTS WITH ADVANCED ERB-2POSITIVE MALIGNANCIES" PROCEEDINGS OF THE ANNUAL MEETING OF THE AMERICAN ASSOCIATION FOR CANCER RESEARCH,US,PHILADELPHIA, AACR, vol. MEETING 87, 20 April 1996 (1996-04-20), page 165 XP002066249 ISSN: 0197-016X
- JELLEY-GIBBS D M ET AL: "Differences in IgG subclass do not effect immune complex-enhanced T cell activation despite differential binding to antigen presenting cells." HUMAN IMMUNOLOGY, vol. 60, no. 6, June 1999 (1999-06), pages 469-478, XP000983996 ISSN: 0198-8859
- LIU CHUNLEI ET AL: "Fc-gamma-RII on human B cells can mediate enhanced antigen presentation." CELLULAR IMMUNOLOGY, vol. 167, no. 2, 1996, pages 188-194, XP000983982 ISSN: 0008-8749
- GUYRE ET AL: "Increased potency Fc-receptor-targeted antigens" CANCER IMMUNOLOGY AND IMMUNOTHERAPY,DE,BERLIN, vol. 45, no. 3/04, 1997, pages 146-148, XP002081336 ISSN: 0340-7004
- HEIJNEN I.A.F.M. ET AL.: "Antigen targeting to myeloid-specific human Fc-gamma-RI-CD64 triggers enhanced antibody responses in transgenic mice." JOURNAL OF CLINICAL INVESTIGATION,US,NEW YORK, NY, vol. 97, no. 2, 1996, pages 331-338, XP002081335 ISSN: 0021-9738
- GOSSELIN EDMUND J ET AL: "Enhanced antigen presentation using human Fc-gamma receptor (monocyte/macrophage)-specific immunogens." JOURNAL OF IMMUNOLOGY, vol. 149, no. 11, 1992, pages 3477-3481, XP000942182 ISSN: 0022-1767
- LIU CHUNLEI ET AL: "Fc-gamma-RI-targeted fusion proteins results in efficient presentation by human monocytes of antigenic and antagonist T cell epitopes." JOURNAL OF CLINICAL INVESTIGATION, vol. 98, no. 9, 1996, pages 2001-2007, XP002131533 ISSN: 0021-9738
- MORTON H C ET AL: "STRUCTURE AND FUNCTION OF HUMAN IGA FC RECEPTORS (FCALPHAR)" CRITICAL REVIEWS IN IMMUNOLOGY,XX,CRC PRESS, INC, vol. 16, 1996, pages 423-440, XP002066873 ISSN: 1040-8401

### SEQUENCE LISTING

<110> Medarex, Inc. et al.
<120> THERAPEUTIC COMPOUNDS COMPRISES OF ANTI-Fc RECEPTOR BINDING AGENTS
<130> MXI-043CP3PC
<140> 09/523,279
   <141> 2000-03-10
<150> 09/369,088
   <151> 1999-07-30
<160> 19
<170> PatentIn Ver. 2.0
<210> 1
   <211> 24
   <212> DNA
   <213> Mus musculus
<220>
   <221> CDS
   <222> (1)..(24)
<400> 1
   act cac aca tgc cca ccg tgc cca                                     24
   Thr His Thr Cys Pro Pro Cys Pro
     1               5
<210> 2
   <211> 27
   <212> DNA
   <213> Mus musculus
<220>
   <221> CDS
   <222> (1)..(18)
<400> 2
   act cac aca tgc cca ccg tgaggatcc                                   27
   Thr His Thr Cys Pro Pro
     1               5
<210> 3
   <211> 42
   <212> DNA
   <213> Mus musculus
<220>
   <221> CDS
   <222> (1)..(33)
<400> 3
   act cac aca tgc tcg agc ctt cac ggc ggc cgc tgaggatcc               42
   Thr His Thr Cys Ser Ser Leu His Gly Gly Arg
     1               5                  10
<210> 4
   <211> 300
   <212> PRT
   <213> Mus musculus
<400> 4
   Glu Val Gln Leu Val Glu Ser Gly Gly Gly Val Val Gln Pro Gly Arg
   1               5                   10                  15
   Ser Leu Arg Leu Ser Cys Ser Ser Ser Gly Phe Ile Phe Ser Asp Asn
               20                  25                  30
   Tyr Met Tyr Trp Val Arg Gln Ala Pro Gly Lys Gly Leu Glu Trp Val
           35                  40                  45
   Ala Thr Ile Ser Asp Gly Gly Ser Tyr Thr Tyr Tyr Pro Asp Ser Val
       50                  55                  60
   Lys Gly Arg Phe Thr Ile Ser Arg Asp Asn Ser Lys Asn Thr Leu Phe
   65                  70                  75                  80
   Leu Gln Met Asp Ser Leu Arg Pro Glu Asp Thr Gly Val Tyr Phe Cys
                   85                  90                  95
   Ala Arg Gly Tyr Tyr Arg Tyr Glu Gly Ala Met Asp Tyr Trp Gly Gln
               100                 105                 110
   Gly Thr Pro Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Arg Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Ser Thr Thr Ser Thr Thr Gly Thr Ser His Leu
   225                 230                 235                 240
   Val Lys Cys Ala Glu Lys Glu Lys Thr Phe Cys Val Asn Gly Gly Glu
                   245                 250                 255
   Cys Phe Met Val Lys Asp Leu Ser Asn Pro Ser Arg Tyr Leu Cys Lys
   260 265 270
   Cys Pro Asn Glu Phe Thr Gly Asp Arg Cys Gln Asn Tyr Val Met Ala
   275 280 285
   Ser Phe Tyr Lys Ala Glu Glu Leu Tyr Gln Lys Arg
   290 295 300
<210> 5
   <211> 14
   <212> PRT
   <213> Mus musculus
<400> 5
   Gln Arg Leu Gly Asn Gln Trp Ala Val Gly His Leu Met Gly
   1 5 10
<210> 6
   <211> 15
   <212> PRT
   <213> Mus musculus
<400> 6
   Gln Tyr Ile Lys Ala Asn Ser Lys Phe Ile Gly Ile Thr Glu Leu
   1 5 10 15
<210> 7
   <211> 54
   <212> DNA
   <213> Mus musculus
<400> 7
   tcgagccagt acatcaaggc gaattccaag ttcatcggca tcaccgagct ctga 54
<210> 8
   <211> 53
   <212> DNA
   <213> Mus musculus
<400> 8
   cggtcatgta gttccgctta aggttcaagt agccgtagtg gctcgagact ccg 53
<210> 9
   <211> 15
   <212> PRT
   <213> Mus musculus
<400> 9
   Gln Tyr Ile Ser Ala Asn Ser Lys Phe Ile Gly Ile Thr Glu Leu
   1 5 10 15
<210> 10
   <211> 54
   <212> DNA
   <213> Mus musculus
<400> 10
   tcgagccagt acatcagcgc gaattccaag ttcatcggca tcaccgagct ctga 54
<210> 11
   <211> 53
   <212> DNA
   <213> Mus musculus
<400> 11
   cggtcatgta gtcgcgctta aggttcaagt agccgtagtg gctcgagact ccg 53
<210> 12
   <211> 21
   <212> PRT
   <213> Mus musculus
<400> 12
   Phe Asn Asn Phe Thr Val Ser Phe Trp Leu Arg Val Pro Lys Val Ser
   1 5 10 15
   Ala Ser His Leu Glu
   20
<210> 13
   <211> 913
   <212> DNA
   <213> Mus musculus
<220>
   <221> CDS
   <222> (11)..(913)
<400> 13
   aagcttcacc atg gga tgg agc tgt atc atc ctc ttc ttg gtg gcc aca    49
              Met Gly Trp Ser Cys Ile Ile Leu Phe Leu Val Ala Thr
                1               5                  10
   gct acc ggt gtc cac tcc gat atc caa ctg gtg gag agc ggt gga ggt   97
   Ala Thr Gly Val His Ser Asp Ile Gln Leu Val Glu Ser Gly Gly Gly
        15                  20                  25
   gtt gtg caa cct ggc cgg tcc ctg cgc ctg tcc tgc tcc tcg tct ggc   145
   Val Val Gln Pro Gly Arg Ser Leu Arg Leu Ser Cys Ser Ser Ser Gly
    30                  35                  40                  45
   ttc agt ttc agt gac aat tac atg tat tgg gtg aga cag gca cct gga   193
   Phe Ser Phe Ser Asp Asn Tyr Met Tyr Trp Val Arg Gln Ala Pro Gly
                    50                  55                  60
   aaa ggt ctt gag tgg gtt gca acc att agt gat ggt ggt agt tac acc   241
   Lys Gly Leu Glu Trp Val Ala Thr Ile Ser Asp Gly Gly Ser Tyr Thr
                   65                  70                  75
   tac tat cca gac agt gtg aag gga aga ttt aca ata tcg aga gac aac    289
   Tyr Tyr Pro Asp Ser Val Lys Gly Arg Phe Thr Ile Ser Arg Asp Asn
            80                  85                  90
   agc aag aac aca ttg ttc ctg caa atg gac agc ctg aga ccc gaa gac    337
   Ser Lys Asn Thr Leu Phe Leu Gln Met Asp Ser Leu Arg Pro Glu Asp
        95                 100                 105
   acc ggg gtc tat ttt tgt gca aga ggc tac tat agg tac gag ggg gct    385
   Thr Gly Val Tyr Phe Cys Ala Arg Gly Tyr Tyr Arg Tyr Glu Gly Ala
   110                 115                 120                 125
   atg gac tac tgg ggc caa ggg acc ccg gtc acc gtg agc tca gga ggt    433
   Met Asp Tyr Trp Gly Gln Gly Thr Pro Val Thr Val Ser Ser Gly Gly
                   130                 135                 140
   ggc ggc tcc gga ggt gga ggc agc gga ggg ggc gga tcc gac atc cag    481
   Gly Gly Ser Gly Gly Gly Gly Ser Gly Gly Gly Gly Ser Asp Ile Gln
               145                 150                 155
   ctg acc cag agc cca agc agc ctg agc gcc agc gtg ggt gac aga gtg    529
   Leu Thr Gln Ser Pro Ser Ser Leu Ser Ala Ser Val Gly Asp Arg Val
           160                 165                 170
   acc atc acc tgt aag tcc agt caa agt gtt tta tac agt tca aat cag    577
   Thr Ile Thr Cys Lys Ser Ser Gln Ser Val Leu Tyr Ser Ser Asn Gln
       175                 180                 185
   aag aac tac ttg gcc tgg tac cag cag aag cca ggt aag gct cca aag    625
   Lys Asn Tyr Leu Ala Trp Tyr Gln Gln Lys Pro Gly Lys Ala Pro Lys
   190                 195                 200                 205
   ctg ctg atc tac tgg gca tcc act agg gaa tct ggt gtg cca agc aga    673
   Leu Leu Ile Tyr Trp Ala Ser Thr Arg Glu Ser Gly Val Pro Ser Arg
                   210                 215                 220
   ttc agc ggt agc ggt agc ggt acc gac ttc acc ttc acc atc agc agc    721
   Phe Ser Gly Ser Gly Ser Gly Thr Asp Phe Thr Phe Thr Ile Ser Ser
               225                 230                 235
   ctc cag cca gag gac atc gcc acc tac tac tgc cat caa tac ctc tcc    769
   Leu Gln Pro Glu Asp Ile Ala Thr Tyr Tyr Cys His Gln Tyr Leu Ser
           240                 245                 250
   tcg tgg acg ttc ggc caa ggg acc aag gtg gaa atc aaa tct agc tgc    817
   Ser Trp Thr Phe Gly Gln Gly Thr Lys Val Glu Ile Lys Ser Ser Cys
       255                 260                 265
   tcg agc gga ggc ggg ggt agc gat atc gcg gcc gca gaa cag aaa ctc    865
   Ser Ser Gly Gly Gly Gly Ser Asp Ile Ala Ala Ala Glu Gln Lys Leu
   270                 275                 280                 285
   atc tca gaa gag gat ctg aat ggc gcc gca cat cac cat cat cac cat    913
   Ile Ser Glu Glu Asp Leu Asn Gly Ala Ala His His His His His His
                   290                 295                 300
<210> 14
   <211> 301
   <212> PRT
   <213> Mus musculus
<400> 14
   Met Gly Trp Ser Cys Ile Ile Leu Phe Leu Val Ala Thr Ala Thr Gly
   1               5                   10                  15
   Val His Ser Asp Ile Gln Leu Val Glu Ser Gly Gly Gly Val Val Gln
               20                  25                  30
   Pro Gly Arg Ser Leu Arg Leu Ser Cys Ser Ser Ser Gly Phe Ser Phe
           35                  40                  45
   Ser Asp Asn Tyr Met Tyr Trp Val Arg Gln Ala Pro Gly Lys Gly Leu
       50                  55                  60
   Glu Trp Val Ala Thr Ile Ser Asp Gly Gly Ser Tyr Thr Tyr Tyr Pro
   65                  70                  75                  80
   Asp Ser Val Lys Gly Arg Phe Thr Ile Ser Arg Asp Asn Ser Lys Asn
                   85                  90                  95
   Thr Leu Phe Leu Gln Met Asp Ser Leu Arg Pro Glu Asp Thr Gly Val
               100                 105                 110
   Tyr Phe Cys Ala Arg Gly Tyr Tyr Arg Tyr Glu Gly Ala Met Asp Tyr
           115                 120                 125
   Trp Gly Gln Gly Thr Pro Val Thr Val Ser Ser Gly Gly Gly Gly Ser
       130                 135                 140
   Gly Gly Gly Gly Ser Gly Gly Gly Gly Ser Asp Ile Gln Leu Thr Gln
   145                 150                 155                 160
   Ser Pro Ser Ser Leu Ser Ala Ser Val Gly Asp Arg Val Thr Ile Thr
                   165                 170                 175
   Cys Lys Ser Ser Gln Ser Val Leu Tyr Ser Ser Asn Gln Lys Asn Tyr
               180                 185                 190
   Leu Ala Trp Tyr Gln Gln Lys Pro Gly Lys Ala Pro Lys Leu Leu Ile
           195                 200                 205
   Tyr Trp Ala Ser Thr Arg Glu Ser Gly Val Pro Ser Arg Phe Ser Gly
       210                 215                 220
   Ser Gly Ser Gly Thr Asp Phe Thr Phe Thr Ile Ser Ser Leu Gln Pro
   225                 230                 235                 240
   Glu Asp Ile Ala Thr Tyr Tyr Cys His Gln Tyr Leu Ser Ser Trp Thr
                   245                 250                 255
   Phe Gly Gln Gly Thr Lys Val Glu Ile Lys Ser Ser Cys Ser Ser Gly
               260                 265                 270
   Gly Gly Gly Ser Asp Ile Ala Ala Ala Glu Gln Lys Leu Ile Ser Glu
           275                 280                 285
   Glu Asp Leu Asn Gly Ala Ala His His His His His His
       290                 295                 300
<210> 15
   <211> 1679
   <212> DNA
   <213> Mus musculus -
<220>
   <221> CDS
   <222> (11)..(1669)
<400> 15
   aagcttcacc atg gga tgg agc tgt atc atc ctc ttc ttg gtg gcc aca      49
              Met Gly Trp Ser Cys Ile Ile Leu Phe Leu Val Ala Thr
                1               5                  10
   gct acc ggt gtc cac tcc gat atc caa ctg gtg gag agc ggt gga ggt     97
   Ala Thr Gly Val His Ser Asp Ile Gln Leu Val Glu Ser Gly Gly Gly
        15                  20                  25
   gtt gtg caa cct ggc cgg tcc ctg cgc ctg tcc tgc tcc tcg tct ggc    145
   Val Val Gln Pro Gly Arg Ser Leu Arg Leu Ser Cys Ser Ser Ser Gly
    30                  35                  40                  45
   ttc att ttc agt gac aat tac atg tat tgg gtg aga cag gca cct gga    193
   Phe Ile Phe Ser Asp Asn Tyr Met Tyr Trp Val Arg Gln Ala Pro Gly
                    50                  55                  60
   aaa ggt ctt gag tgg gtt gca acc att agt gat ggt ggt agt tac acc    241
   Lys Gly Leu Glu Trp Val Ala Thr Ile Ser Asp Gly Gly Ser Tyr Thr
                65                  70                  75
   tac tat cca gac agt gtg aag gga aga ttt aca ata tcg aga gac aac    289
   Tyr Tyr Pro Asp Ser Val Lys Gly Arg Phe Thr Ile Ser Arg Asp Asn
            80                  85                  90
   agc aag aac aca ttg ttc ctg caa atg gac agc ctg aga ccc gaa gac    337
   Ser Lys Asn Thr Leu Phe Leu Gln Met Asp Ser Leu Arg Pro Glu Asp
        95                 100                 105
   acc ggg gtc tat ttt tgt gca aga ggc tac tat agg tac gag ggg gct    385
   Thr Gly Val Tyr Phe Cys Ala Arg Gly Tyr Tyr Arg Tyr Glu Gly Ala
   110                 115                 120                 125
   atg gac tac tgg ggc caa ggg acc ccg gtc acc gtg agc tca gga ggt    433
   Met Asp Tyr Trp Gly Gln Gly Thr Pro Val Thr Val Ser Ser Gly Gly
                   130                 135                 140
   ggc ggc tcc gga ggt gga ggc agc gga ggg ggc gga tcc gac atc cag    481
   Gly Gly Ser Gly Gly Gly Gly Ser Gly Gly Gly Gly Ser Asp Ile Gln
               145                 150                 155
   ctg acc cag agc cca agc agc ctg agc gcc agc gtg ggt gac aga gtg    529
   Leu Thr Gln Ser Pro Ser Ser Leu Ser Ala Ser Val Gly Asp Arg Val
           160                 165                 170
   acc atc acc tgt aag tcc agt caa agt gtt tta tac agt tca aat cag     577
   Thr Ile Thr Cys Lys Ser Ser Gln Ser Val Leu Tyr Ser Ser Asn Gln
       175                 180                     185
   aag aac tac ttg gcc tgg tac cag cag aag cca ggt aag gct cca aag     625
   Lys Asn Tyr Leu Ala Trp Tyr Gln Gln Lys Pro Gly Lys Ala Pro Lys
   190                 195                     200                 205
   ctg ctg atc tac tgg gca tcc act agg gaa tct ggt gtg cca agc aga     673
   Leu Leu Ile Tyr Trp Ala Ser Thr Arg Glu Ser Gly Val Pro Ser Arg
                   210                     215                 220
   ttc agc ggt agc ggt agc ggt acc gac ttc acc ttc acc atc agc agc     721
   Phe Ser Gly Ser Gly Ser Gly Thr Asp Phe Thr Phe Thr Ile Ser Ser
               225                     230                     235
   ctc cag cca gag gac atc gcc acc tac tac tgc cat caa tac ctc tcc     769
   Leu Gln Pro Glu Asp Ile Ala Thr Tyr Tyr Cys His Gln Tyr Leu Ser
           240                     245                     250
   tcg tgg acg ttc ggc caa ggg acc aag gtg gaa atc aaa tct agc tgc     817
   Ser Trp Thr Phe Gly Gln Gly Thr Lys Val Glu Ile Lys Ser Ser Cys
       255                     260                     265
   tcg agc gga ggc ggg ggt agc gat atc aaa ctg cag cag tct ggg gca     865
   Ser Ser Gly Gly Gly Gly Ser Asp Ile Lys Leu Gln Gln Ser Gly Ala
   270                 275                     280                 285
   gaa ctt gtg agg tca ggg acc tca gtc aag ttg tcc tgc aca gct tct     913
   Glu Leu Val Arg Ser Gly Thr Ser Val Lys Leu Ser Cys Thr Ala Ser
                   290                     295                 300
   ggc ttc aac att aaa gac tcc tat atg cac tgg ttg agg cag ggg cct     961
   Gly Phe Asn Ile Lys Asp Ser Tyr Met His Trp Leu Arg Gln Gly Pro
               305                     310                     315
   gaa cag ggc ctg gag tgg att gga tgg att gat cct gag aat ggt gat     1009
   Glu Gln Gly Leu Glu Trp Ile Gly Trp Ile Asp Pro Glu Asn Gly Asp
           320                     325                     330
   act gaa tat gcc ccg aag ttc cag ggc aag gcc act ttt act aca gac     1057
   Thr Glu Tyr Ala Pro Lys Phe Gln Gly Lys Ala Thr Phe Thr Thr Asp
       335                     340                     345
   aca tcc tcc aac aca gcc tac ctg cag ctg agc agc ctg aca tct gag     1105
   Thr Ser Ser Asn Thr Ala Tyr Leu Gln Leu Ser Ser Leu Thr Ser Glu
   350                 355                     360                 365
   gac act gcc gtc tat tat tgt aat gag ggg act ccg act ggg ccg tac     1153
   Asp Thr Ala Val Tyr Tyr Cys Asn Glu Gly Thr Pro Thr Gly Pro Tyr
                   370                     375                 380
   tac ttt gac tac tgg ggc caa ggg acc acg gtc acc gtc tcc tca ggt     1201
   Tyr Phe Asp Tyr Trp Gly Gln Gly Thr Thr Val Thr Val Ser Ser Gly
               385                     390                     395
   gga ggc ggt tca ggc gga ggt ggc tct ggc ggt ggc gga tca gaa aat     1249
   Gly Gly Gly Ser Gly Gly Gly Gly Ser Gly Gly Gly Gly Ser Glu Asn
           400                     405                     410
   gtg ctc acc cag tct cca gca atc atg tct gca tct cca ggg gag aag     1297
   Val Leu Thr Gln Ser Pro Ala Ile Met Ser Ala Ser Pro Gly Glu Lys
       415                 420                 425
   gtc acc ata acc tgc agt gcc agc tca agt gta agt tac atg cac tgg     1345
   Val Thr Ile Thr Cys Ser Ala Ser Ser Ser Val Ser Tyr Met His Trp
   430                 435                 440                 445
   ttc cag cag aag cca ggc act tct ccc aaa ctc tgg att tat agc aca     1393
   Phe Gln Gln Lys Pro Gly Thr Ser Pro Lys Leu Trp Ile Tyr Ser Thr
                   450                 455                 460
   tcc aac ctg gct tct gga gtc cct gct cgc ttc agt ggc agt gga tct     1441
   Ser Asn Leu Ala Ser Gly Val Pro Ala Arg Phe Ser Gly Ser Gly Ser
               465                 470                 475
   ggg acc tct tac tct ctc aca atc agc cga atg gag gct gaa gat gct     1489
   Gly Thr Ser Tyr Ser Leu Thr Ile Ser Arg Met Glu Ala Glu Asp Ala
           480                 485                 490
   gcc act tat tac tgc cag caa cgg agt agt tac cca ctc acg ttc ggt     1537
   Ala Thr Tyr Tyr Cys Gln Gln Arg Ser Ser Tyr Pro Leu Thr Phe Gly
       495                 500                 505
   gct ggc acc aag ctg gag ctg aaa cgg gcg gca ggc tcg agc gga ggc     1585
   Ala Gly Thr Lys Leu Glu Leu Lys Arg Ala Ala Gly Ser Ser Gly Gly
   510                 515                 520                 525
   ggg ggt agc gat atc gcg gcc gca gaa cag aaa ctc atc tca gaa gag     1633
   Gly Gly Ser Asp Ile Ala Ala Ala Glu Gln Lys Leu Ile Ser Glu Glu
                   530                 535                 540
   gat ctg aat ggc gcc gca cat cac cat cat cac cat tgattctaga          1679
   Asp Leu Asn Gly Ala Ala His His His His His His
               545                 550
<210> 16
   <211> 553
   <212> PRT
   <213> Mus musculus
<400> 16
   Met Gly Trp Ser Cys Ile Ile Leu Phe Leu Val Ala Thr Ala Thr Gly
     1               5                  10                  15
   Val His Ser Asp Ile Gln Leu Val Glu Ser Gly Gly Gly Val Val Gln
               20                  25                  30
   Pro Gly Arg Ser Leu Arg Leu Ser Cys Ser Ser Ser Gly Phe Ile Phe
           35                  40                  45
   Ser Asp Asn Tyr Met Tyr Trp Val Arg Gln Ala Pro Gly Lys Gly Leu
       50                  55                  60
   Glu Trp Val Ala Thr Ile Ser Asp Gly Gly Ser Tyr Thr Tyr Tyr Pro
    65                  70                  75                  80
   Asp Ser Val Lys Gly Arg Phe Thr Ile Ser Arg Asp Asn Ser Lys Asn
   85 90 95
   Thr Leu Phe Leu Gln Met Asp Ser Leu Arg Pro Glu Asp Thr Gly Val
   100 105 110
   Tyr Phe Cys Ala Arg Gly Tyr Tyr Arg Tyr Glu Gly Ala Met Asp Tyr
   115 120 125
   Trp Gly Gln Gly Thr Pro Val Thr Val Ser Ser Gly Gly Gly Gly Ser
   130 135 140
   Gly Gly Gly Gly Ser Gly Gly Gly Gly Ser Asp Ile Gln Leu Thr Gln
   145 150 155 160
   Ser Pro Ser Ser Leu Ser Ala Ser Val Gly Asp Arg Val Thr Ile Thr
   165 170 175
   Cys Lys Ser Ser Gln Ser Val Leu Tyr Ser Ser Asn Gln Lys Asn Tyr
   180 185 190
   Leu Ala Trp Tyr Gln Gln Lys Pro Gly Lys Ala Pro Lys Leu Leu Ile
   195 200 205
   Tyr Trp Ala Ser Thr Arg Glu Ser Gly Val Pro Ser Arg Phe Ser Gly
   210 215 220
   Ser Gly Ser Gly Thr Asp Phe Thr Phe Thr Ile Ser Ser Leu Gln Pro
   225 230 235 240
   Glu Asp Ile Ala Thr Tyr Tyr Cys His Gln Tyr Leu Ser Ser Trp Thr
   245 250 255
   Phe Gly Gln Gly Thr Lys Val Glu Ile Lys Ser Ser Cys Ser Ser Gly
   260 265 270
   Gly Gly Gly Ser Asp Ile Lys Leu Gln Gln Ser Gly Ala Glu Leu Val
   275 280 285
   Arg Ser Gly Thr Ser Val Lys Leu Ser Cys Thr Ala Ser Gly Phe Asn
   290 295 300
   Ile Lys Asp Ser Tyr Met His Trp Leu Arg Gln Gly Pro Glu Gln Gly
   305 310 315 320
   Leu Glu Trp Ile Gly Trp Ile Asp Pro Glu Asn Gly Asp Thr Glu Tyr
   325 330 335
   Ala Pro Lys Phe Gln Gly Lys Ala Thr Phe Thr Thr Asp Thr Ser Ser
   340 345 350
   Asn Thr Ala Tyr Leu Gln Leu Ser Ser Leu Thr Ser Glu Asp Thr Ala
   355 360 365
   Val Tyr Tyr Cys Asn Glu Gly Thr Pro Thr Gly Pro Tyr Tyr Phe Asp
   370 375 380
   Tyr Trp Gly Gln Gly Thr Thr Val Thr Val Ser Ser Gly Gly Gly Gly
   385 390 395 400
   Ser Gly Gly Gly Gly Ser Gly Gly Gly Gly Ser Glu Asn Val Leu Thr
                   405                 410                 415
   Gln Ser Pro Ala Ile Met Ser Ala Ser Pro Gly Glu Lys Val Thr Ile
               420                 425                 430
   Thr Cys Ser Ala Ser Ser Ser Val Ser Tyr Met His Trp Phe Gln Gln
           435                 440                 445
   Lys Pro Gly Thr Ser Pro Lys Leu Trp Ile Tyr Ser Thr Ser Asn Leu
       450                 455                 460
   Ala Ser Gly Val Pro Ala Arg Phe Ser Gly Ser Gly Ser Gly Thr Ser
   465                 470                 475                 480
   Tyr Ser Leu Thr Ile Ser Arg Met Glu Ala Glu Asp Ala Ala Thr Tyr
                   485                 490                 495
   Tyr Cys Gln Gln Arg Ser Ser Tyr Pro Leu Thr Phe Gly Ala Gly Thr
               500                 505                 510
   Lys Leu Glu Leu Lys Arg Ala Ala Gly Ser Ser Gly Gly Gly Gly Ser
           515                 520                 525
   Asp Ile Ala Ala Ala Glu Gln Lys Leu Ile Ser Glu Glu Asp Leu Asn
       530                 535                 540
   Gly Ala Ala His His His His His His
   545                 550

## Description

### Background of the Invention

Immunoglobulins (Igs) are composed of two heavy and two light chains, each of which contains an NH₂-terminal antigen-binding variable domain and a COOH-terminal constant domain responsible for the effector functions of antibodies. The COOH-terminal domains of Ig heavy chains form the Fc region and are involved in triggering cellular activities through interaction with specific receptors known as Fc receptors (FcRs). Fc receptors for all Ig classes, or isotypes, (e.g., IgG (FcγR), IgE (FcεR), IgA (FcαR), IgM (FcµR) and IgD (FcδR) have been identified. The different biological activities of antibodies of different isotypes are based in part on their ability to bind to different FcR expressed on different immune (effector) cells (Fridman, W.H. (Sept. 1991) The FASEB Journal Vol. 5. 2684-2690). Murine antibodies, which are directed against FcRs have been made (See e.g. U.S. Patent No. 4,954,617 entitled *Monoclonal Antibodies To Fc Receptors for Immunoglobulin G on Human Mononuclear Phagocytes* and International Patent Application Publication No. WO 91/05871 entitled *Monoclonal Antibody Specific For IgA Receptor).*

Murine monoclonal antibodies can be useful as human therapeutics and can be produced free of contamination by human pathogens such as the hepatitis or human immunodeficiency virus. However, use of murine monoclonal antibodies in some human therapies, have resulted in the development of an immune response to the "foreign" murine proteins. This response has been termed a human anti-mouse antibody or HAMA response (Schroff R. et al. (1985), Cancer Res., 45, 879-885) and is a condition which causes serum sickness in humans and results in rapid clearance of the murine antibodies from an individual's circulation. The immune response in humans has been shown to be against both the variable and the constant regions of murine immunoglobulins.

Recombinant DNA technology can be used to alter antibodies, for example, by substituting specific immunoglobulin regions from one species with immunoglobulin regions from another species. Neuberger et al. (Patent Cooperation Treaty Patent Application No. PCT/GB85/00392) describes a process whereby the complementary heavy and light chain variable domains of an Ig molecule from one species may be combined with the complementary heavy and light chain Ig constant domains from another species. This process may be used to substitute the murine constant region domains-to create a "chimeric" antibody which may be used for human therapy. A chimeric antibody produced as described by Neuberger et al. has a human Fc region for efficient stimulation of antibody mediated effector functions, such as complement fixation, but still has the potential to elicit an immune response in humans against the murine ("foreign") variable regions.

Winter (British Patent Application Number GB2188538A) describes a process for altering antibodies by substituting the complementarity determining regions (CDRs) with those from another species. This process may be used to substitute the CDRs from the murine variable region domains of a monoclonal antibody with desirable binding properties (for instance to a human pathogen) into human heavy and light chain Ig variable region domains. These altered Ig variable regions may then be combined with human Ig constant regions to create antibodies which are totally human in composition except for the substituted murine CDRs. The "reshaped" or "humanized" antibodies described by Winter elicit a considerably reduced immune response in humans compared to chimeric antibodies because of the considerably less murine components. Further, the half life of the altered antibodies in circulation should approach that of natural human antibodies. However, as stated by Winter, merely replacing the CDRs with complementary CDRs from another antibody which is specific for an antigen such as a viral or bacterial protein, does not always result in an altered antibody which retains the desired binding capacity. In practice, some amino acids in the framework of the antibody variable region interact with the amino acid residues that make up the CDRs so that amino acid substitutions into the human Ig variable regions are likely to be required to restore antigen binding.

Bispecific molecules, (e.g., heteroantibodies) comprising an anti-Fc receptor portion and an anti-target portion have been formulated and used therapeutically, e.g., for treating cancer (e.g. breast or ovarian) or pathogenic infections (e.g., HIV) (See, e.g., International Patent Application Publication No. WO 91/05871 entitled *Bispecific Heteroantibodies With Dual Effector Functions*; and Intemational.Patent Application Publication No. WO 91/00360 entided *Bispecific Reagents for AIDS Therapy).* In addition, bispecific molecules, which recognize antigens and antigen presenting cells can be administered to a subject to stimulate an immune response (See, e.g., International Patent Application Publication No. WO 92/05793 entitled *Targeted lmmunostimulation With Bispecific Reagents).*

WO 98/02463 describes multispecific compounds comprising a binding determinant which binds to an Fcα receptor and a binding determinant which binds to one or more target antigens. WO 98/23646 describes bifunctional antibodies or heteroantibodies containing a binding region derived from an anti- Frα receptor antibody and the binding region of a target-specific antibody.

### Summary of the Invention

The present invention relates to bispecific molecules, in vitro methods and use of bispecific molecules as detailed in the appended claims.

Multispecific molecules include molecules comprised of at least one portion which binds to a molecule on an effector cell, such as an Fc receptor, and at least one portion (e.g., two, three, four or more portions) which binds to a different target, such as an antigen on a tumor cell or a pathogen. Therefore, these molecules can be used to induce effector cell-mediated elimination of a target. Multispecific molecules may comprise human or humanized antibodies (or antibody fragments) as binding specificities.

Multispecific molecules may include antigen "multimer complexes" comprised of multiple (i.e., two or more) portions which bind to a molecule on an antigen presenting cell (APC), such as an Fc receptor, linked to one or more antigens. These multimer complexes target antigens, such as self antigens, to APCs to induce and/or enhance internalization (endocytosis), processing and/or presentation of the antigen by the APC. Therefore, these molecules can be used to induce or enhance an immune response either *in vivo* or *in vitro* against a normally non-immunogenic protein, such as a self-antigen.

Also described are multispecific molecules which minimally comprise an anti-Fc receptor portion, an anti-target portion and optionally a third portion directed against a third target. This third portion, also referred to herein as an "anti-enhancement factor" (anti-EF) portion, can be for example an antibody or antibody fragment, or a cell receptor ligand. The multispecific molecules may comprise human or humanized antibodies (or antibody fragments) as binding specificities.

Further described are methods for generating multispecific molecules. In one example, both specificities are encoded in the same vector and are expressed and assembled in a host cell, for example, as bispecific molecules or fusion proteins, or each binding specificity is generated separately (e.g., recombinantly) and then chemically conjugated to one another via e.g., sulfhydryl bonding of the C-terminus hinge regions of the heavy chain in the case of antibodies.

Further described are multispecific molecules, also referred to herein as "antigen multimer complexes", which comprise two or more binding specificities linked to at least one antigen. The binding specificities bind to a component on the surface of an antigen presenting cell, such as an Fc receptor, which is capable mediating internalization of the molecular complex when bound by the binding specificities. The molecular complex may comprise three or more binding specificities, and at least one of the binding specificities are specific to an Fc Receptor (e.g., FcγRI or FcαR). In one example, the antigen is non-immunogenic when administered in uncomplexed, form.

Further described are methods for inducing or enhancing an immune response against an antigen in a subject, by administering to the subject an antigen multimer complex, and methods for immunizing a subject, by administering to the subject an antigen multimer complex.

Multispecific molecules can be used in multiple immunotherapies based on their ability to cause effector cell-mediated killing or target cells. The multispecific molecules can be used to kill or inhibit the growth of a variety of tumor cells.

### Brief Description of the Drawings

Figure 1 shows the nucleotide and amino acid sequences of a portion of the hinge region of a humanized Fcγ RI antibody, H22. [A] that was altered to produce a truncated single-sulfhydryl version [B] and then altered further to engineer two unique cloning sites [C]. Underlined nucleotides indicate changes from the previous sequence. Overlined nucleotides are the recognition sequences for the indicated restriction sites.
Figure 2 is a schematic representation of the heavy chain-EGF fusion expression construct pJG055.
Figure 3 is a schematic representation of the generation of anti-Fc receptor-ligand bispecific molecules.
Figure 4 is a schematic representation of the flow cytometric assay used for testing the activity of the humanized Fc γ receptor- epidermal growth factor fusion protein.
Figure 5 is a graph, which plots Mean Fluorescence Intensity (MFI) an indication of the binding of various concentrations of epidermal growth factor (EGF) fusion protein (H22-EGF fusion) and the fully humanized bispecific (BsAb) H447 to EGF receptor (EGFR) expressing 1483 cells.
Figure 6 is a graph, which plots the binding of various concentrations of the EGF fusion protein or the BsAb H447 to A431 cells in the presence and absence of murine antibody M425, which binds EGFR.
Figure 7 is a graph, which plots the antibody dependent cytotoxicity (ADCC) resulting from the binding of various concentrations of the EGF fusion protein, BsAb H447 or the H425 antibody to A431 cells.
- Figure 8 is a a bar graph which plots the ADCC resulting from the binding of EGF fusion protein, BsAb H447 or the H425 antibody in the presence of media alone, media containing 25% human serum (HS) or media containing a fab fragment of the Fcγ receptor antibody m22.
Figure 9 is a schematic diagram representing the number of viable A431 cells cultured in the presence of various amounts of EGF, H22-EGF, the Fab fragment of H22 (H22 Fab), or the F(ab')₂ fragment of H425 (H425 F(ab')2).
Figure 10 shows the amino acid sequence of the H22Fd-HRG fusion protein.

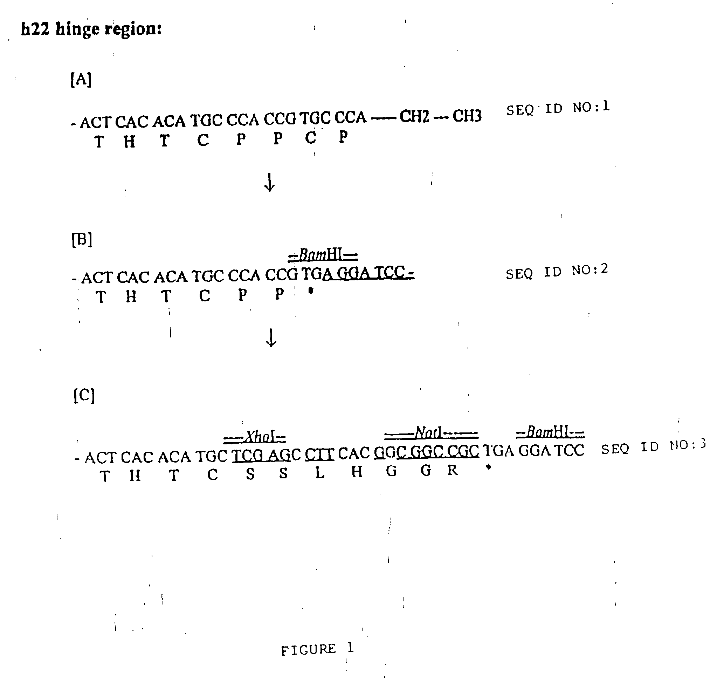

Figure 1 is a histogram indicating the percentage of specific PC-3 or SKBr-3 tumor cell killing resulting from incubation of these cells with interferon-γ-treated monocytes and a 1:3 or 1:30 dilution of supernatant from myeloma cells expressing an H22-heregulin fusion protein.

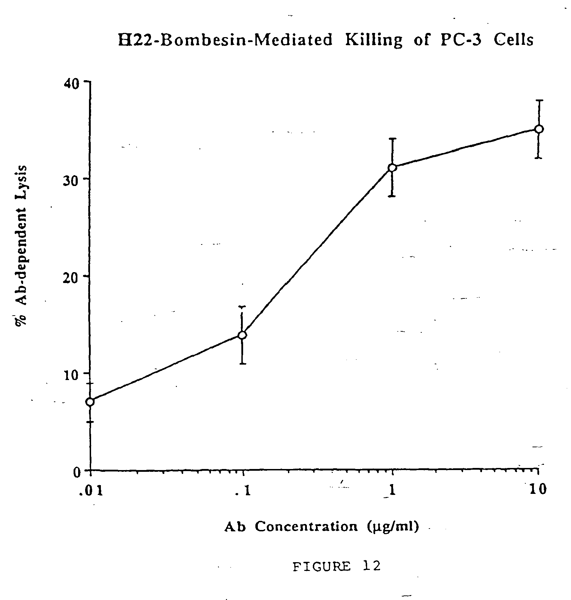

Figure 12 is a diagram indicating the percentage of PC-3 tumor cell lysis in the presence of monocytes and in the presence of various concentrations of H22-bombesin fusion protein concentrations.

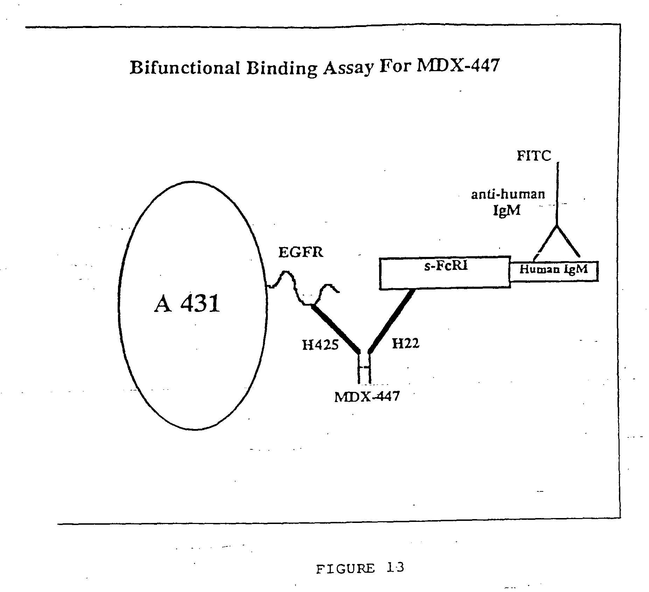

Figure 13 is a schematic representation of the flow cytometric assay used for testing the activity of BsAb 447 generated either by the o-PDM or the DTNB method.

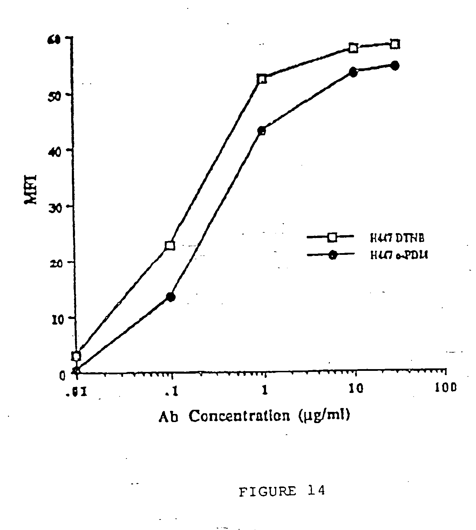

Figure 14 is a graph, which plots the MFI of various concentrations of o-PDM and DTNB derived BsAb 447 to EGFR and FcγRI expressing A431 cells.

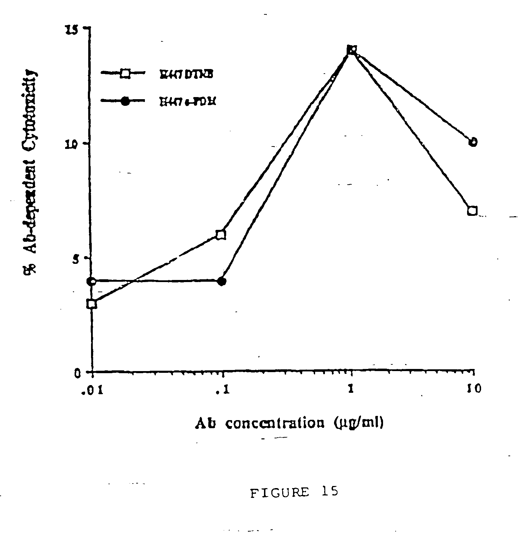

Figure 15 is a graph, which plots the antibody dependent cytotoxicity resulting from the binding of o-PDM and DTNB derived BsAb 447 to A431 cells.

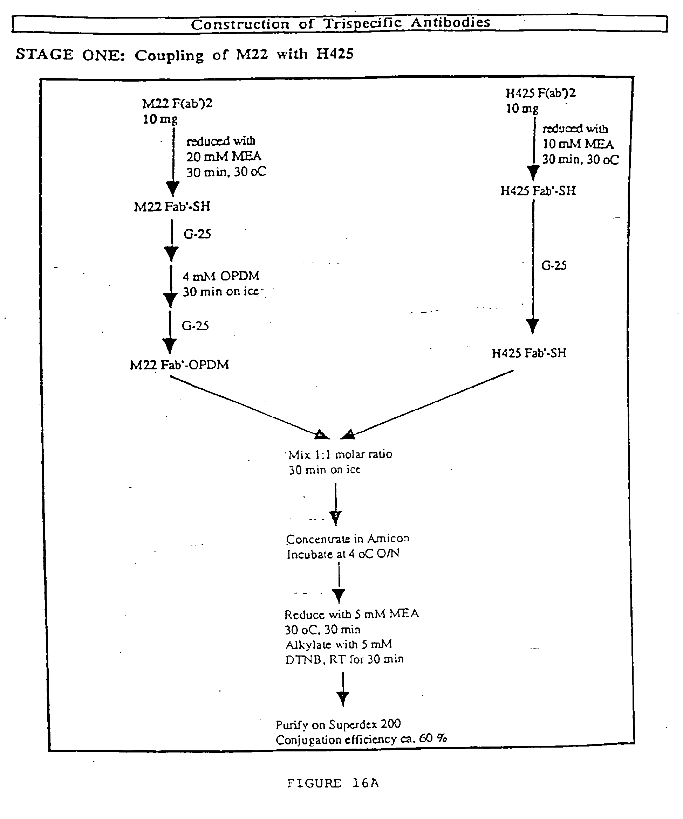

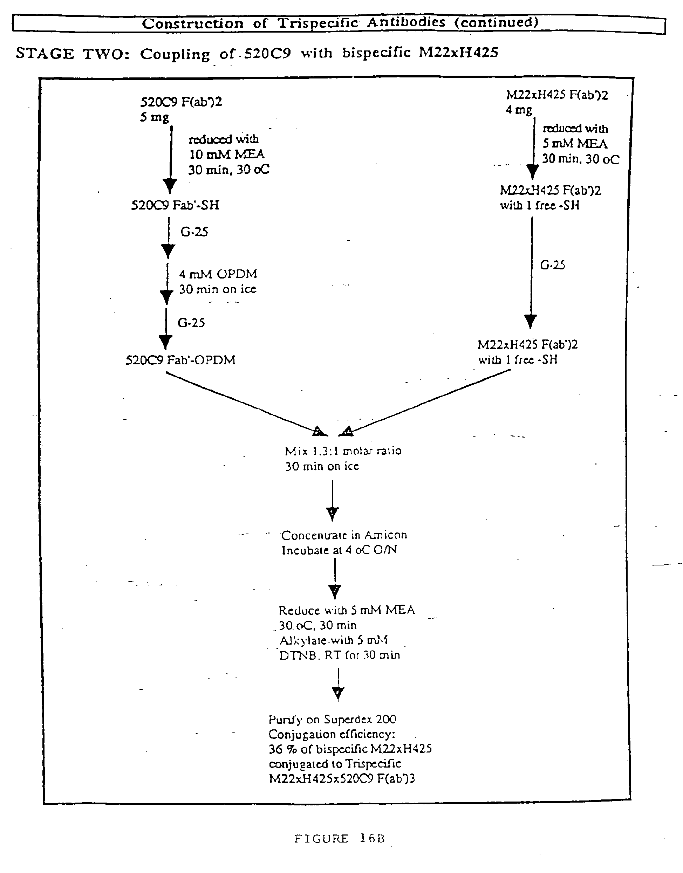

Figure 16 is a flow chart that depicts the construction of trispecific antibodies.

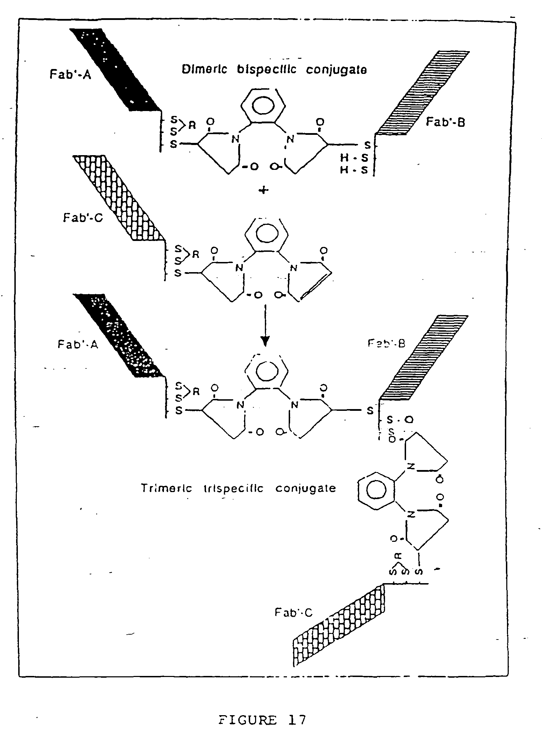

Figure 17 depicts the transformation of a bivalent, bispecific antibody into a trivalent, bispecific antibody. The bivalent, bispecific conjugate is reduced and mixed with o-PDM-treated 520C9 Fab' resulting in the TsAb.

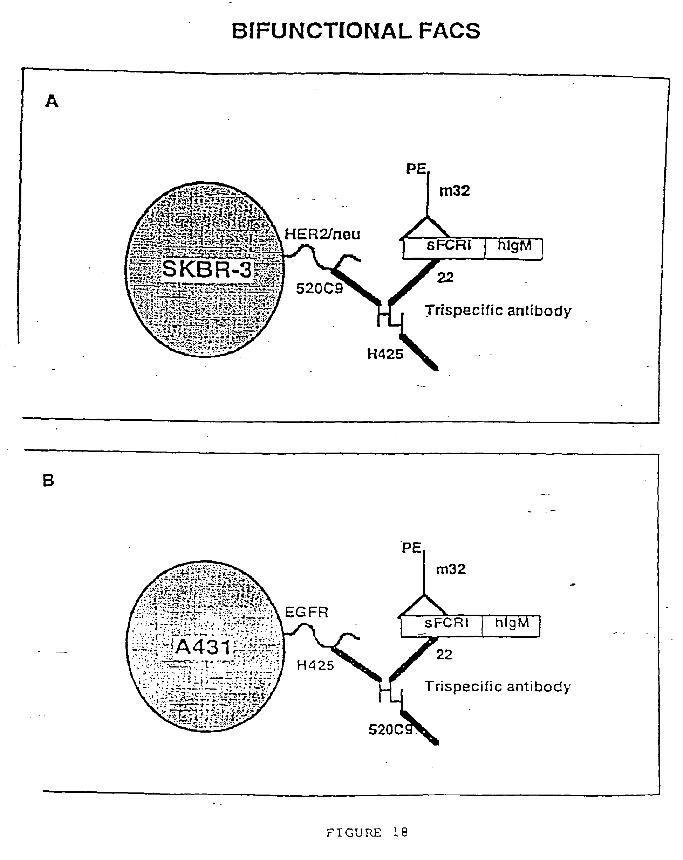

Figure 18 depicts a bifunctional fluorescence-activated cell sorting assay for HER2/neu (panel A) and EGFR (panel B).

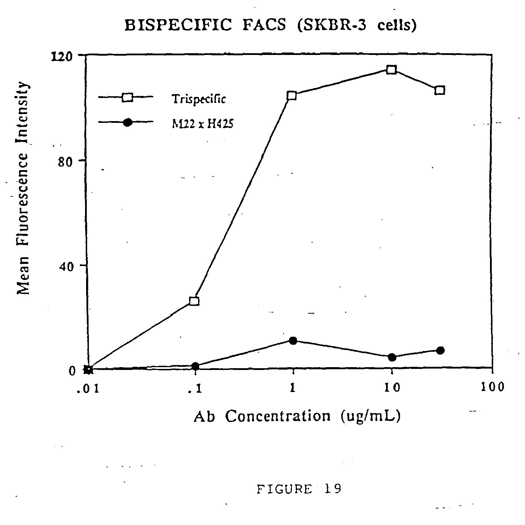

Figure 19 is a graph which plots the binding of various concentrations of antibody, either BsAb or TsAb, to target cells. Mean Fluorescence Intensity (MFI) increases as Ab binding increases. It shows that the TsAb bound both HER2/neu on SKBr-3 cells and soluble FcγRI simultaneously in a dose-dependent fashion.

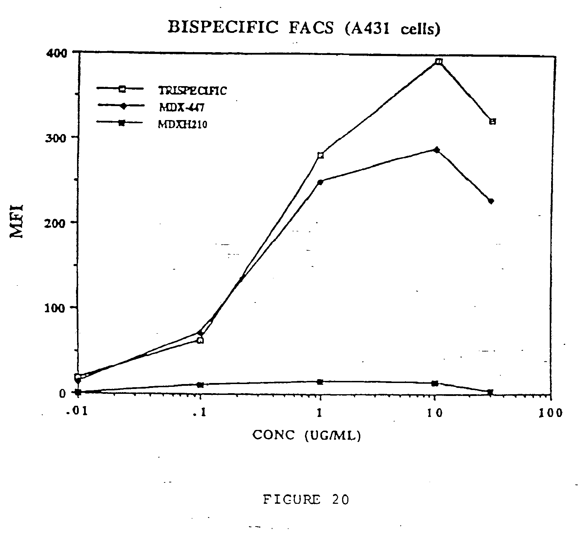

Figure 20 is a graph that shows the TsAb bound both EGFR on A431 cells and soluble FcγRI simultaneously in a dose-dependent fashion. The assay is similar to that used in Figure 19.

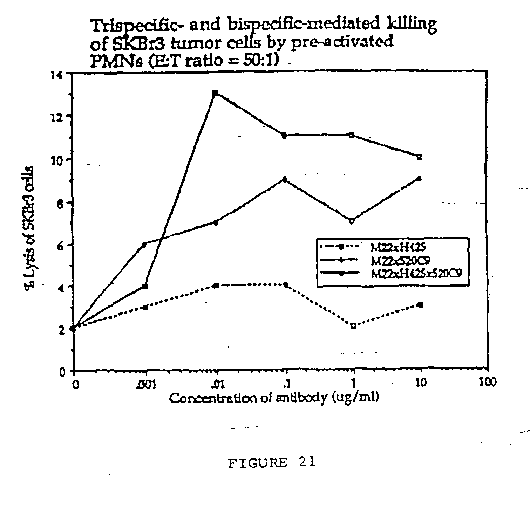

Figure 21 is a graph that shows the TsAb, M22 x H425x 520C9, and the BsAb, M22 x 520C9 were capable of inducing ADCC of SKBR-3 cells but the BsAb, M22 x H425, was not. Various concentrations of antibodies were incubated with SKBR-3 cells and pre-activated PMNs.

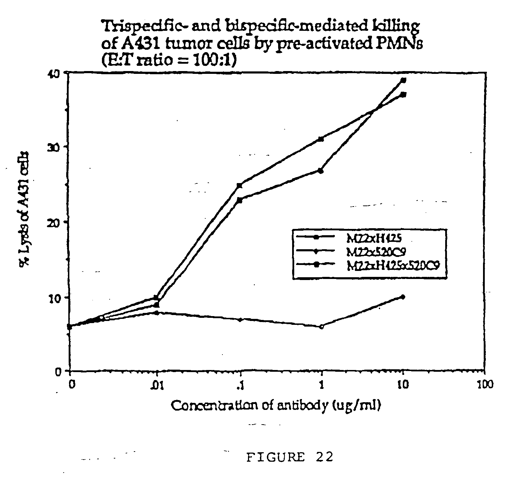

Figure 22 is a graph that shows the TsAb, M22 x H425x 520C9, and the BsAb, M22 x H425 were capable of inducing ADCC of A431 cells but the BsAb, M22 x 520C9, was not. The assay was performed in a similar manner as the assay in Fig. 21.

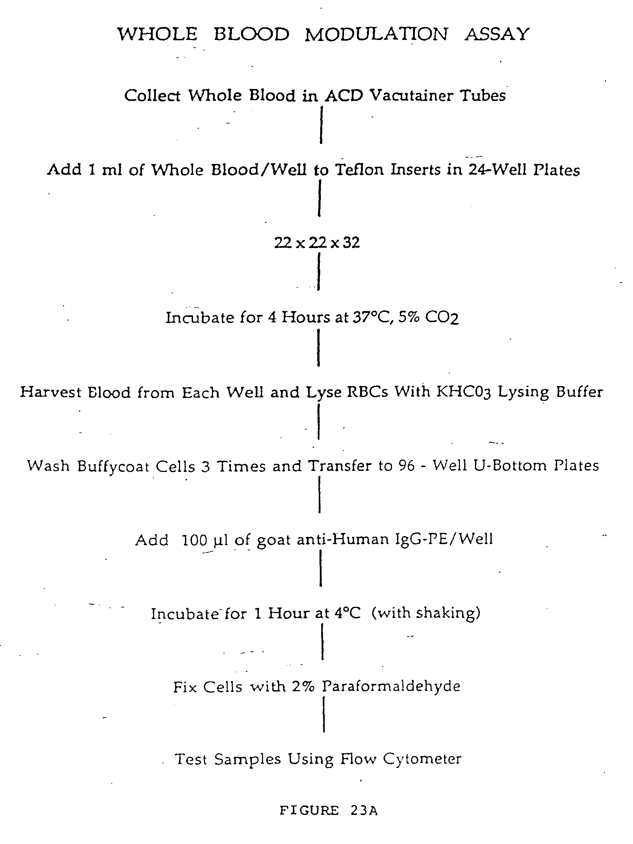

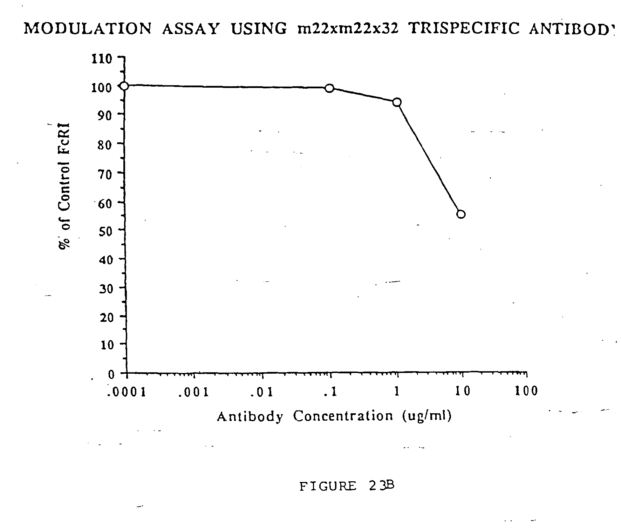

Figure 23 is a flow chart for a whole blood modulation assay (panel A) and the results from the assay (panel B). This trivalent antibody rapidly modulates FcγRI from the surface of monocytes.

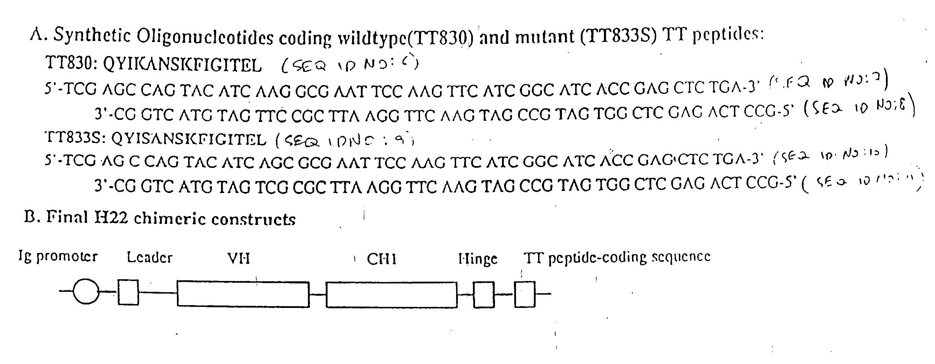

Figure 24, panel A, shows the amino acid sequence of oligonucleotides encoding the wildtype (TT830) and mutant (TT833) tetanus toxin peptides. Panel B is a diagram of an H22Fd-TT fusion protein.

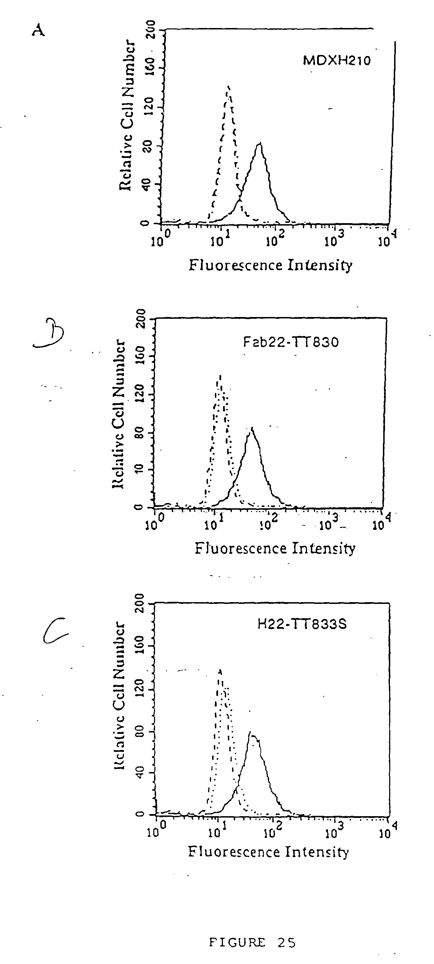

Figure 25 panels A, B, and C represent flow cytometry analysis results showing binding of MDXH210, Fab22-TT830, and H22-TT833S to FcγRI positive U937 cells, respectively. The dashed lines represent negative controls, the solid lines denote staining with the fusion proteins, and the dotted lines respresent fusion protein binding blocked by murine mAb 22 F(ab')₂.

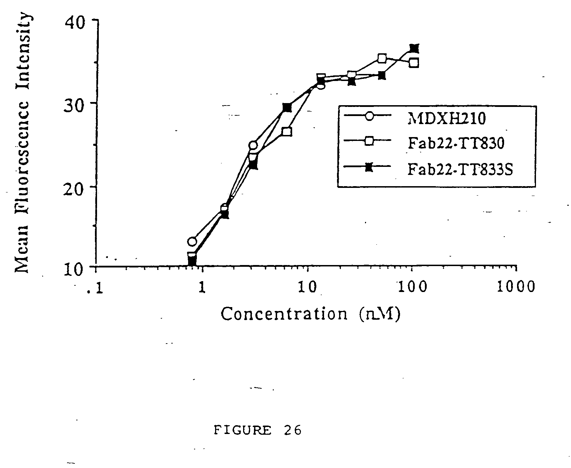

Figure 26 is a schematic diagram showing the mean fluorescence intensity resulting from incubation of various amounts of the fusion proteins MDXH210, FAb22-TT830, and Fab22-TT833S to FcγRI positive U937 cells.

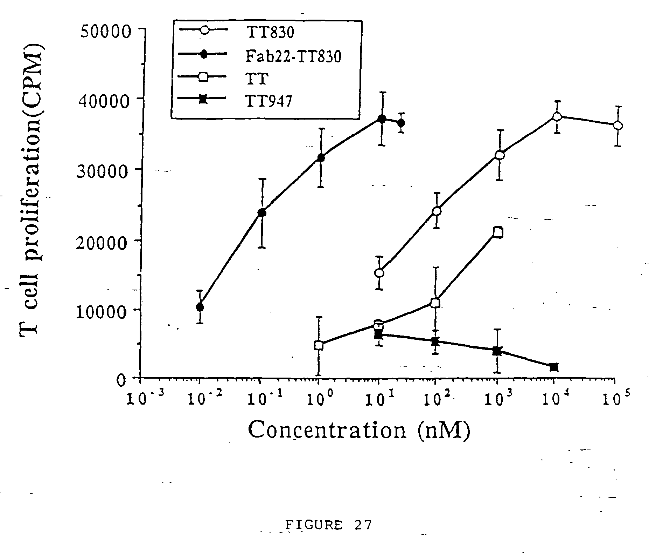

Figure 27 is a graphic representation of the proliferation of T cells incubated with irradiated monocytes and various concentrations of TT830, Fab22-TT830, TT, or TT947, showing that the fusion protein Fab22-TT830 enhances the presentation of the Th epitope by about 1000 fold as compared to TT830.

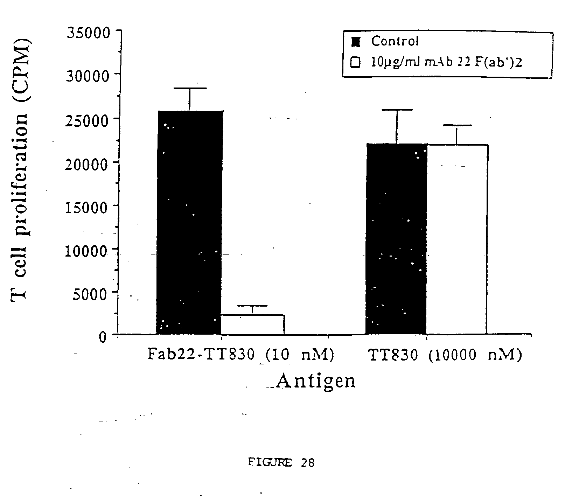

Figure 28 represents a histogram showing the proliferation of T cells incubated with TT830 at 1000 nM or FAb22-TT830 at 10 nM and monocytes, preincubated or not with saturating amounts of mAb 22 F(ab')2 prior to addition of the T cells and the antigen.

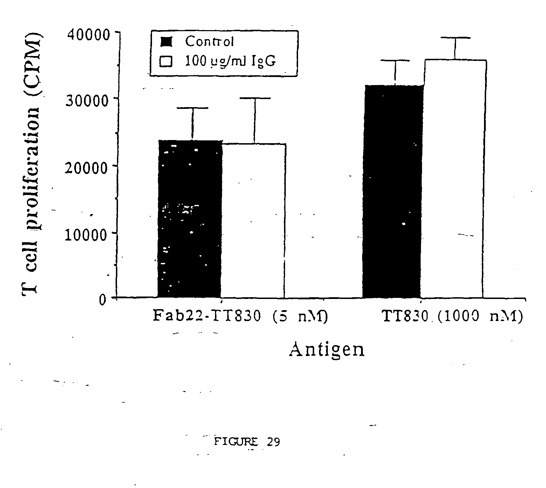

Figure 29_represents a histogram showing the proliferation of T cells incubated with monocytes and Fab22-TT830 at 5 nM or TT830 at 1000 nM in the absence (control) or presence of IgG.

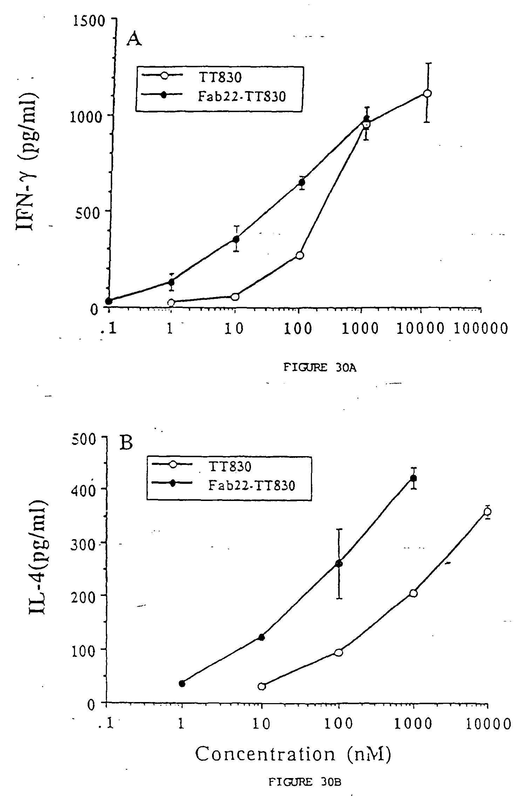

Figure 30, panels A and B, are graphic representations showing the concentration of IFN-γ (panel A) and IL-4 (panel B) in the supernatant of T cells cultured for 2 days with monocytes and various concentrations of TT830 or Fab22-TT830.

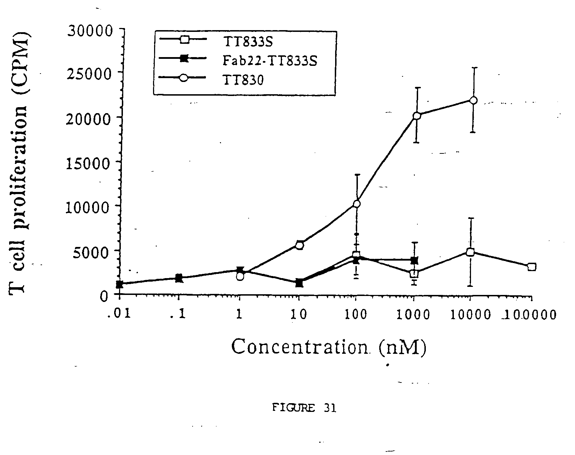

Figure 31 is a graphic representation depicting the proliferation of T cells incubated with monocytes and various concentrations of TT833S, Fab22-TT833S, or TT830.

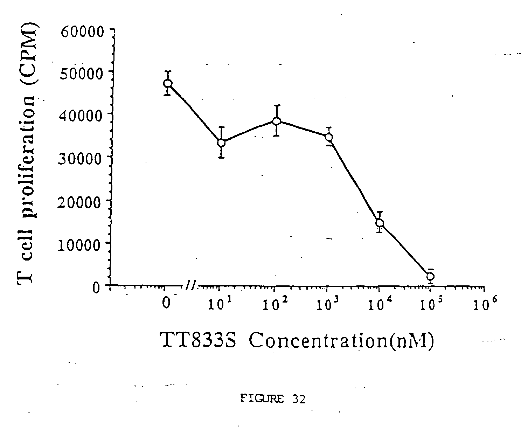

Figure 32 is a graphic representation of the proliferation of T cells incubated for 2 days with TT830 and monocytes, preincubed overnight with various concentrations of TT833S.

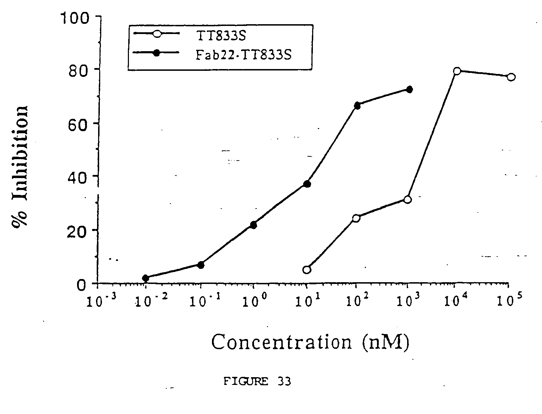

Figure 33 is a graphic representation of the percent inhibition of proliferation of T cells incubated for 2 days with TT830 and monocytes, preincubated overnight with various concentrations of TT833S or FAb22-TT833S.

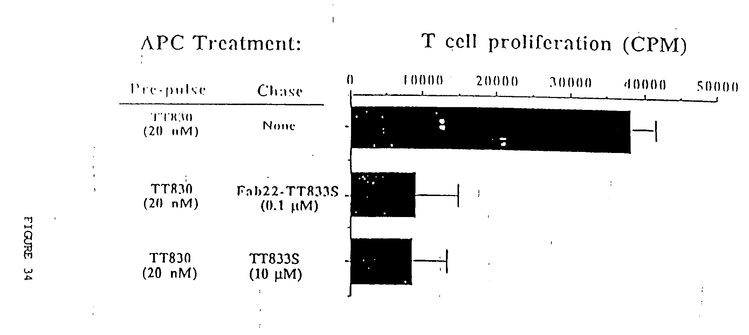

Figure 34 is a histogram representing the proliferation of T cells incubated for 2 days with monocytes, which were first incubated with TT830 for 4 hours (Pre-pulse) and then incubated overnight with 10µM TT833S or 0.1µM Fab22-TT833S (Chase) prior to addition of the T cells.

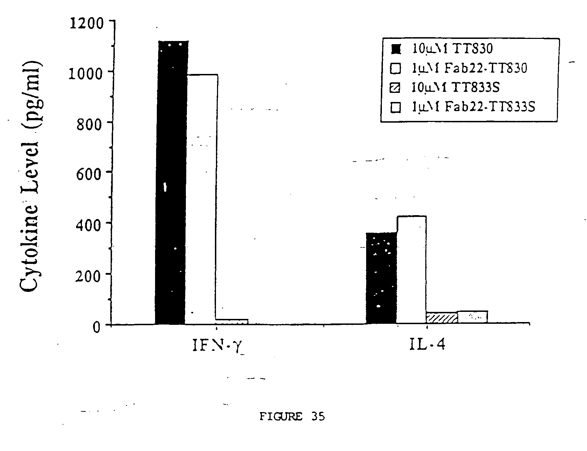

Figure 35 is a histogram representing the concentration of interferon-γ (IFN-γ) and IL-4 in the supernatant ofT cells cultured with monocytes and TT830, FAb22-TT830, TT833S, and Fab22-TT833S.

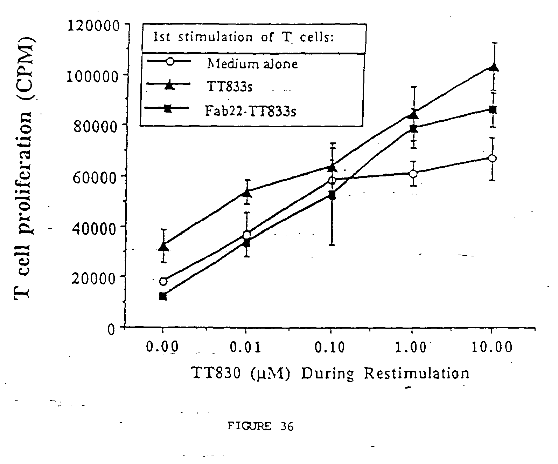

Figure 36 is a graphic representation of the proliferation of T cells stimulated for one day with monocytes in medium alone, with TT833S, or with Fab22-TT833S and then restimulated with monocytes and various concentrations of TT830 for two days, indicating that TT833S and Fab22-TT833S do not lead to T cell anergy.

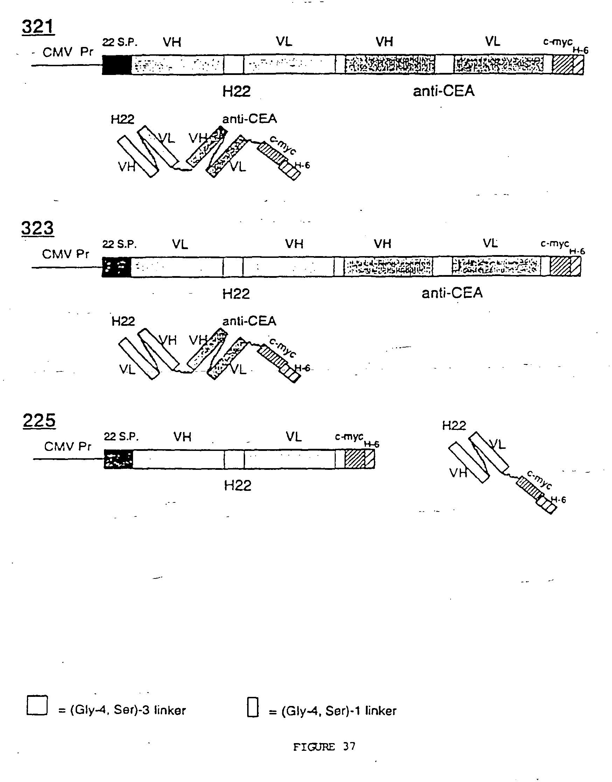

Figure 37 is a graphic representation of two expression constructs encoding single chain bispecific molecules having one binding specificity for an FcγRI (H22) and one binding specificity for a carcinoembryonic antigen (CEA) (constructs 321 adn 323) and one expression construct encoding a single chain antibody having one binding specificity for an FcγRI. The coding regions are under the control of the CMV promoter (CMV Pr). In addition to the variable regions from the heavy (VH) and light chains (VL) of the antibodies, the proteins encoded by these constructs are fused to a peptide from c-myc (c-myc) and to a hexa-histidine peptide (H-6).

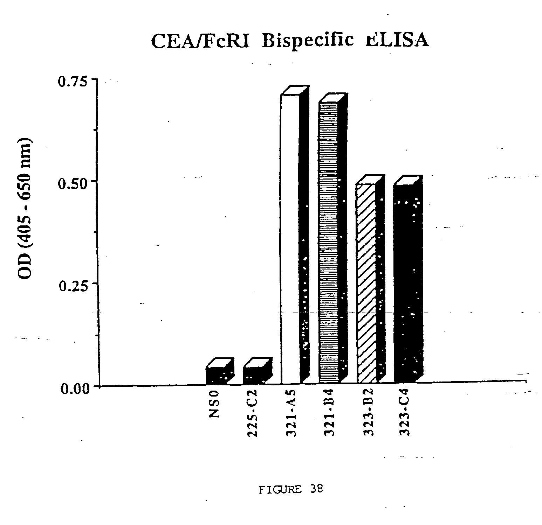

Figure 38 shows a histogram indicating the level of binding of the single chain bispecific molecules H22-anti-CEA encoded by the expression constructs 321 (321-A5 and 321-B4) and 323 (323-B2 and 323-C4) and the single chain H22 antibody encoded by the construct 225 (225-C2) as measured by bispecific ELISA.

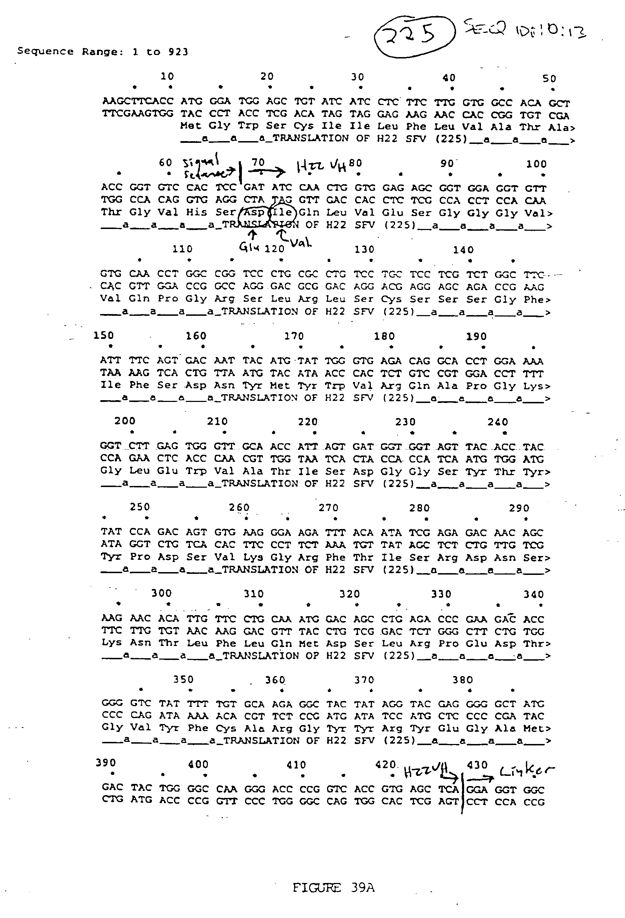

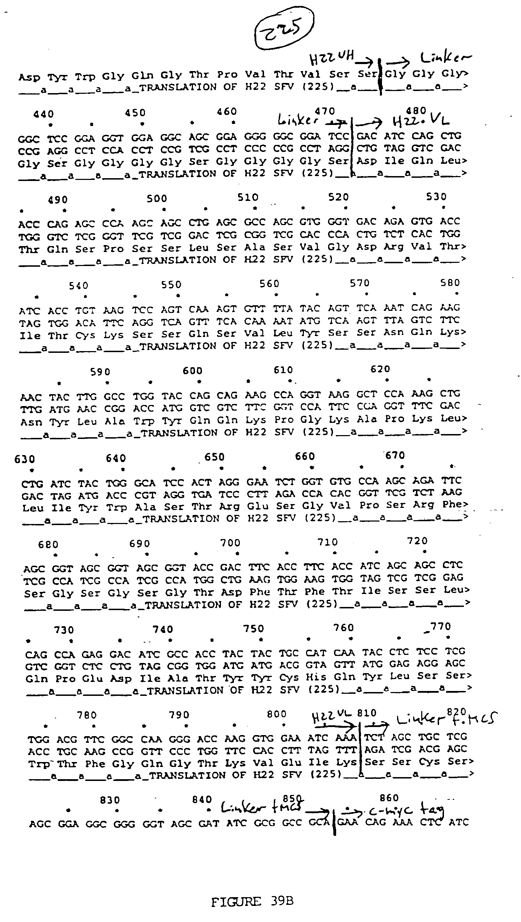

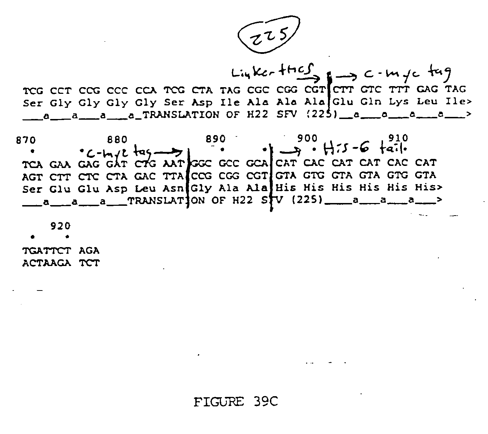

Figure 39 shows the nucleic acid sequence of the single chain humanized anti-Fc γRI antibody and the amino acid sequence encoded by the nucleic acid.

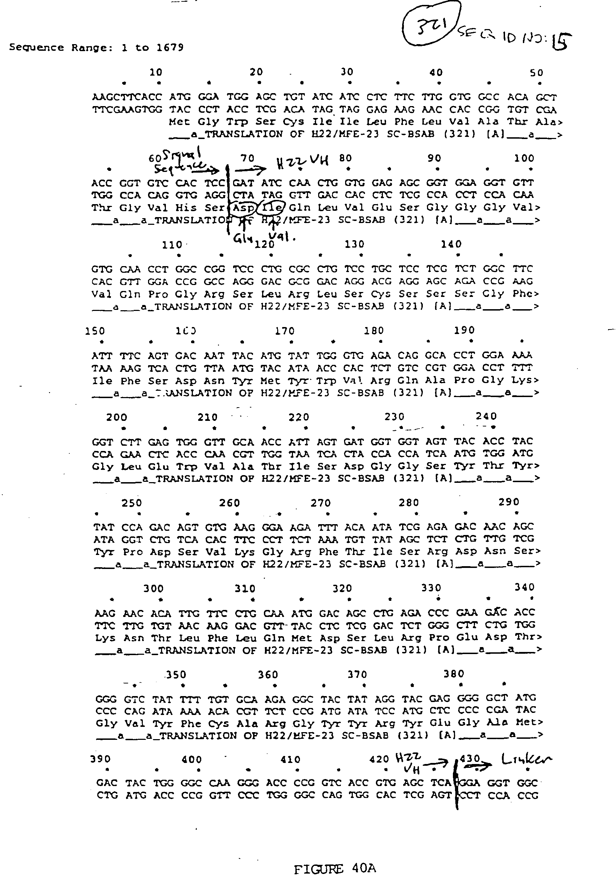

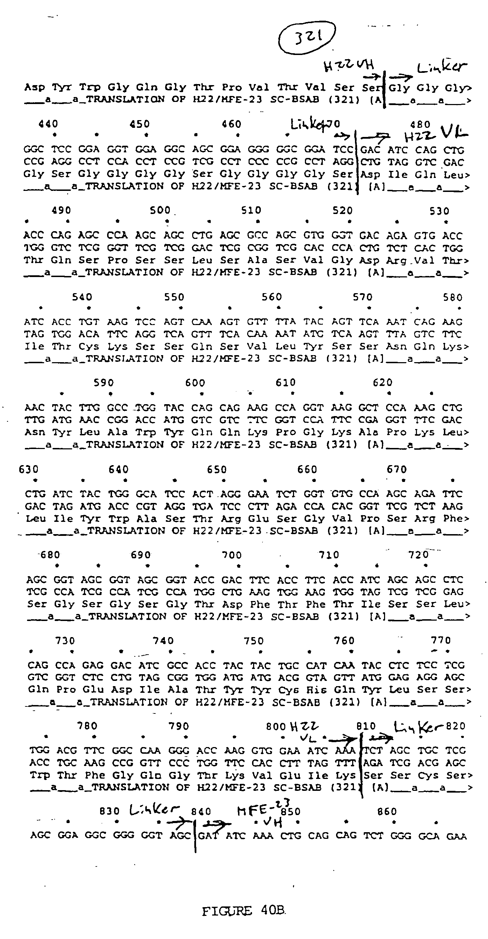

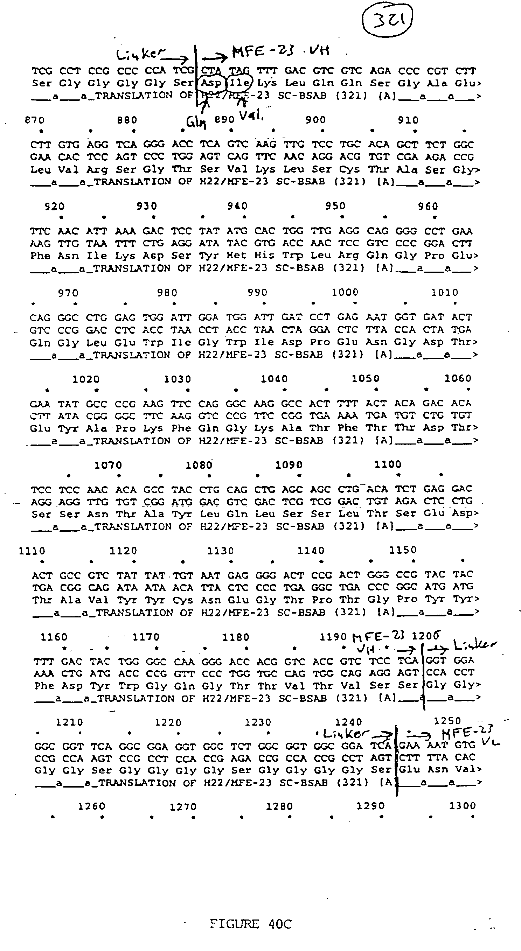

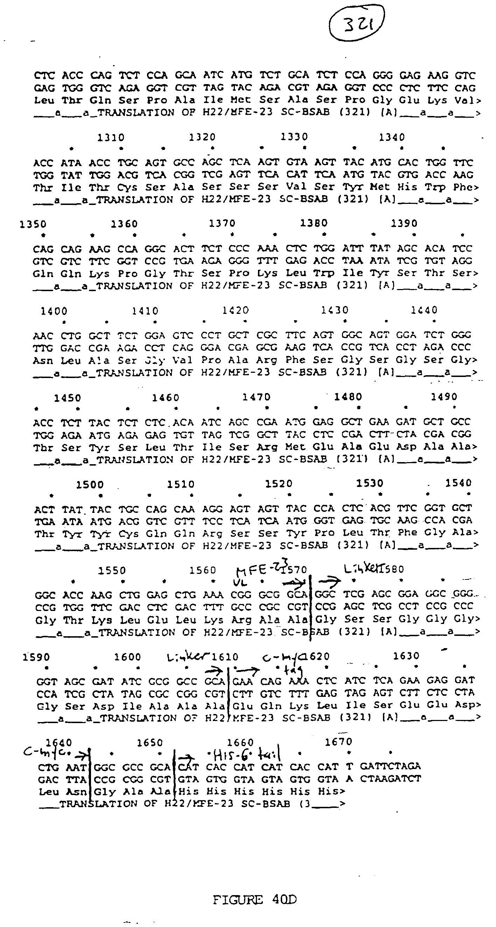

Figure 40 shows the nucleic acid sequence of the single chain bispecific molecule having one binding specificity for the FcγRI and one binding specificity for CEA and the amino acid sequence encoded by the nucleic acid.

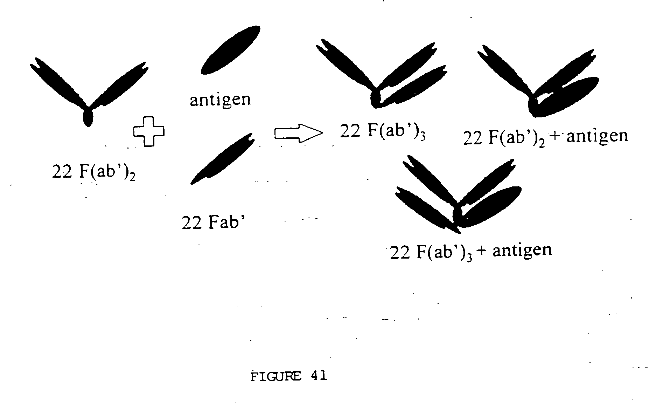

Figure 41 is a schematic representation of the formation of multimer complexes made up of multiple Fab' fragments linked to an antigen.

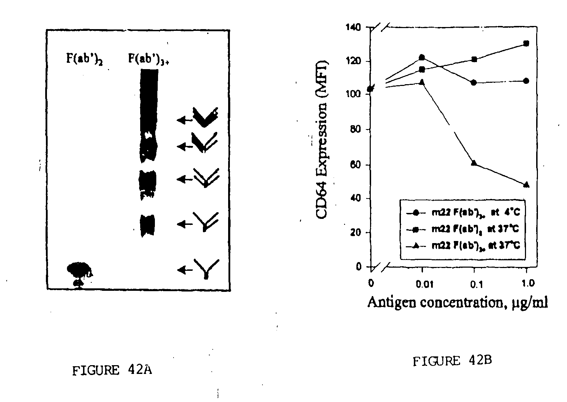

Figure 42(a) is a non-reducing SDS-PAGE gel showing a purified M22 F(ab')2 multimer (lane 1) and a chemically linked M22 Fab' multimer complex (lane 2). Figure 42(b) is a graph showing that incubation with M22 F(ab')3+ results in up to 50% reduction in CD64 expression on the surfaces of macrophages in a dose dependent fashion.

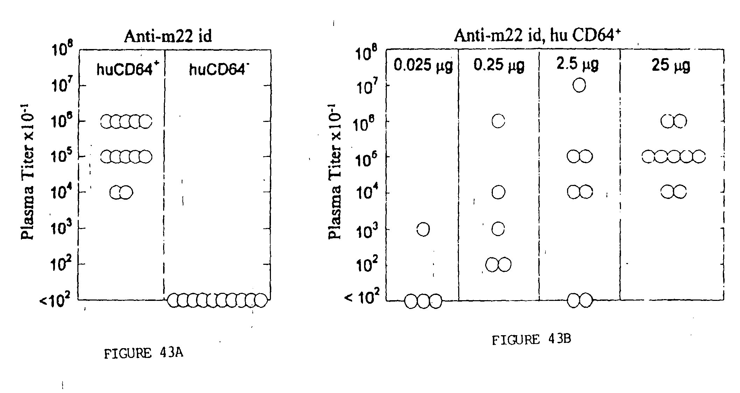

Figure 43(a) shows that high titers of M22-specific antibody were generated in all of the CD64 transgenic mice immunized three times, compared to their nontransgenic littermates. Figure 43(b) shows that immunizing FcγRI (CD64) transgenic mice with as little as 0.25 mg of a multimer complex still leads to a detectable immune response.

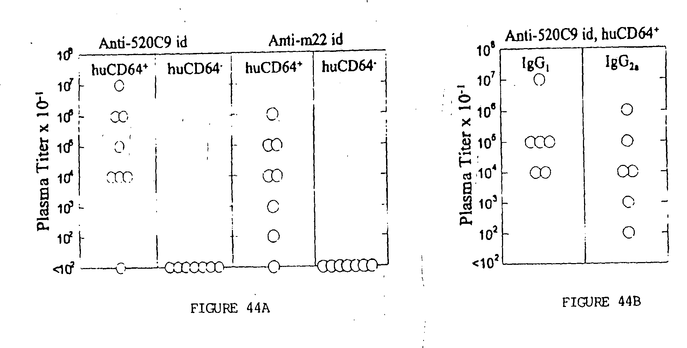

Figures 44(a) and 44(b) are graphs showing the immune response of FcγRI (CD64) transgenic and nontransgenic mice as judged by anti-520C9 and anti-M22 titers. The mice were immunized with a Fab' fragment of a murine antibody, 520C9, coupled to a M22 multimer complex.

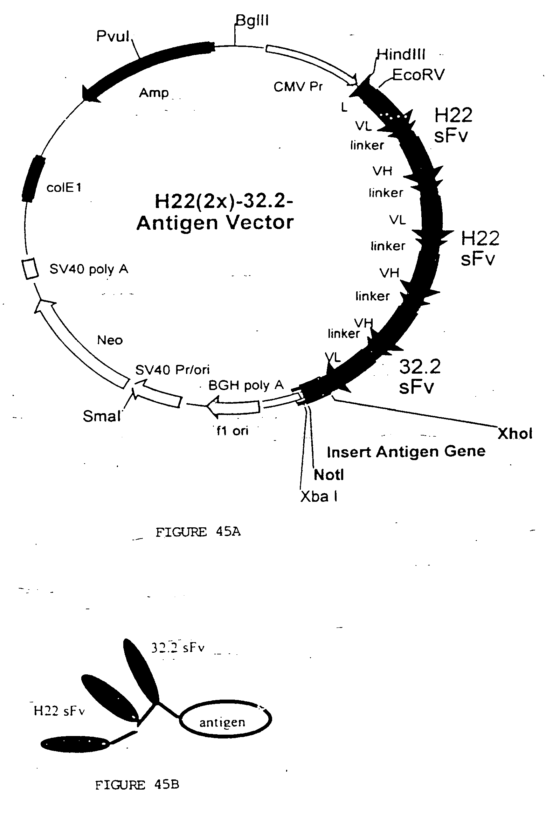

Figure 45(a) shows a map of an expression vector encoding a genetically linked multimer complex containing two H22 sFv regions linked to one M32.2 sFv region linked to an antigen (H22(2x)-32.2-antigen complex). Figure 45(b) is a drawing of the resulting multimer complex.

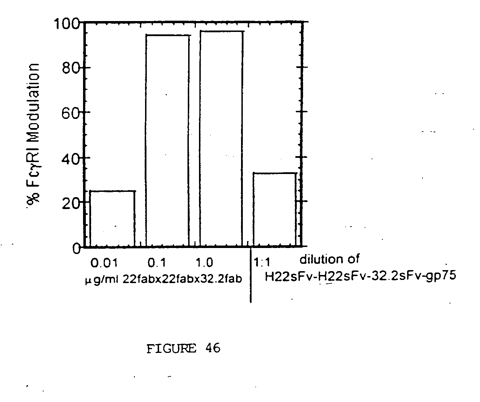

Figure 46 is a bar graph showing the ability of M22 Fab x M22 Fab x M32 and H22sFv-H22sFv-32.2sFv-gp75 multimer complexes to bind to FcγRI efficiently and induce internalization.

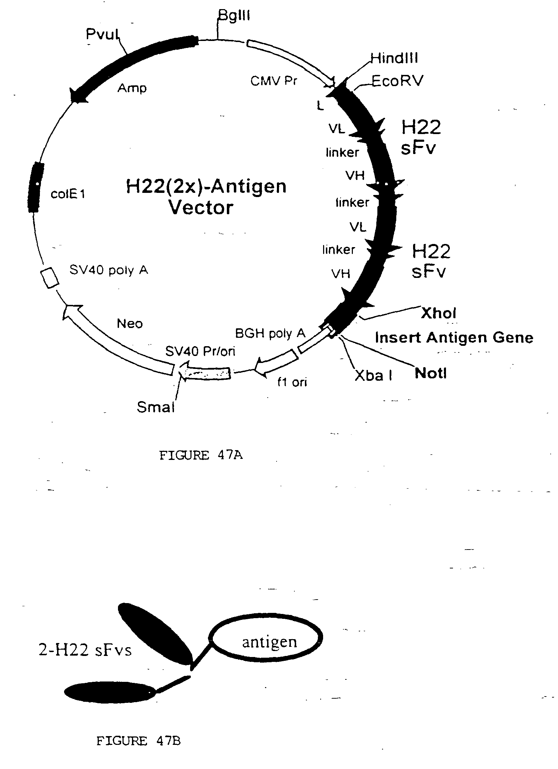

Figure 47(a) shows a map of an expression vector encoding a genetically linked multimer complex containing two H22 sFv regions linked together to an antigen (H22(2x)-antigen multimer complex). Figure 47(b) is a drawing of the resulting multimer complex.

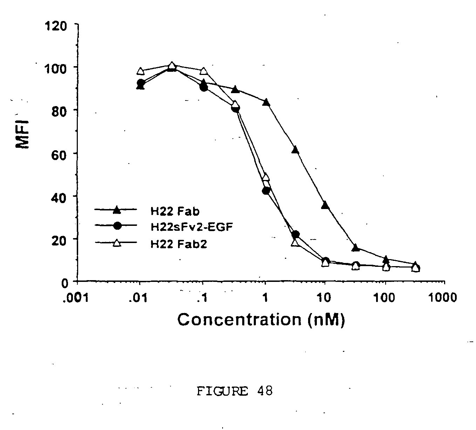

Figure 48 is a graph showing the results of a binding competition assay using H22 Fab', H22sFv2-EGF multimer and H22 Fab2 multimer. Results are shown in terms of binding inhibition of an M22-phycoerythrin conjugate by these multimers.

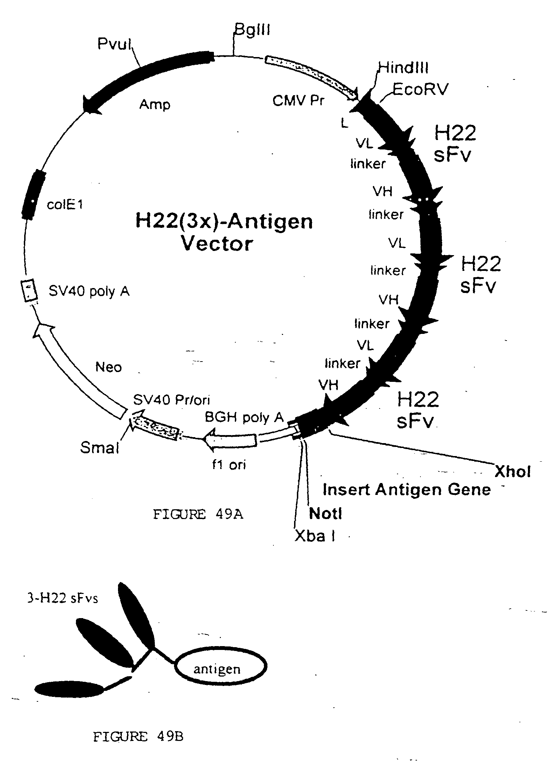

Figure 49(a) shows a map of an expression vector encoding a genetically linked multimer complex containing three H22 sFv fragments linked to an antigen (H22(3x)-antigen multimer complex). Figure 49(b) is a drawing of the resulting protein.

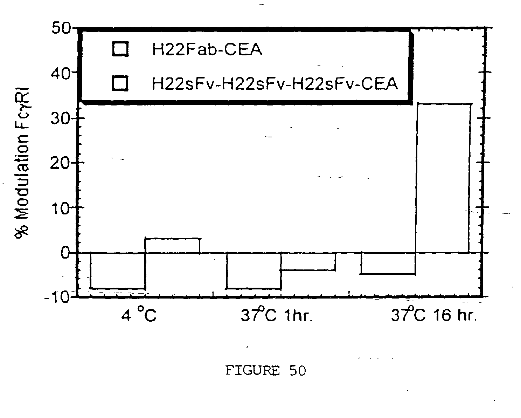

Figure 50 is a bar graph showing the ability of an H22(3x)-CEA multimer complex to induce internalization of FcγRI compared to other multimer complexes.

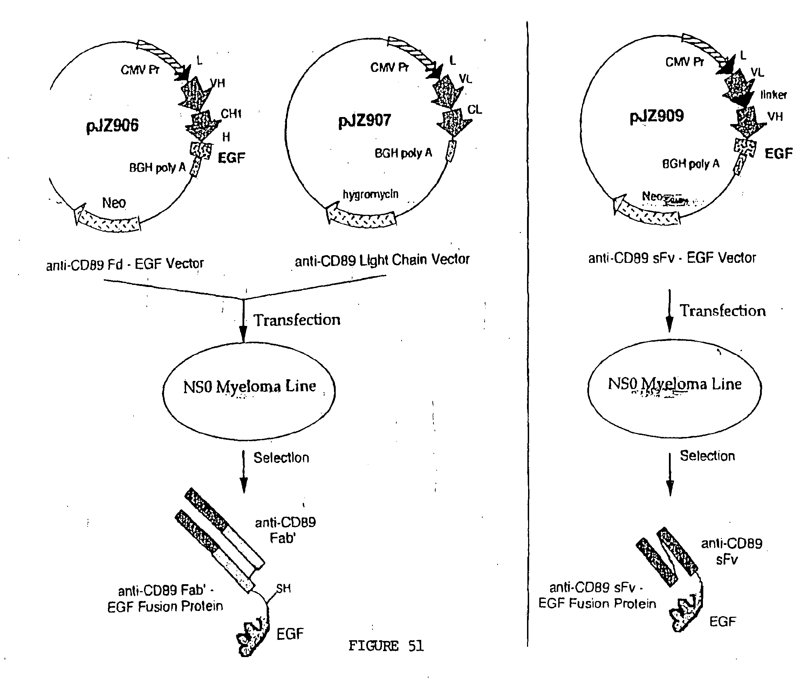

Figure 51 is a schematic representation of the production of anti-CD89 (14A8) HuMAb fusion molecules. PJZ906 (14Afd-EGF) and pJZ907 (14A8 L-chain) were cotransfected by electroporation into NSO cells, and selected in media containing both G418 and hygromycin. PJZ909 (14A8sFv-EGF) was similarly transfected and selected in media containing G418. Cell lines expressing fusion protein were subcloned by limiting dilution cloning.

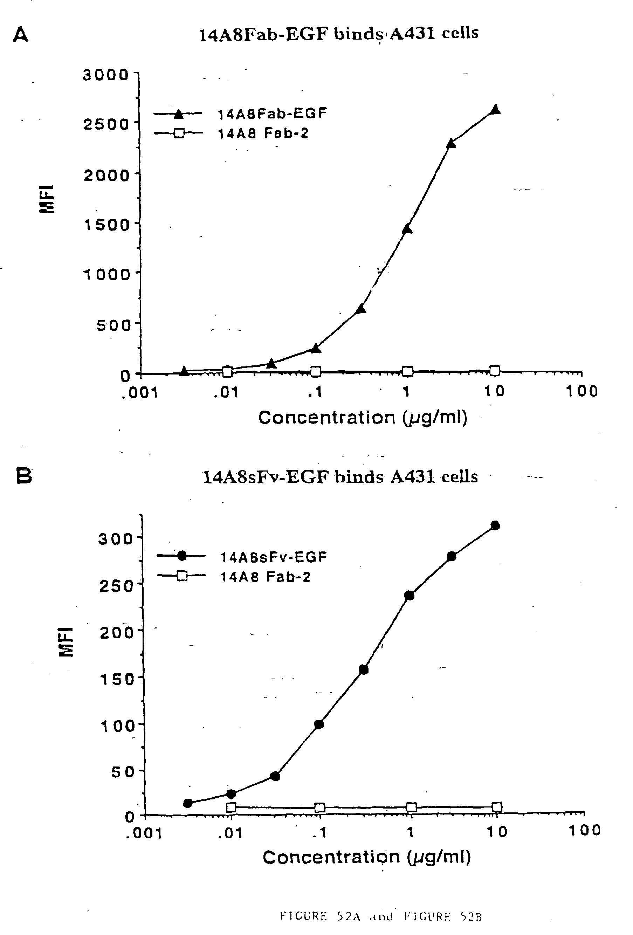

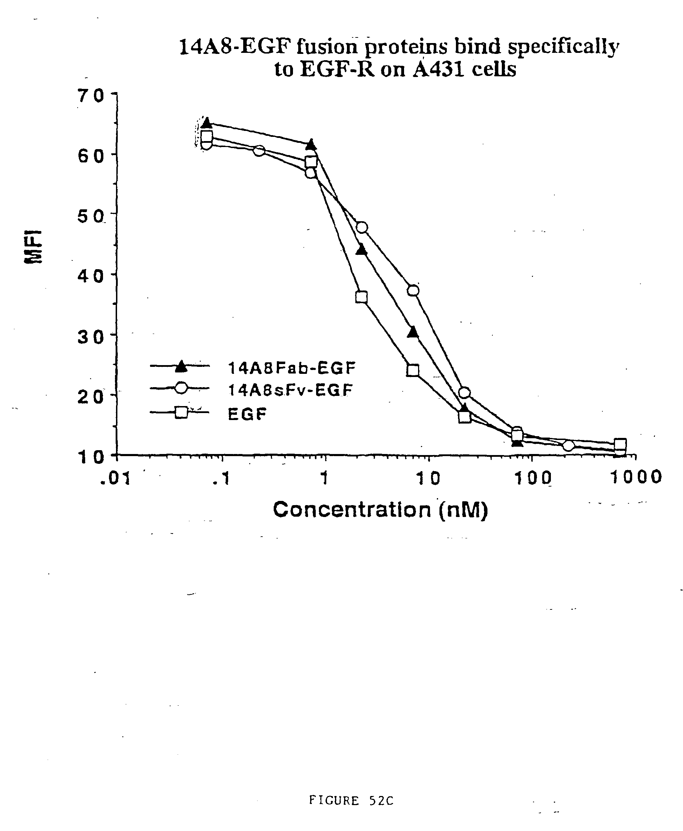

Figure 52(a) is a graph showing binding (as measured by flow cytometry) of 14A8fab'-EGF fusion construct to EGF-R on A431 cells. Figure 52(a) is a graph showing binding of 14A8sFv-EGF fusion construct to EGF-R on A431 cells. A431 cells were stained with goat anti-human IgG Fab-2 conjugated to phycoerythrin. The fab-2 fragment of 14A8 (a human monoclonal anti-FcαR antibody, also referred to as 14.1) which does not contain EGF, was used as control. Figure 52(c) shows that fusion proteins and commercially obtained EGF compete for binding to A431 cells with an EGF-FITC conjugate (7nM).

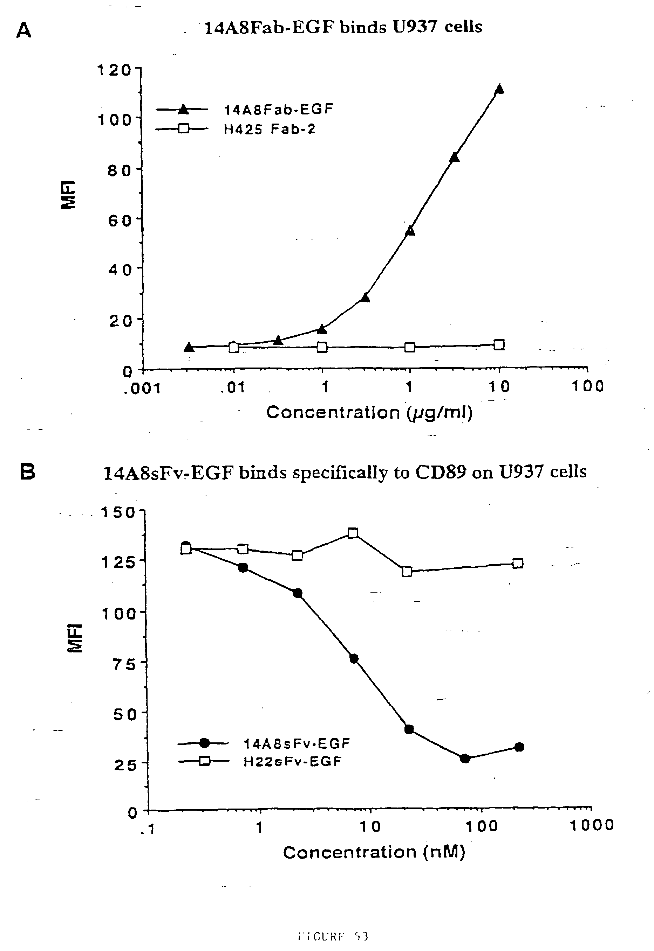

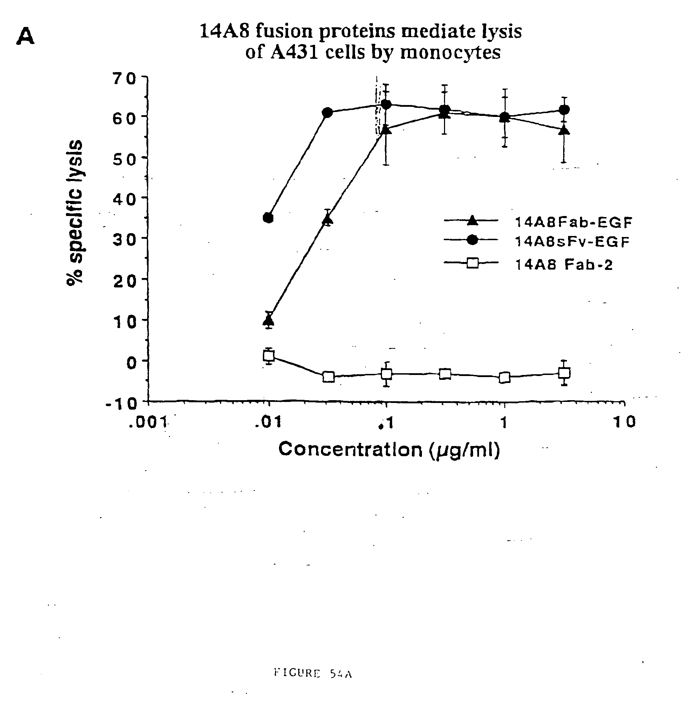

Figure 53(a) is a graph showing binding (as measured by flow cytometry) of 14A8fab'-EGF fusion construct to U937 cells. Fusion protein bound to U937 cells was stained with goat anti-human IgG Fab-2 conjugated to phycoerythrin. The fab-2 fragment of humanized mAb H415, which is specific for EGF-R, was used as control. Figure 53(b) shows binding of 14A8sFv-EGF fusion construct to U937 cells. The experiment was done in a manner similar as in Figure 53(a), except that various concentrations of 14A8sFv-EGF were assessed for their ability to inhibit the binding of 14A8fab'-EGF (23 nM). H22sFv-EGF fusion protein was as control. mAb H22 binds to CD64 (FcγRI), a different Fc receptor on U937 cells.

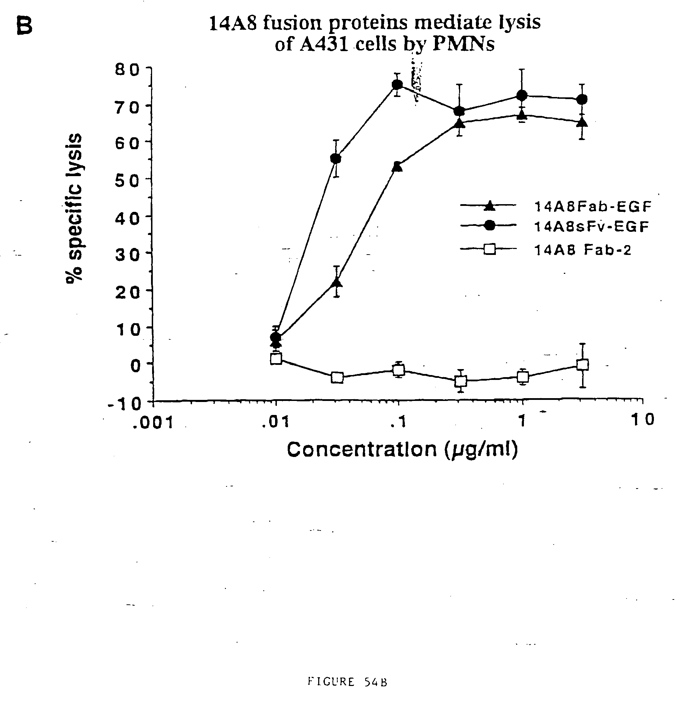

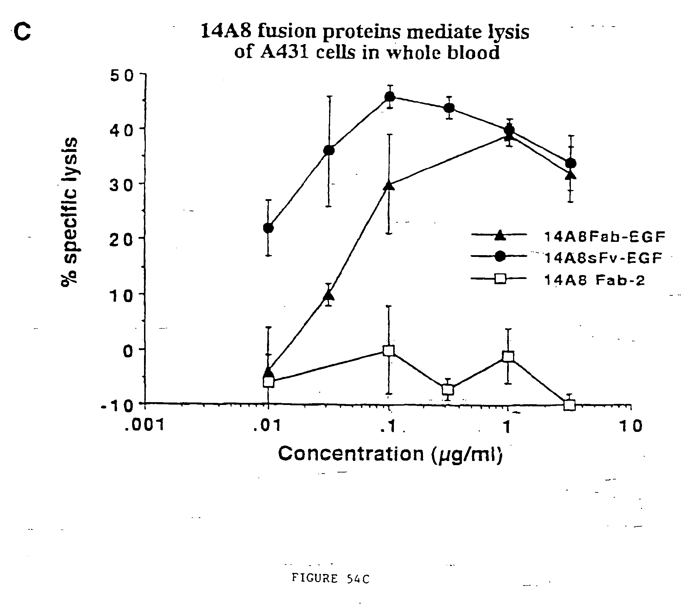

Figure 54 is a graph showing dose-dependent fusion protein-mediated cytotoxicity of A431 cells. Chromium release assays were done with an incubation period of 16-18 h and using an effector to target ration (for monocytes and PMN) of 100:1. The effector cells included fresh monocytes (Figure 54(a)), PMNs (Figure 54(b) and whole blood (Figure 54(c). The fab-2 fragment of 14A8 was used as control.

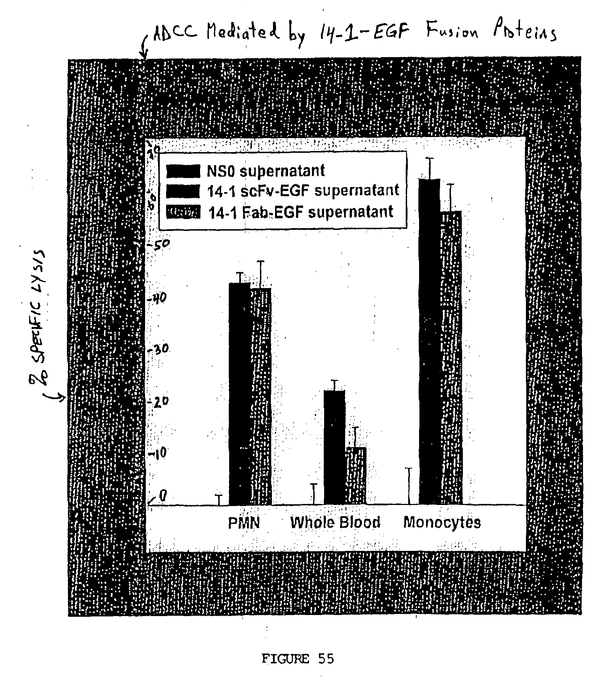

Figure 55 is a bar graph comparing the results in Figures 54(a)-(c).

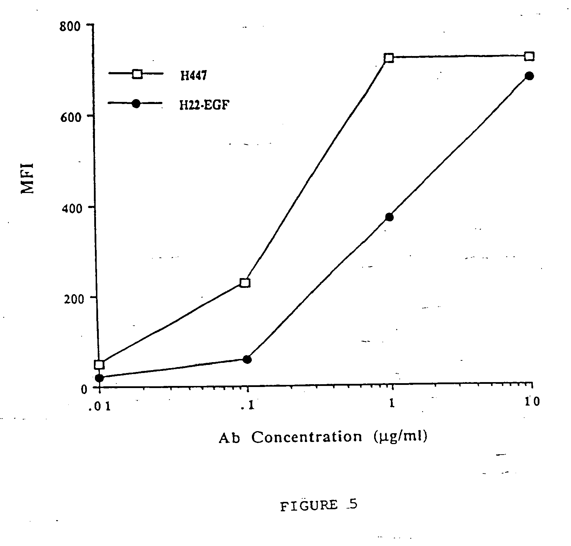

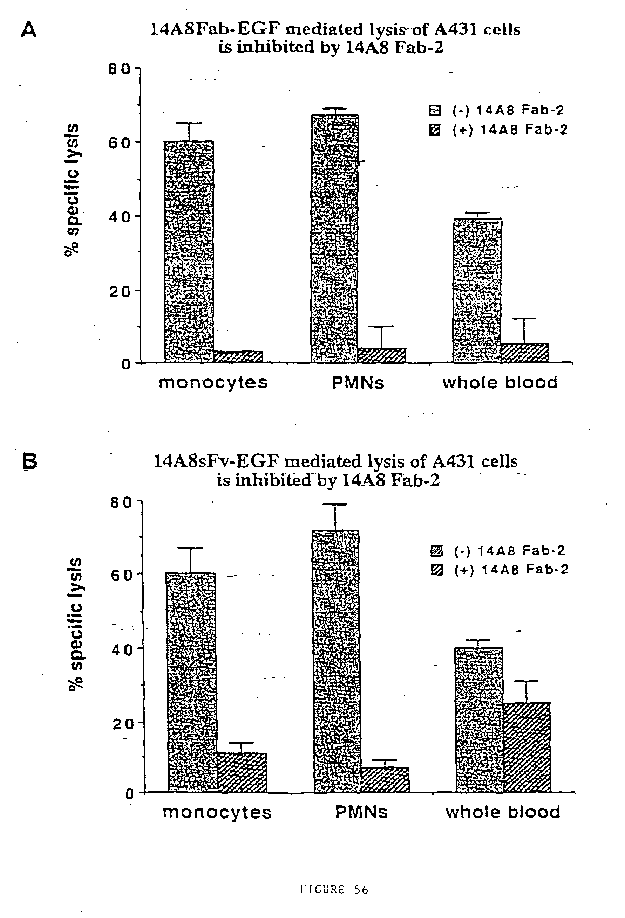

Figure 56(a) is a bar graph showing that 14A8fab'-EGF (1µg/ml) fusion protein-mediated cytotoxicity of A431 cells is inhibited by the fab-2 fragment of 14A8. Figure 5-6(b) is a bar graph showing that 14a8sfv-egf (1 µg/ml) fusion protein-mediated cytotoxicity of A431 cells is also inhibited by the fab-2 fragment of 14A8 (40 µg/ml).

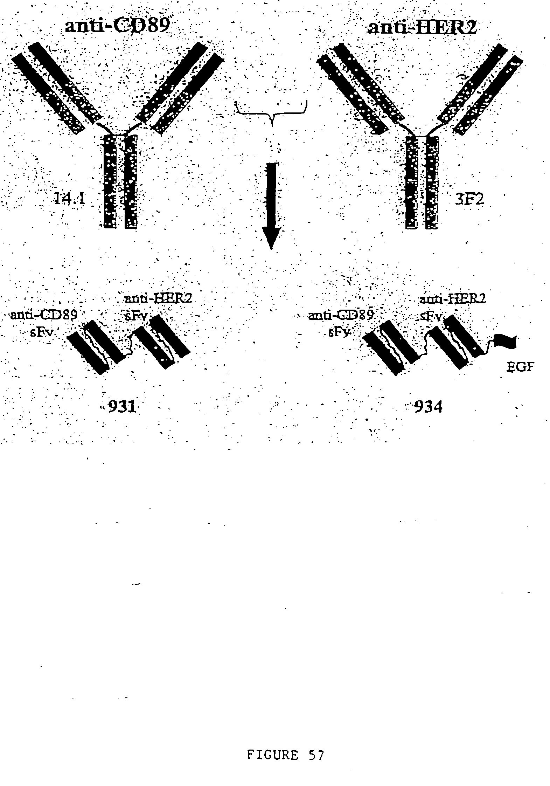

Figures 57 is a schematic representation of bispecific single chain fusion molecules, 931 and 934, and their parent HuMabs. The variable light and heavy chain regions of 14.1 and 3F2 were used to generate the 931 and 934 (w/EGF) constructs.

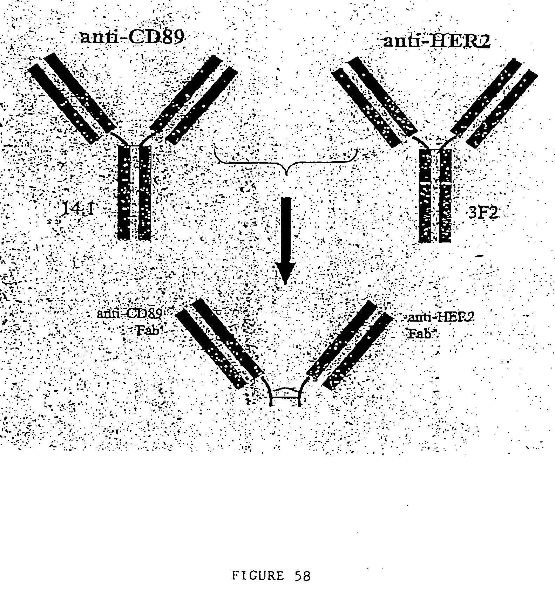

Figure 58 is a schematic representation of a chemically conjugated bispecific molecule, made up of the the fab' fragments of 14.1 and 3F2 (anti-HER2), used as positive control.

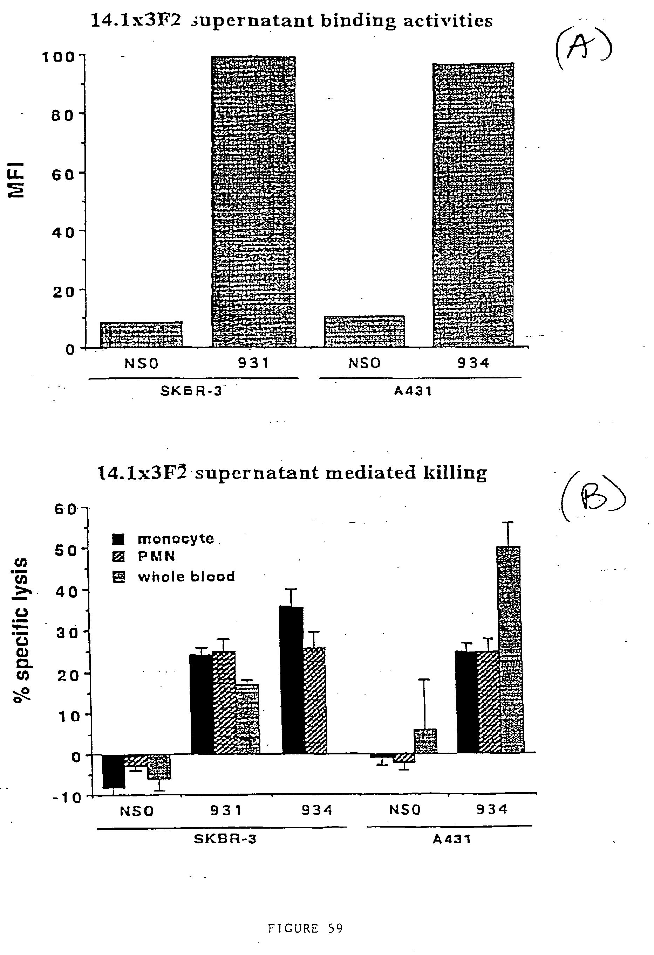

Figure 59(a) is a bar graph showing screening (binding) assays for fusion proteins 931 and 934 expressed in transfectoma supernatants as measured by flow cytometry analysis. Transfected (931 & 934) and untransfected (NSO) supernatant was incubated with either SKBR-3 or A431 tumor cell lines. Binding of fusion protein was detected by staining with goat anti-human IgG fab-2 conjugated to phycoerythrin. Figure 59(b) is a bar grapg showing ADCC analysis of fusion proteins 931 and 934. Chromium release assays were done with an incubation period of 16-18 h and an effector (monocytes, PMN) to target (SKBR-3, A431) ration of 100:1. Tumor cell killing was detected using transfected (931 & 934) and untransfected (NSO) supernatant to mediate specific lysis.

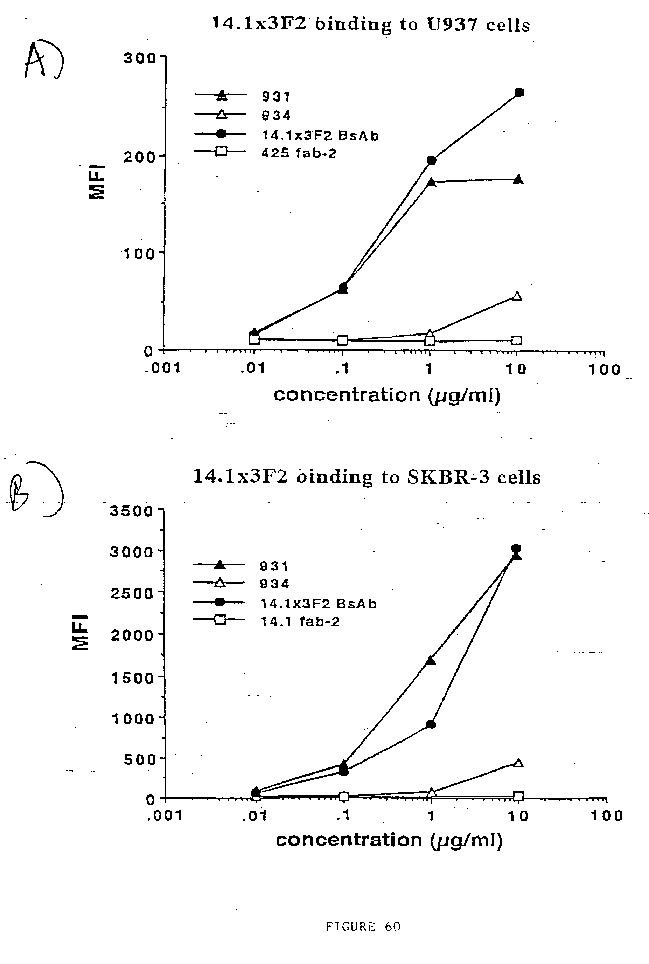

Figure 60(a) is a graph showing binding (as measured by flow cytometry) of single-chain fusion proteins 931 and 934 to U937 cells. An fab' fragment of 425, an anti-EGF-R mAb which does not bind U937 cells, was used as a negative control. The chemically linked bispecific (14.1 x 3F2) shown in Figure 58 was used as a positive control. Figure 60(a) is a graph showing binding (as measured by flow cytometry) of single-chain fusion proteins 931 and 934 to SKBR-3 cells. 14.1 fab-2 was used as a negative control. The chemically linked bispecific (14.1 x 3F2) shown in Figure 58 was again used as a positive control.

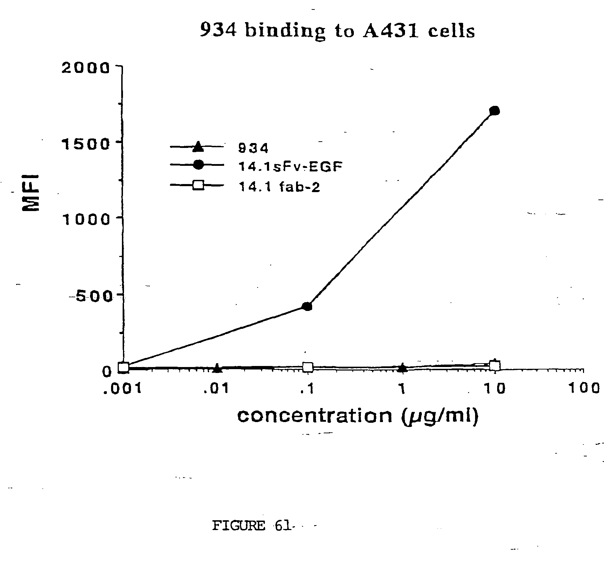

Figure 61 is a graph showing binding (as measured by flow cytometry) of single-chain fusion protein 934 to A431 cells. This experiment was done the same way as shown in Figure 60, except that A431 tumor cells which over-express the EGF-R were used. A fusion protein consisting of the sFv fragment of 14.1 fused to EGF was used as a positive control, and 14.1 fab-2 as a negative control.

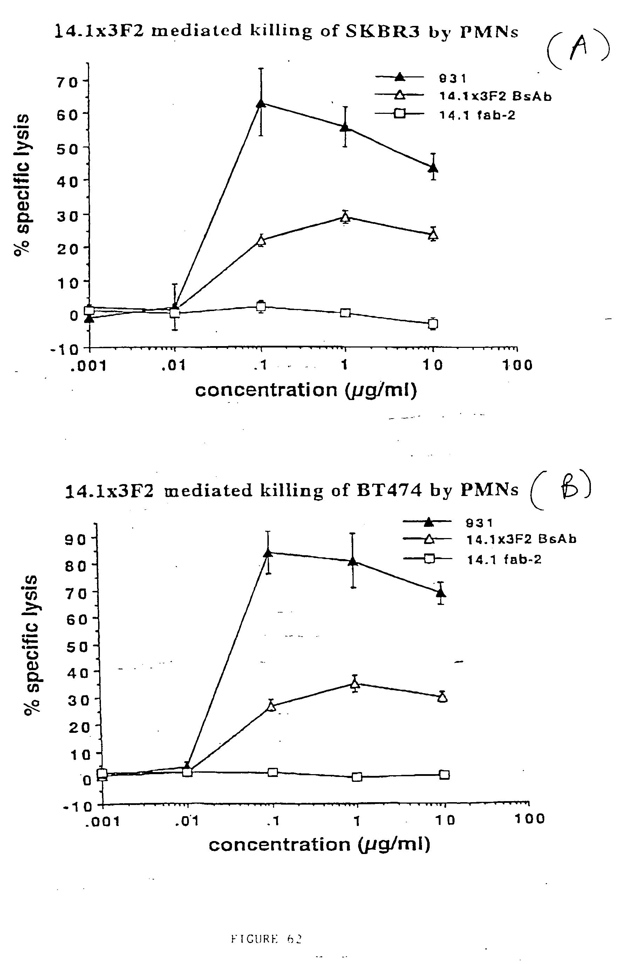

Figure 62 is a graph showing PMN (effector cell)-mediated cytotoxicity of SKBR3 (Figure 62(a)) and BT474 (Figure 62(b)) tumor cells by single-chain fusion protein 931. Chromium release assays were done with an incubation period of 16-18 h and an effector to target ration of 100:1. 14.1 fab-2 was used as negative control in each experiment.

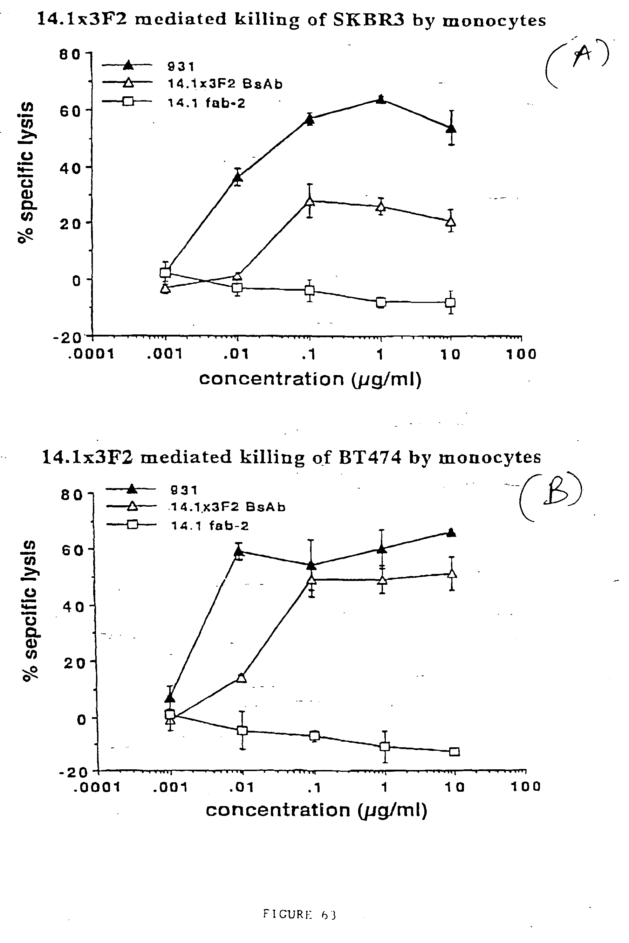

Figure 63 is a graph showing monocyte (effector cell)-mediated cytotoxicity of SKBR3 (Figure 62(a)) and BT474 (Figure 62(b)) tumor cells by single-chain fusion protein 931. Chromium release assays were done with an incubation period of 16-18 h and an effector to target ration of 100:1. 14.1 fab-2 was used as negative control in each experiment.

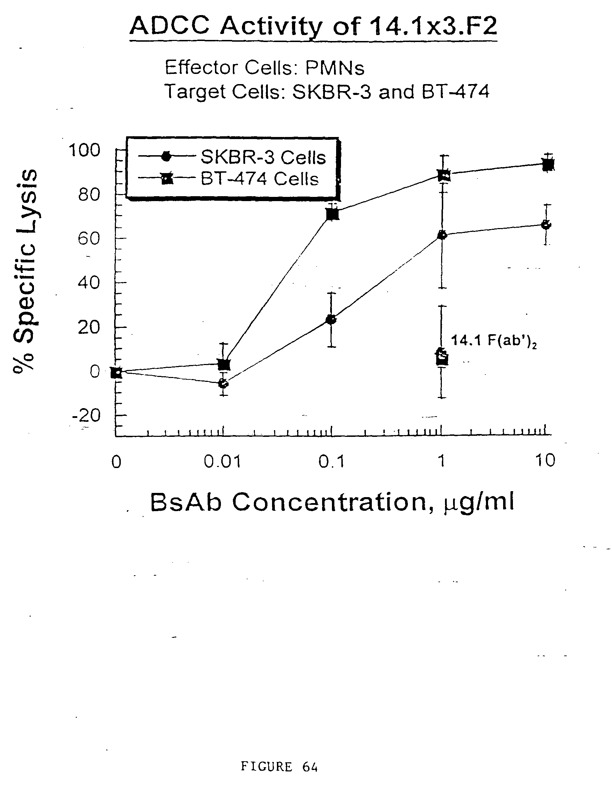

Figure 64 is a graph depicting the antibody dependent cytolysis of SKBR-3 and BT-474 tumor cells by polymorphonuclear cells mediated by a human anti-HER2/neu × anti-CD89 bispecific molecule (14.1 × 3.F2), as measured by ⁵¹Cr release.

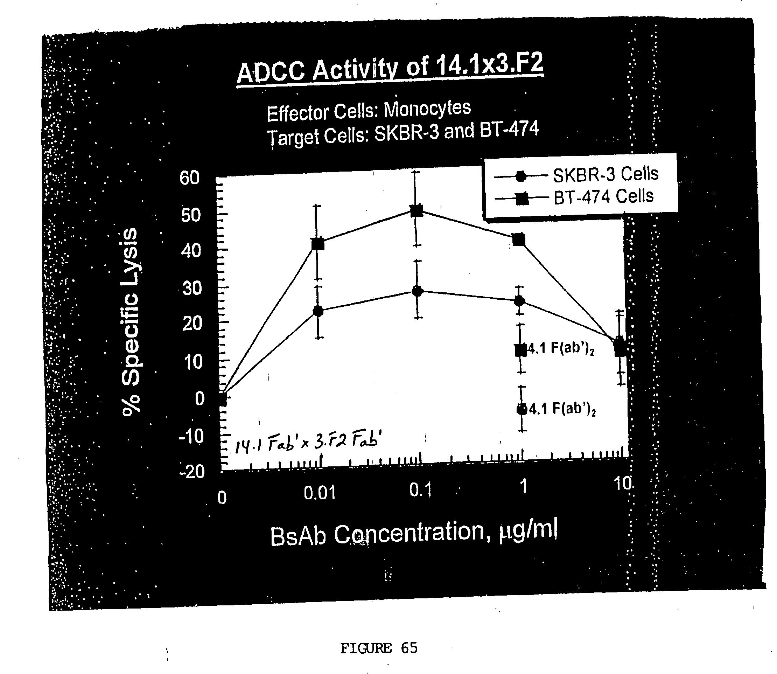

Figure 65 is a graph depicting the antibody dependent cytolysis of SKBR-3 and BT-474 tumor cells by monocytes mediated by a human anti-HER2/neu × anti-CD89 bispecific molecule (14.1 × 3.F2), as measured by ⁵¹Cr release.

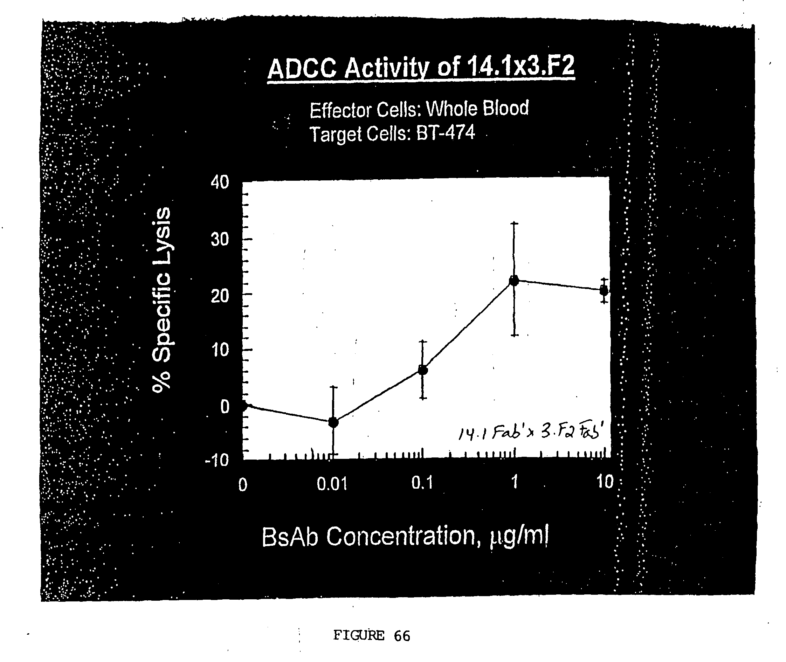

Figure 66 is a graph depicting the antibody dependent cytolysis of BT-474 tumor cells by whole blood mediated by a human anti-HER2/neu × anti-CD89 bispecific molecule (14.1 × 3.F2), as measured by ⁵¹Cr release.

### Detailed Description

### Multispecific molecules

Described herein are recombinant and chemically synthesized multispecific molecules which target immune cells. The molecules are "multispecific" because they bind to multiple (two or more), distinct targets, one of which is a molecule on the surface of an immune cell. Multispecific molecules are described which are comprised of at least one portion which binds to a molecule on an effector cell, preferably an Fc receptor, and at least one portion (e.g., two, three, four or more portions) which binds to a different target, such as an antigen on a tumor cell or a pathogen. Multispecific molecules further include antigen "multimer complexes" comprised of multiple (i.e., two or more) portions which bind to a molecule on an antigen presenting cell (APC), such as an Fc receptor, linked to one or more antigens. These multimer complexes target antigens, such as self-antigens, to APCs to induce and/or enhance internalization (endocytosis), processing and/or presentation of the antigen by the APC. Therefore, these molecules can be used to induce or enhance an immune response either *in vivo* or *in vitro* against a normally non-immunogenic protein, such as a self-antigen.

Further, multispecific molecules include human or humanized (chimeric) antibodies or antibody fragments which bind to an Fc receptor, typically FcδR (e.g., FcδRI) or FcαR. The multispecific molecules additionally include one or more binding specificities, such as a ligand and/or another human antibody or antibody fragment, which binds to an antigen on a different target cell (e.g., a tumor cell). Such multispecific molecules may be chemically linked or genetically expressed as fusion proteins. Moreover, because they target effector cells, e.g., via FcδRI or FcαR, they can be used to induce effector cell (e.g., PMN, macrophage and monocyte)-mediated killing of target (e.g., tumor) cells or, if linked to an antigen, can be used to enhance antigen presentation by antigen presenting (AP) effector cells (e.g., dendritic cells).

As used herein to describe the multispecific molecules the term a "portion which binds to" is used interchangeably with the term a "binding specificity for." Both of these terms refer to regions of the multispecific molecules which bind to a target epitope. As described in detail below, these portions or binding specificities include any compound capable of binding to a target epitope including, but not limited to antibodies, antibody fragments (e.g., an Fab, Fab', F(ab')₂, Fv, or a single chain Fv) and mimetics thereof (e.g., peptide, chemical and organic mimetics which "mimic" the binding of an antibody or antibody fragment). Suitable antibodies and antibody fragments include, but are not limited to murine, humanized (chimeric), single chain, and human monoclonal antibodies and fragments thereof. In a particular example:, the binding specificities include one or more antibodies selected from H22 (ATCC Deposit No.CRL 11177), M22 (ATCC Deposit No. HB 12147), M32.2 (ATCC Deposit No HB 9469), and antigen binding fragments thereof, each of which binds to FcγRI (CD64). Humanized antibody H22, and its murine equivalent, M22, provide the advantage of binding to FcγRI outside the natural ligand (IgG) binding site and, thus, are not blocked or competed off by endogenous ligand, when administered *in vivo.* In other particular examples, the binding specificities include one or more antibodies selected from human monoclonal antibody 14.1 which binds to FcαR (CD89) and human monoclonal antibody 3.F2 which binds to HER2.

The term "antigen-binding portion" of an antibody (or simply "antibody portion"), as used herein, refers to one or more fragments of an antibody that retain the ability to specifically bind to an antigen (e.g., an Fc receptor). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

### I. Multispecific Molecules Comprising Antigen Multimer Complexes Which Target Antigen Presenting Cells (APCs)

Antigen multimer complexes include multiple portions or binding specificities which target a component on the surface of an antigen presenting cell (APC cell surface component), linked to one or more antigens. The multiple binding specificities can bind to the same or different epitopes on the APC cell surface component, or to different APC cell surface components. In one example, the multimer complex includes at least three binding specificities for APC cell surface component(s). Suitable components on APCs for targeting include those which, when bound by the binding specificity, mediate its internalization, so that the antigen linked to the binding specificity is efficiently internalized and presented by the APC.

As used herein, the term "antigen presenting cell" or "APC" refers to an immune cell capable of internalizing and processing an antigen, so that antigenic determinants are presented on the surface of the cell as MHC-complexes, in a manner capable of being recognized by the immune system (e.g., class II-MHC restricted helper T lymphocytes). The two requisite properties that allow a cell to function as an APC are the ability to process endocytosed antigens and the expression of class II MHC gene products. The best defined APCs for helper T cells include mononuclear phagocytes (e.g., macrophages), B lymphocytes, dendritic cells, Langerhans cells of the skin and, in humans, endothelial cells.

The APC cell surface component targeted by the binding specificity can be an Fc receptor, typically FcγR. Therefore, in a particular example, the multimer complex includes multiple binding specificities (e.g., two or more, preferably three or more) which target different epitopes on FcγRI. Alternatively, the multimer can include multiple binding specificities which target the same epitope on FcγRI. However, other suitable APC cell surface components also can be targeted. Such components can be identified, for example, by immunizing an animal (e.g., mice transgenic for human FcγRI) with APCs and determining cell surface components bound by sera taken from the animal, e.g., using standard (e.g., Western blot) assays. These APC cell surface components can then be tested for their ability to internalize a compound which binds to the component.

Accordingly, in one example, the multimer complex includes contains at least one antibody or fragment thereof (e.g., an Fab, Fab', F(ab')₂, Fv, or a single chain Fv) which binds to an Fc receptor, such as a human IgG receptor, e.g., an Fc-gamma receptor (FcγR), such as FcγRI (CD64), FcγRII(CD32), and FcγRIII (CD 16). A preferred Fcγ receptor is the high affinity Fcγ receptor, FcγRI. However, other Fc receptors, such as human IgA receptors (e.g FcαRI) also can be targeted. The Fc receptor is located on the surface of an APC, e.g., a monocyte or macrophage. In a particular example, the multimer complex binds to an Fc receptor at a site which is distinct from the immunoglobulin (e.g., IgG or IgA) binding site of the receptor. Therefore, the binding of the multimer complex is not blocked by physiological levels of immunoglobulins. Humanized anti- Fc R monoclonal antibodies are described in PCT application WO 94/10332 and U.S. Patent No. 4,954,617.

As described in the Examples herein, particular humanized and murine monoclonal anti- FcγRI antibodies and antibody fragments suitable for use in the antigen multimer complexes include, but are not limited to, humanized antibody H22 (ATCC Deposit No.CRL11177), its murine counterpart, M22 (ATCC Deposit No. HB12147), and murine M32.2 (ATCC Deposit No. HB 9469), as well as antigen binding fragments of these antibodies.

Multimer complexes which target APCs further include one or more antigens. The term "antigen", as used herein, refers to any molecule (e.g., protein, peptide, carbohydrate etc.) which is capable of being recognized by an immune cell (i.e., eliciting an immune response, such as a T cell-mediated immune response). Antigens include "self antigens" or "autoantigens" which are normally present in a host, and which normally do not elicit an immune response from the host (because the immune system recognizes them as "self and not "foreign"). In certain disorders, antigens which are not normally present in a host and, therefore, should elicit an immune response by a host's immune system, do not. In other words, they "escape" recognition by a host's immune system. Such antigens include, for example; certain tumor antigens and pathogenic (e.g., viral and bacterial) antigens. In these situations, it may be desirable to modify the antigen in a way that induces or enhances its recognition by immune cells, so that the antigen or cell/organism which produces the antigen is eliminated from the host. In other words, the antigen is modified so that it is no longer recognized by immune cells as a "self antigen".

Antigen multimer complexes can be prepared by chemically linking one or more antigens to multiple (two or more) binding specificities for a component on an APC, as described herein, using standard cross-linking reagents and conjugation protocols well known in the art. Alternatively, the antigen multimer complexes can be recombinantly produced as a single fusion protein, also as described herein.

Accordingly, in yet another example is a nucleic acid encoding an antigen multimer complex. Typically, the nucleic acid is operatively linked, within an expression vector, to a promoter and other genetic regulatory sequences which control expression of the multimer complex. A nucleic acid is "operably linked" when it is placed into a functional relationship with another nucleic acid sequence. For instance, a promoter or enhancer is operably linked to a coding sequence if it affects the transcription of the sequence. With respect to transcription regulatory sequences, operably linked means that the DNA sequences being linked are contiguous and, where necessary to join two protein coding regions, contiguous and in reading frame. For switch sequences, operably linked indicates that the sequences are capable of effecting switch recombination.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, other forms of expression vectors, such as viral vectors (e.g., replication defective retroviruses, adenoviruses and adeno-associated viruses), which serve equivalent functions, are also described

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

Antigen multimer complexes can be used to induce or enhance internalization, processing and presentation of an antigen by an immune cell (e.g., an APC). Accordingly, further described is a method of inducing or enhancing an immune response against an antigen in a subject by administering to the subject an effective amount of an antigen multimer complex. For example, an antigen multimer complex can be administered to induce an immune response against a self-antigen (including tumor antigens not recognized by an immune system), or to enhance an immune response against an antigen, such as a tumor antigen or a component of a pathogen. As such, the antigen multimer complexes also can be used as vaccines to immunize a host.

### II. Multispecific Molecules which Target Effector cells

Multispecific molecules include molecules designed to target effector cells, for example, to cause effector cell-mediated elimination of a target cell, pathogen, allergen or other entity. A multispecific molecule comprised of at least one portion which binds to a component on an effector cell, typically an Fc receptor, and at least one portion (e.g., two, three, four or more portions) which binds to a different target, such as an antigen on a tumor cell or a pathogen, is described.

An "effector cell", as used herein refers to an immune cell. Specific effector cells express specific Fc receptors and carry out specific immune functions. For example, monocytes, macrophages, neutrophils and dendritic cells, which express FcγRI and FcαR, are involved in both specific killing of target cells and presenting antigens to other components of the immune system. Thus, FcγRI and FcαR, because they are expressed on each of these effector cell types, are preferred trigger targets.

Moreover, the expression of a particular FcR on an effector cell can be regulated by humoral factors such as cytokines. For example, expression of FcγRI has been found to be up-regulated by interferon gamma (IFN-γ). This enhanced expression increases the cytotoxic activity of FcγRI cells against targets.

Multispecific molecules which target effector cells have one or more binding specificities or portions which bind to a component on an effector cell, such as an Fc receptor. In one example, the binding specificity is provided by an antibody or antibody fragment (e.g., Fab' or single chain Fv), as previously described herein. In a particular example, the antibody or antibody fragment is human or is "humanized" (i.e. derived from a human antibody, but having at least a portion of a complementarity determining region (CDR) derived from a non-human antibody, the portion being selected to provide specificity of the humanized antibody for e.g., a human Fc receptor). The humanized antibody has CDRs derived from a non-human antibody and the remaining portions of the antibody molecule are human.

The antibody may be whole, i.e. having heavy and light chains or any fragment thereof, e.g., Fab or (Fab')₂ fragment. The antibody further may be a light chain or heavy chain dimer, or any minimal fragment thereof such as a Fv or a single chain construct as described in Ladner et al. (U.S. Patent No. 4,946,778, issued August 7, 1990). The humanized antibody or fragment may be any human antibody capable of retaining non-human CDRs. The human antibody can be derived from known proteins NEWM and KOL for heavy chain variable regions (VHs) and REI for Ig kappa chain, variable regions (VKs).

The portion of the non-human CDR inserted into the human antibody is selected to be sufficient for allowing binding of the humanized antibody to the Fc receptor. A sufficient portion may be selected by inserting a portion of the CDR into the human antibody and testing the binding capacity of the created humanized antibody using the enzyme linked immunosorbent assay (ELISA).

All of the CDRs of a particular human antibody may be replaced with at least a portion of a non-human CDR or only some of the CDRs may be replaced with non-human CDRs. It is only necessary to replace the number of CDRs required for binding of the humanized antibody to the Fc receptor. A non-human CDR derived from a murine monoclonal antibody (mab), mab 22, is described in International Patent Application Publication No. WO 94/10332. The mab 22 antibody is specific to the Fc receptor and further is described in U.S. Patent No. 4,954,617, issued September 4, 1988. The humanized mab 22 antibody producing cell line was deposited at the American Type Culture Collection on November 4, 1992 under the designation HA022CL1 and has the accession no. CRL 11177.

An antibody can be humanized by any method, which is capable of replacing at least a portion of a CDR of a human antibody with a CDR derived from a non-human antibody. Winter describes a method which may be used to prepare the humanized antibodies of the present invention (UK Patent Application GB 2188638A, filed on March 26, 1987). The human CDRs may be replaced with non-human CDRs using oligonucleotide site-directed mutagenesis as described in International Patent Application Number: WO 94/10332 entitled, *Humanized Antibodies to Fc Receptors for Immunoglobulin G on Human Mononuclear Phagocytes.*

In addition to an anti-Fc receptor portion, the multispecific molecules can comprise an "anti-target portion", i.e. an antibody, a functional antibody fragment or a ligand that recognizes and binds a pathogen (e.g., virus; bacteria, fungi), a pathogen infected cell, a cancer or tumor cell (e.g., breast, ovarian, prostate, etc.) or other unwanted cell in a subject (e.g., a human or animal) or an antigen or modified form thereof. Additionally, the target portion may comprise or be directed against an antigen. One example contains an antigen that can be used to stimulate the immune system, for example, in instances of chronic infection, to deplete antigen in the circulation, and to treat tumors. A particular example has an antigen that is attached to a multivalent molecule containing an anti-FcR antibody.

In another example, the multispecific molecule contains a ligand. The ligand can be any ligand that interacts with a molecule. In one example, the ligand binds a protein, e.g., a surface protein on a target cell, such as a cancer cell. Ligands include ligands to receptors, such as growth or differentiation factors. For example, a multivalent molecule can comprise an epidermal growth factor, e.g., an epidermal growth factor receptor. In another example, the ligand is a small peptide, such as bombesin, gastrin-releasing peptide (GRP), litorin, neuromedin B, or neuromedin C. The sequences of the peptides can be found, e.g., in US Patent No. 5,217,955. The ligand can also be a modified form of any of these peptides. The modification can increase binding to the receptor, decrease binding, or not affect the binding to a receptor. The modification of the ligand can also transform an agonist into an antagonist, such that the ligand inhibit rather than stimulate cell proliferation. The modification of the ligand can be an addition, a deletion, a substitution, or a modification of at least one amino acid.

A multivalent or bispecific molecule may comprise an antigen. As used herein, the term "antigen" means any natural or synthetic immunogenic substance, a fragment or portion of an immunogenic substance, a peptidic epitope, or a hapten. The term "antigen" also includes substances which are non-immunogenic in uncomplexed form, but are immunogenic when complexed. The term "uncomplexed" includes substances which are not linked to form a molecular complex. The term "complexed" includes substances which are linked to form a molecular complex

In one example, a bi- or multispecific molecule is employed to target an antigen to the cell to enhance the processes of internalization and presentation by these cells, and utlimately, to stimulate an immune response therein. In a specific sample, the bispecific binding agent specifically binds the antigen (either directly, to an epitope of the antigen, or indirectly, to an epitope attached to the antigen) and, at the same time, binds a surface receptor of an antigen-presenting cell which can internalize antigen for processing and presentation. In another example, the antigen is linked to the multi- or bispecific molecule and at the same time binds a surface receptor of an antigen-presenting cell. The receptor-binding component of these bi- or multispecific molecule (and thus the bi- or multispecific molecule, itself) binds the receptor of the antigen-presenting cell. In some instances, binding of the molecule occurs without the molecule substantially being blocked by the natural ligand for the receptor. As a result, targeting of the antigen to the receptor will not be prevented by physiological levels of the ligand and the targeted receptor will remain capable of binding the ligand and functioning.

One type of antigen can be an allergen. An "allergen" refers to a substance that can induce an allergic or asthmatic response in a susceptible subject. The list of allergens is enormous and can include pollens, insect venoms, animal dander dust, fungal spores and drugs (e.g. penicillin). Examples of natural, animal and plant allergens include proteins specific to the following genuses: *Canine (Canis familiaris); Dermatophagoides (e.g. Dermatophagoides farinae); Felis (Felis domesticus); Ambrosia* (*Ambrosia artemiisfolia; Lolium* (e.g. *Lolium perenne or -Lolium multiflorum); Cryptomeria (Cryptomeria japonica); Alternaria (Alternaria alternata); Alder; Alnus (Alnus gultinosa); Betula (Betula verrucosa); Quercus (Quercus alba); Olea (Olea europa); Artemisia (Artemisia- vulgaris); Plantago* (e.g. *Plantago lanceolata); Parietaria* (e.g. *Parietaria officinalis* or *Parietaria judaica); Blattella* (e.g. *Blattella germanica); Apis* (e.g. *Apis multiflorum); Cupressus* (e.g. *Cupressus sempervirens, Cupressus arizonica* and *Cupressus macrocarpa); Juniperus* (e.g. *Juniperus sabinoides, Juniperus virginiana, Juniperus communis* and *Juniperus ashei) Thuya* (e.g. *Thuya orientalis); Chamaecyparis* (e.g. *Chamaecyparis obtusa); Periplaneta* (e.g. *Periplaneta americana)*; *Agropyron* (e.g. *Agropyron repens); Secale* (e.g. *Secale cereale); Triticum* (e.g. *Triticum aestivum); Dactylis* (e.g. *Dactylis glomerata); Festuca (e.g. Festuca elatior); Poa* (e.g. *Poa pratensis* or *Poa compressa); Avena* (e.g. *Avena sativa); Holcus* (e.g. *Holcus lanatus); Anthoxanthum (e.g. Anthoxanthum odoratum); Arrhenatherum* (e.g. *Arrhenatherum elatius); Agrostis* (e.g. *Agrostis alba); Phleum (e.g. Phleum pratense); Phalaris* (e.g. *Phalaris arundinacea); Paspalum* (e.g. *Paspalum notatum); Sorghum* (e.g. *Sorghum halepensis);* and Bromus (e.g. *Bromus inermis).*

Many allergens are found in airborne pollens of ragweed, grasses, or trees, or in fungi, animals, house dust, or foods. As a class, they are relatively resistant to proteolytic digestion. Preferable allergens are those which bind to IgE on mast cells and basophils, thereby causing a type I anaphylaxis hypersensitivity reaction. When at least one specificity of the multivalent agent is for an epitope of the high affinity Fc receptor that is outside the ligand binding domain for IgG, this bispecific binding agent can decrease hypersensitivity in a subject. This is accomplished when the bispecific binding agent competes for an IgE-binding allergen before the allergen binds to IgE on a mast cell or basophil, thereby reducing the possibility of a type I hypersensitivity reaction. In addition, as a result of directing allergen to FcγR, a state of T cell tolerance to the allergen may be induced which interferes with IgE-mediated type I reactions. Tolerance can be accomplished by inducing IgG which competes with IgE for binding to allergen using doses of allergen substantially lower than those currently used.

In some cases, it may be desirable to couple a substance which is weakly antigenic or nonantigenic in its own right (such as a hapten) to a carrier molecule, such as a large immunogenic protein (e.g., a bacterial toxin) for administration. In these instances, the bispecific binding reagent can be made to bind an epitope of the carrier to which the substance is coupled, rather than an epitope of the substance itself.

The antigen that can be linked either directly, or indirectly, to bispecific molecule of the invention can be soluble or particulate; it may carry B cell epitopes, T cell epitopes or both. The antigen can be bacterial, viral or parasitic in origin. Often, the antigen will comprise a component of the surface structure of a pathogenic organism. For example, the antigen can comprise a viral surface structure such as an envelope glycoprotein of human immunodeficiency virus (HIV) or the surface antigen of hepatitis virus. In addition, the antigen can be associated with a diseased cell, such as a tumor cell, against which an immune response may be raised for treatment of the disease. The antigen can comprise a tumor-specific or tumor-associated antigen, such as the Her-2/new proto-oncogene product which is expressed on human breast and ovarian cancer cells (Slamon et al. (1989) Science 244:707).

The cells of a subject can be exposed *in vitro* or *in vivo* to the multivalent molecules. The multivalent molecule can be used to target an antigen to antigen-presenting cells in culture. Immunocompetent cells are separated and purified from patient blood. The cells are then exposed to a multivalent molecule comprising the antigen or the cells can be exposed to the antigen together with a multivalent molecule having a binding specificity for the antigen. Targeted antigen-presenting cells will process the antigen and present fragments on their surface. After stimulation, the cells can be returned to the patient.

The method can be used to enhance or reinforce the immune response to an antigen. For example, the method is valuable for the treatment of chronic infections, such as hepatitis and AIDS, where the unaided immune system is unable to overcome the infection. It can also be used in the treatment of the acute stages of infection when reinforcement of immune response against the invading organism may be necessary.

The method can be used to reduce the dose of antigen required to obtain a protective or therapeutic immune response or in instances when the host does not respond or responds minimally to the antigen. Although generally desirable, the lowering of effective dose can be especially desirable when the antigen is toxic to the host such as is the case for allergies. Methods and uses for using bi- or multispecific molecules comprising an antigen or comprising an ligand, e.g., an antibody interacting with an antigen, are further described in the published PCT application PCT/US91/07283.

In another example, a multispecific molecule comprises an antigen that has been modified, such that its-effect on T cell activation is modified upon presentation of the modified antigen to the T cell by-an antigen presenting cell. Allan et al. have in fact shown that substitution of one or more amino acids of a peptide that stimulates T cells, e.g., stimulates T cell proliferation, can result in an antigen which fails to stimulate the T cell or which induces anergy in the T cell. Such modified peptides are termed Altered Peptide Ligands (APL). Accordingly, such APLs can be linked to bispecific or multispecific molecules having at least one binding specificity for the FcγRI. Upon phagocytosis of these molecules by antigen presenting cells and presentation to T cells, the proliferation of the T cells may be inhibited or anergized. Accordingly, administration to a subject of a multispecific molecule comprising (a) at least one altered peptide of an antigen which normally stimulates T cells, but which upon modification induces anergy of the T cells, and (b) at least one anti-FcγRI antibody will result in induction of tolerance of the subject to the antigen. Thus, such multi- or bispecific molecules can be used to tolerize a subject to a variety of antigens, e.g., auto-antigens. Thus, depending on the antigen used, the methods provide methods for increasing an immune response, i.e., by using an antigen which stimulates T cells, and methods for reducing an immune response, either by inhibiting T cell stimulation or by inducing anergy of the T cells.

The multispecific, multivalent molecules may also include an "anti-enhancement factor (anti-EF) portion". The "anti-enhancement factor portion" can be an antibody, functional antibody fragment or a ligand that binds to an antigen and thereby results in an enhancement of the effect of the anti-F_{c} receptor portion or the anti-target portion. The "anti-enhancement factor portion" can bind an F_{c} receptor or a target. A multivalent molecule comprising an anti-target portion that binds to one target cell antigen and an anti-enhancement factor portion that binds to a different target antigen is particularly useful where the target cell undergoes antigen modulation or antigenic variation (e.g., as has been described for certain parasites (such as trypanosomes). Alternatively, the anti-enhancement factor portion can bind an entity that is different from the entity to which the anti-target or anti-F_{c} receptor portion binds. For example, the anti-enhancement factor portion can bind a cytotoxic T-cell (e.g. via CD2, CD3, CD8, CD28, CD4, CD40, ICAM-1 or other immune cell that results in an increased immune response against the target).

### Methods for Making Multispecific Molecules

The multispecific molecules described above can be made by a number of methods. For example, both specificities can be encoded in the same vector and expressed and assembled in the same host cell. This method is particularly useful where the multi-specific molecule is a (ligand) x (fab) fusion protein as described in Example 2 below. A bispecific molecule can be a single chain bispecific molecule, such as a single chain bispecific antibody, a single chain bispecific molecule comprising one single chain antibody and a ligand, or a single chain bispecific molecule comprising two ligands. Multivalent molecules can also be single chain molecules or may comprise at least two single chain molecules. Methods for preparing bi- or multivalent antibodies are for example described in U.S. Patent Number 5,260,203; U.S. Patent Number 5,455,030; U.S. Patent Number 4,881,175; U.S. Patent Number 5,132,405; U.S. Patent Number 5,091,513; U.S. Patent Number 5,476,786; U.S. Patent Number 5,013,653; U.S. Patent Number 5,258,498; and U.S. Patent Number 5,482,858.

Binding of the single chain molecules to their specific targets can be confirmed by bispecific ELISA as described in the Examples herein.

Alternatively, each specificity of a multispecific molecule can be generated separately and the resulting proteins or peptides conjugated to one another. For example, two humanized antibodies can be conjugated via sulfhydryl bonding of the C-terminus hinge regions of the two heavy chains. In a particular example, the hinge region is modified to contain an odd number of sulfhydryl residues, preferably one, prior to conjugation.

The bispecific molecules can be prepared by conjugating the anti-FcR and anti-target portions using methods described in the following Example or those well-known in the art. For example, a variety of coupling or cross-linking agents can be used for covalent conjugation. Examples of cross-linking agents include protein A, carbodiimide, N-succinimidyl-S-acetyl-thioacetate (SATA), N-succinimidyl-3-(2-pyridyldithio)propionate (SPDP), and sulfosuccinimidyl 4-(N-maleimidomethyl) cyclohaxane-1-carboxylate (sulfo-SMCC) (see e.g., Karpovsky et al. (1984) J. Exp. Med. 160:1686; Liu, MA et al. (1985) Proc. Natl. Acad. Sci. USA 82:8648). Other methods include those described by Paulus (Behring Ins. Mitt. (1985) No. 78, 118-132); Brennan et al. (Science (1985) 229:81-83), and Glennie et al. (J. Immunol. (1987) 139: 2367-2375). Preferred conjugating agents are SATA and sulfo-SMCC, both available from Pierce Chemical Co. (Rockford, IL).

In the case of using antibodies as binding specificities within the multispecific molecules, preferred antibodies include human monoclonal and humanized antibodies (including fragments, e.g., Fab' and single chain Fv) thereof. Methods for making humanized antibodies are known in the art and are described in detail in the examples below. Methods for making fully human monoclonal antibodies also are known in the art. These antibodies can be produced by a variety of techniques, including conventional monoclonal antibody methodology e.g., the standard somatic cell hybridization technique of Kohler and Milstein, Nature 256: 495 (1975). Although somatic cell hybridization procedures are preferred, in principle, other techniques for producing monoclonal antibody can be employed e.g., viral or oncogenic transformation of B lymphocytes.

The preferred animal system for preparing hybridomas is the murine system. Hybridoma production in the mouse is a very well-established procedure. Immunization protocols and techniques for isolation of immunized splenocytes for fusion are known in the art. Fusion partners (e.g., murine myeloma cells) and fusion procedures are also known.

In one example, human monoclonal antibodies are generated using transgenic mice carrying the parts of the human immune system rather than the mouse system. These transgenic mice, referred to herein as "HuMAb" mice, contain a human immunoblobulin gene miniloci that encodes unrearranged human heavy (µ and γ) and κ light chain immunoglobulin sequences, together with targeted mutations that inactivate the endogenous µ and κ chain loci (Lonberg, N. et al. (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg, N. *et al*. (1994), *supra;* reviewed in Lonberg, N. (1994) Handbook of Experimental Pharmacology 113:49-101; Lonberg, N. and Huszar, D. (1995) Intern. Rev. Immunol. Vol. 13: 65-93, and Harding, F. and Lonberg, N. (1995) Ann. N. Y. Acad. Sci 764:536-546). The preparation of HuMab mice is described in detail Section II below and in Taylor, L. et al. (1992) Nucleic Acids Research 20:6287-6295; Chen, J. et al. (1993) International Immunology 5: 647-656; Tuaillon et al. (1993) Proc. Natl. Acad Sci USA 90:3720-3724; Choi et al. (1993) Nature Genetics 4:117-123; Chen, J. et al. (1993) EMBO J. 12: 821-830; Tuaillon et al. (1994) J. Immunol. 152:2912-2920; Lonberg et al., (1994) Nature 368(6474): 856-859; Lonberg, N. (1994) Handbook of Experimental Pharmacology 113 :49-101; Taylor, L. et al. (1994) International Immunology 6: 579-591; Lonberg, N. and Huszar, D. (1995) Intern. Rev. Immunol. Vol. 13: 65-93; Harding, F. and Lonberg, N. (1995) Ann. N. Y. Acad Sci 764:536-546; Fishwild, D. et al. (1996) Nature Biotechnology 14: 845-851. See further, U.S. Patent Nos. 5,545,806 and 5,625,825 both to Lonberg and Kay, and GenPharm International; U.S. Patent No. 5,545,807 to Surani *et al.;* International Publication Nos. WO 98/24884, published on June 11, 1998; WO 94/25585, published November 10, 1994; WO 93/1227, published June 24, 1993; WO 92/22645, published December 23, 1992; WO 92/03918, published March 19, 1992. Alternatively, the HCO12 transgenic mice described in Example 2, can be used to generate fully human monoclonal antibodies.

To generate fully human monoclonal antibodies, mice can be immunized with a purified or enriched preparation of the desired immunogen and/or cells expressing the desired immunogen, as described by Lonberg, N. et al. (1994) Nature 368(6474): 856-859; Fishwild, D. et al. (1996) Nature Biotechnology 14: 845-851 and WO 98/24884. Preferably, the mice will be 6-16 weeks of age upon the first infusion. For example, a purified or enriched preparation (5-20 µg) of HER2/neu antigen can be used to immunize HuMab mice intraperitoneally. In the event that immunizations using a purified or enriched preparation of HER2/neu antigen do not result in antibodies, mice can also be immunized with cells expressing HER2/neu, e.g., a tumor cell line, to promote immune responses.

Cumulative experience with various antigens has shown that the HuMAb transgenic mice respond best when initially immunized intraperitoneally (IP) with antigen in complete Freund's adjuvant, followed by every other week IP immunizations (up to a total of 6) with antigen in incomplete Freund's adjuvant. The immune response can be monitored over the course of the immunization protocol with plasma samples being obtained by-retroorbital bleeds. The plasma can be screened by ELISA (as described below), and mice with sufficient titers of human immunoglobulin against the desired immunogen can be used for fusions. Mice can be boosted intravenously with antigen 3 days before sacrifice and removal of the spleen. It is expected that 2-3 fusions for each antigen may need to be performed. Six mice will be immunized for each antigen. For example, a total of twelve HuMAb mice of the HC07 and HC012 strains can be immunized.

The mouse splenocytes can then be isolated and fused with PEG to a mouse myeloma cell line based upon standard protocols. The resulting hybridomas can then be screened for the production of antigen-specific antibodies. For example, single cell suspensions of splenic lymphocytes from immunized mice are fused to one-sixth the number of P3X63-Ag8.653 nonsecreting mouse myeloma cells (ATCC, CRL 1580) with 50% PEG. Cells are plated at approximately 2 x 10⁵ in flat bottom microtiter plate, followed by a two week incubation in selective medium containing-20% fetal Clone Serum, 18% "653" conditioned media, 5% origen (IGEN), 4 mM L-glutamine, 1 mM L~glutamine, 1 mM sodium pyruvate, 5mM HEPES, 0.055 mM 2-mercaptoethanol, 50 units/ml penicillin, 50 mg/ml streptomycin, 50 mg/ml gentamycin and 1X HAT (Sigma; the HAT is added 24 hours after the fusion). After two weeks, cells are cultured in medium in which the HAT is replaced with HT. Individual wells are then screened by ELISA for human monoclonal IgM and IgG antibodies against the desired immunogen. Once extensive hybridoma growth occurs, medium is observed usually after 10-14 days. The antibody secreting hybridomas are replated, screened again, and if still positive for human IgG monoclonal antibodies against the desired immunogen, can be subcloned at least twice by limiting dilution. The stable subclones are then cultured *in vitro* to generate small amounts of antibody in tissue culture medium for characterization.

To characterize binding of human monoclonal antibodies, sera from immunized mice can be tested, for example, by ELISA. Briefly, microtiter plates are coated with the desired immunogen (purified) at approximately 0.25 µg/ml in PBS, and then blocked with 5% bovine serum albumin in PBS. Dilutions of plasma from mice immunized with the desired immunogen are added to each well and incubated for 1-2 hours at 37°C. The plates are washed with PBS/Tween and then incubated with a goat-anti-human IgG Fc-specific polyclonal reagent conjugated to alkaline phosphatase for 1 hour at 37°C. After washing, the plates are developed with pNPP substrate (1 mg/ml), and analyzed at OD of 405-650. Preferably, mice which develop the highest titers will be used for fusions.

An ELISA assay as described above can also be used to screen for hybridomas that show positive reactivity with the desired immunogen. Hybridomas that bind with high avidity to the desired immunogen will be subcloned and further characterized. One clone from each hybridoma, which retains the reactivity of the parent cells (by ELISA), can be chosen for making a 5-10 vial cell bank stored at -140°C, and for antibody purification.

To purify human monoclonal antibodies against the desired immunogen, selected hybridomas can be grown in two-liter spinner-flasks for monoclonal antibody purification. Supernatants can be filtered and concentrated before affinity chromatography with protein A-sepharose (Pharmacia, Piscataway, NJ). Eluted IgG can be checked by gel electrophoresis and high performance liquid chromatography to ensure purity. The buffer solution can be exchanged into PBS, and the concentration can be determined by OD₂₈₀ using 1.43 extinction coefficient. The monoclonal antibodies can be aliquoted and stored at -80 °C.

To determine if the selected human monoclonal antibodies bind to unique epitopes on the desired immunogen; each antibody can be biotinylated using commercially available reagents (Pierce, Rockford, IL). Competition studies using unlabeled monoclonal antibodies and biotinylated monoclonal antibodies can be performed using ELISA plates coated with the desired immunogen as described above. Biotinylated mAb binding can be detected with a strep-avidin-alkaline phosphatase probe.

To determine the isotype of purified antibodies, isotype ELISAs can be performed. Wells of microtiter plates can be coated with 10 µg/ml of anti-human Ig overnight at 4°C. After blocking with 5% BSA, the plates are reacted with 10 µg/ml of monoclonal antibodies or purified isotype controls, at ambient temperature for two hours. The wells can then be reacted with either human IgGI or human IgM-specific alkaline phosphatase-conjugated probes. Plates are developed and analyzed as described above.

In order to demonstrate binding of monoclonal antibodies to live cells expressing the desired immunogen, e.g., tumor cell receptor, flow cytometry can be used. Briefly, cell lines expressing the desired immunogen (grown under standard growth conditions) are mixed with various concentrations of monoclonal antibodies in PBS containing 0.1% Tween 80 and 20% mouse serum, and incubated at 37°C for 1 hour. After washing, the cells are reacted with Fluorescein-labeled anti-human IgG antibody under the same conditions as the primary antibody staining. The samples can be analyzed by FACScan instrument using light and side scatter properties to gate on single cells. An alternative assay using fluorescence microscopy may be used (in addition to or instead of) the flow cytometry assay. Cells can be stained exactly as described above and examined by fluorescence microscopy. This method allows visualization of individual cells, but may have diminished sensitivity depending on the density of the antigen.

Human IgGs which bind to the desired immunogen can be further tested for reactivity with the desired immunogen by Western blotting. Briefly, cell extracts from cells expressing the desired immunogen can be prepared and subjected to sodium dodecyl sulfate polyacrylamide gel electrophoresis. After electrophoresis, the separated antigens will be transferred to nitrocellulose membranes, blocked with 20% mouse serum, and probed with the monoclonal antibodies to be tested. Human IgG binding can be detected using anti-human IgG alkaline phophatase and developed with BCIP/NBT substrate tablets (Sigma Chem. Co., St. Louis, MO).

### Therapeutic Uses for Multispecific Molecules

Based on their ability to bind FcR bearing immune cells and specific target cells, a specific multispecific molecule can be administered to a subject to treat or prevent a variety of diseases or conditions, including: cancer (e.g., breast, ovarian, small cell carcinoma of the lung), pathogenic infections (e.g., viral (such as HIV)), protozoan (such as Toxoplasma gondii), fungal (such as candidiasis); an autoimmunity (e.g. immune thrombocytopenia purpura and systemic lupus). The multispecific multivalent can also be administered prophylactically to vaccinate a subject against infection by a target cell.

For use in therapy, an effective amount of an appropriate multispecific molecule can be administered to a subject by any mode that allows the molecules to exert their intended therapeutic effect. Preferred routes of administration include oral and transdermal (e.g., via a patch). Examples of other routes of administration include injection (subcutaneous, intravenous, parenteral, intraperitoneal, intrathecal, etc.). The injection can be in a bolus or a continuous infusion.

A multispecific molecule can be administered in conjunction with a pharmaceutically acceptable carrier. As used herein, the phrase "pharmaceutically acceptable carrier" is intended to include substances that can be coadministered with a multispecific molecule and allows the molecule to perform its intended function. Examples of such carriers include solutions, solvents, dispersion media, delay agents, emulsions and the like. The use of such media for pharmaceutically active substances are well known in the art. Any other conventional carrier suitable for use with the molecules can be used.

The language "effective amount" of a multispecific molecules refers to that amount necessary or sufficient to realize a desired biologic effect. For example, an effective amount of a multispecific molecule, in which the anti-target portion recognizes a pathogenic cell could be that amount necessary to eliminate a tumor, cancer, or bacterial, viral or fungal infection. The effective amount for any particular application can vary depending on such factors as the disease or condition being treated, the particular multispecific molecule being administered, the size of the subject, or the severity of the disease or condition. One of ordinary skill in the art can empirically determine the effective amount of a particular multispecific molecule without necessitating undue experimentation.

The following invention is further illustrated by the following examples, which should not be construed as further limiting.

### Examples

### Example 1: Production of Bispecific Antibody Comprising Murine or Humanized

### Antibodies Specific for an Fc Receptor and an Anti-her 2 neu Antibody

### Monoclonal Antibodies

The anti-FcγRI monoclonal antibodies (mAbs), M22, M32.2 and 197 were purified from hybridoma supernatant by ion exchange chromatography and DZ33, a human anti-HIV-1 IgG1 mAb, was purified from hybridoma supernatant by protein A affinity chromatography (Pharmacia, Piscataway, NJ) and gel filtration. M32.2 was deposited at the American Type Culture Collection, 12301 Parklawn Drive, Rockville, MD 20852, on July 1, 1987 and has been designated with ATCC Accession No. HB9469.

### Cell Lines

The murine myeloma NSO (ECACC 85110503) is a non-Ig synthesizing line and was used for the expression of recombinant mAbs. NSO cells were cultivated in DMEM plus 10% fetal bovine serum (FBS, Gibco, Paisley, U.K.). SKBR-3 is a human breast carcinoma cell line which overexpresses the HER2/*neu* protooncogene (ATCC, Rockville, MD) and was cultivated in Iscove's Modified Dulbecco's Medium (IMDM, Gibco, Grand Island, NY). U937 is a monocytoid cell line that expresses FcγRI and was obtained from ATCC and grown in RPM-1640 plus 10% FBS (Gibco, Grand Island, NY).

### Cloning Murine Immunoglobulin V Region Genes

Cytoplasmic RNA from the murine hybridoma 22 was prepared as described in Favaloro et al. (Favaloro, J., R. Treisman and R. Kamen (1982) Transcription maps of polyoma-specific RNA: analysis by two-dimensional S1 gel mapping. Meth. Enzymol. 65:718). The Ig V region cDNAs were made from RNA via reverse transcription initiated from primers CG1 FOR and CK2FOR as described in International Patent Application Publication Number WO 94/10332 entitled, *Humanized Antibodies to Fc Receptors for Immunoglobulin G on Human Mononuclear Phagocytes.* The cDNA synthesis was performed under standard conditions using 100 U MMLV reverse transcriptase (Life Technologies, Paisley, UK). The V_{H} and V_{K} cDNAs were amplified by PCR, (Orlandi, R., D.H. Güssow, P.T. Jones and G. Winter (1989) (Cloning immunoglobulin variable domains for expression by the polymerase chain reaction), Proc. Natl. Acad. Sci. USA 86:3833), using the cDNA primers in concert with SH2BACK and VK7BACK as described in International Patent Application Publication Number WO 94/10332. Amplified V_{H} and V_{κ} DNA were purified, cloned into M13, and sequenced by the dideoxy method using T7 DNA polymerase (Pharmacia, Piscataway, NJ).

### Construction of Chimeric Antibody Genes

To facilitate cloning of murine V region DNA into expression vectors, restriction sites were placed in close proximity to the termini of both M22 V region genes. For V_{H}, a 5' *Pst*I site and a 3' *Bst*EII site were introduced into a cloned murine V_{H} gene by PCR using VH1BACK and VH1FOR (Id.). For V_{K} a 5' *P vu*II site and a *3' Bgl* II site were introduced into a cloned murine V_{K} gene by PCR using primers VK1BACK and VK1FOR (Id.). In some instances, these primers changed one or more amino acids from those naturally occurring. These V region genes (ChVH and ChVK) were cut with the appropriate restriction enzymes and cloned into M13VHPCR1 and M13VKPCR1 (Id.) which contain an Ig promoter, signal sequence and splice sites. The DNA were excised from M13 as *Hind*III*-Bam*HI fragments and cloned into the expression vectors pSVgpt and pSVhyg containing human IgG1, (Takahashi, N. et al., (1982), Structure of human immunoglobulin gamma genes: implications for evolution of a gene family, Cell, 29:671), and human kappa constant, (Hieter, R.A. et al., (1980) Cloned human and mouse kappa immunoglobulin constant and J region genes conserve homology in functional segments, Cell 22:197), region genomic DNA.

### Construction of Humanized Antibody Genes

Two humanized heavy chains were constructed and were based on human V_{H}s of NEWM, (Poljak, R.J. et al., Amino acid sequence of the VH region of a human mycloma immunoglobulin, (IgG New), Biochemistry, 16:3412), and KOL,(Marquat, M. et al., (1980) Crystallographic refinement and atomic models of the intact immunoglobulin molecule Kol and its antigen-binding fragment at 3.0A and 1.9A resolution, J. Mol. Biol. 141:369. The humanized light chain was derived from the human Bence-Jones protein REI, (Epp, O. et al, (1974) Crystal and molecular structure of a dimer composed of the vandible portion of the Bence-Jones protein REI, Eur. J. Biochem. 45:513), with some framework region (FR) changes. The modifications were made to make the V_{K} domain more typical of human subgroup I, and included replacement of Thr39, Leu104, Gln105 and Thr107 with Lys39, Val104, Glu105 and Lys 107. In addition, Met4 was changed to Leu4 to accommodate a *Pvu*ll restriction site.

DNA containing the NEWM V_{H} and REI V_{K} FRs with irrelevant CDRs were cloned into the vectors M13VHPCR1 and M13VKPCR1 (Favaloro et al. Supra). DNA encoding the KOL V_{H} was constructed by a series of sequential PCRs, using oligodeoxyribonucleotides encoding KOL FR amino acids and irrelevant CDRs. The constructs were then cloned into M13VHPCR1.

Oligodeoxyribonucleotides were synthesized to encode the mAB M22 CDRs which were flanked by nucleotides corresponding to the human FRs. For the humanized V_{H} based on NEWM, the primers included murine FR amino acids Phe27, IIe28 and Arg71 since these were likely to influence antigen binding, (Chothia, C. and A.M. Lesk (1987), Canonical structures for the hypervariable regions of immunoglobulins, J. Mol. Biol., 196:901; Tramontano, A. et al., (1990), Framework residue 71 is a major determinant of the position and conformation of the second hypervariable region in VH domains of immunoglobulins, J. Mol. Biol., 215:175). For the humanized V_{K}, murine amino acid Phe71 was similarly included as a residue capable of affecting affinity, (Foote, J. and G. Winter, (1992), Antibody framework residues affecting the conformation of the hypervariable loops, J. Mol. Biol. 224:487. No murine FR residues were included in the KOL V_{H}. Oligodeoxyribonucleotides were 5'- phosphorylated and with the M 13 universal forward primer annealed to the human V region genes cloned in M13 in reactions containing M13 ssDNA template. The DNA was extended and ligated with 2.5 U T7 DNA polymerase (United States Biochemicals, Cleveland, OH) and 0.5 U T4 DNA ligase (Gibco BRL, Grand Island, NY). The mutated strand was preferentially amplified from the extension/ligation mixture using M13 reverse sequencing primer with 1 U Vent DNA polymerase (New England Biolabs, Beverly, MA) and was then amplified by PCR using both M13 forward and reverse primers. Product DNA was cut with *Bam*H1 and *Hind*III, cloned into M13 and triple CDR-grafted mutants identified by DNA sequencing.

M13 clones containing the humanized V regions were sequenced in their entirety to ensure the absence of spurious mutations. RF DNA from the confirmed clones was digested with *Hind*III and *Bam*HI, cloned into pSVgpt or pSVhyg and human IgG1 or human kappa constant regions added exactly as described for the construction of the chimeric antibody genes.

### Expression and Purification of Recombinant mAbs

Heavy (5 µg) and light (10 µg) chain expression vectors were digested with *Pvu*I*,* ethanol precipitated and dissolved in 50 µl water. NSO cells (1-2 x 10⁷) were harvested by centrifugation, resuspended in 0.5 ml DMEM and mixed with the DNA in a 0.4 cm electroporation cuvette. After min. on ice the cells were given a single pulse of 170 V, 960 µF (GenePulser, Bio-Rad, Melville, NY) and incubated further for 15 min. on ice. The cells were allowed to recover in DMEM for 24-48 hours. The medium was then made selective by the addition of mycophenolic acid (0.8 ug/ml) and xanthine (250 µg/ml). Aliquots of 200 µl were distributed into 96-well plates. After a further 10-12 days, cells from the wells containing the highest levels of antibody measured by ELISA were selected and cloned by limiting dilution.

Antibodies were purified from overgrown cultures by protein A affinity chromatography (Boehringer Mannheim, Lewes, U.K.) Concentrations were determined by measuring A₂₈₀ₙₘ and confirmed by ELISA and SDS-PAGE.

### ELISA for Measurement of Antibody Binding

The wells of a microtiter plate were coated with goat anti-human IgM antibodies (Sera-Lab, Crawley Down, U.K.) in 50 mM bicarbonate buffer, pH 9.6. The plate was blocked with 1% BSA and followed by the addition of a soluble fusion protein consisting of the extracellular domain of human FcγRI and human IgM heavy chain (sFc γRI-µ) obtained from transiently transfected COS cells (the expression vector was kindly provided by Dr. Brian Seed, Massachusetts General Hospital, Boston, MA). Recombinant 22 or control mAbs were then added in the presence of excess (2.2 µ g/well) human IgG1 antibodies (Sigma, St. Louis, MO) that contained λ light chains to block the non-specific binding of the test mAbs via their Fc portion. Bound 22 mAbs were detected with peroxidase-labeled goat anti-human kappa chain antibodies (Sera-Lab, Crawley Down, U.K.) and o-phenylenediamine.

### Fluoresceination of Antibodies

The pH of mAb solution was adjusted to 9.3 by the addition of 0.1 M Na₂CO₃ Fluorescein iso-thiocyanate (FITC) (Sigma, St. Louis, MO) was dissolved in DMSO at a concentration of 2mg/ml. Forty µg of FITC was added for each milligram of mAb and incubated for two hours at room temperature. The fluoresceinated mAb was separated from the free FITC by G-25 chromatography.

### Preparation of Blood Cells

Buffy coats were prepared from heparinized whole venous blood. Whole blood was diluted with RPMI containing 5% dextran at a ratio of 2.5:1 (v/v). The erythrocytes were allowed to sediment for 45 minutes on ice, then the cells in the supernatant were transferred to a new tube and pelleted by centrifugation. The residual erythrocytes were removed by hypotonic lysis. The remaining lymphocytes, monocytes and neutrophils were kept on ice until use in binding assays. For some experiments, neutrophils were separated from mononuclear cells by ficoll hypaque (Pharmacia, Piscataway, NJ) gradient separation. To up-regulate FcγRI, neutrophils and mononuclear cells were treated with cytokines. Cultures of mononuclear cells were incubated at 37°C, 5% CO₂ for 48 hours in teflon dishes at 4 x 10⁶ Cells/ml of RPMI containing 2.5% normal human serum type AB (Sigma, St. Louis, MO) and 500 IRU/ml IFN-γ (R&D Systems, Minneapolis, MN). Neutrophils were cultured for 48 hours (37°C, 5% CO₂) in AIM V media (Gibco, Grand Island, NY) with 50 ng/ml G-CSF (Kindly provided by R. Repp, U. of Erlanger, Germany) and 500 IRU/ml IFN-γ.

### Flow Cytometry

Cell binding assays were performed using 96-well microtiter plates as previously described, (Guyre, P.M. et al., Monoclonal antibodies that bind to distinct epitopes on Fc γR are able to trigger receptor function. J. lmmunol., 143:1650). Briefly, cells were washed in PBS, pH 7.4 containing 2mg/ml BSA and 0.05% NaN₃ (PBA), and adjusted to 2.0 x 10⁷ cells/ml with PBA. FITC-labeled and unconjugated antibodies were prepared in PBA. Cells (25 µl), antibody (25 µl) and human serum (25 µl), or human IgG (10 mg/ml, Sigma, St. Louis, MO) (25 µl), or PBA (25 µl) were added to the microtiter plate, and left on ice for 45-60 minutes. Unbound antibody was removed from the wells by washing the cells 3 times with PBA. The cells were fixed with 1% paraformaldehyde. Cell associated fluorescence was analyzed on a Becton Dickinson FACScan.

### BsAb Coupling Procedure

BsAb were constructed using the method of Glennie et al, (Glennie, M.J. et al., (1987), Preparation and performance of bispecific F(ab' gamma)2, antibody containing thioether-linked Fab' gamma fragments, J. Immunol., 139:2367). mAbs 22 (both murine and humanized) and 520C9 (anti-HER2/neu) antibodies were produced by *in vitro* cultivation of the respective hybridoma cells. The antibodies were separately digested with pepsin to F(ab')₂, and subsequently reduced to Fab' by addition of 10 mM mercaptoethanolamine (MEA) for 30 minutes at 30°C. The Fab' fragments were applied to a Sephadex G-25 column equilibrated in 50mM Na Acetate, 0.5mM EDTA, pH 5.3 (4°C). Ortho-phenylenedimaleimide (o-PDM, 12mM) dissolved in dimethyl formamide and chilled in a methanol/ice bath was added (one half volume) to the murine 22 Fab' in the case of M 22 x 520C9, and to 520C9 Fab' in the case of H 22 x 520C9 and incubated for 30 minutes on ice. The Fab'-maleimide was then separated from free o-PDM on Sephadex G-25 equilibrated in 50mM Na Acetate, 0.5mM EDTA, pH 5.3 (4°C). For preparation of the BsAbs, the M22 Fab'-maleimide was added to the 520C9 Fab' or the 520C9 Fab'-maleimide was added to H22 Fab' at a 1:1 molar ratio. The reactants were concentrated under nitrogen to the starting volume using a Diaflo membrane in an Amicon chamber (all at 4°C). After 18 hours the pH was adjusted to 8.0 with 1M Tris-HCI, pH 8.0. The mixture was then reduced with 10mM MEA (30 minutes, 30°C) and alkylated with 25 mM iodoacetamide. The bispecific F(ab')₂ was separated from unreacted Fab's and other products by a Superdex 200 (Pharmacia, Piscataway, NJ) column equilibrated in PBS.

### Antibody Dependent Cellular Cytotoxicity (ADCC)

The HER2/neu over-expressing human breast carcinoma cells, SKBR-3, were used as targets for lysis by cytokine activated neutrophils (see preparation of blood cells). Targets were labeled with 100 µCi of ⁵¹Cr for I hour prior to combining with neutrophils and antibodies in a U-bottom microtiter plate. After incubation for 5 hours at 37°C supernatants were collected and analyzed for radioactivity. Cytotoxicity was calculated by the formula: % lysis = (experimental CPM - target leak CPM/detergent lysis CPM - target leak CPM) x 100%. Specific lysis = % lysis with antibody - % lysis without antibody. Assays were performed in triplicate.

### Superoxide Induction

U937 cells were used for measuring the ability of H22 to trigger a superoxide burst via FcγRI, (Pfefferkorn, L.C. and G.R. Yeaman (1994), Association of IgA-Fc receptors (Fc x R) with Fcε RIγ 2 subunits in U937 cells, J. Immunol. 153:3228; Hallet, H.B. and A.K. Campbell (1983). Two distinct mechanisms for stimulating of oxygen - radical production in polymorphonuclear leucocytes, Biochem J. 216:459). U937 cells were cultured for five days in RPMI-1640 (Gibco, Grand Island, NY) with 10% FBS (Hyclone, Logan, UT) in the presence of 100 U/ml IFN-γ (Genentech, S. San Francisco, CA) to induce differentiation and increased expression of FcγRI. On the day of the experiment, these differentiated cells were incubated for 20 minutes in fresh RPMI-1640 with 10% FBS at 37°C. The cells were then pelleted and resuspended at a concentration of 3 x 10⁶ cells/ml in PBS supplemented with 1mM CaCl₂, 1mM MgCl₂, 11mM glucose, and 100µg/ml BSA (Sigma, St. Louis, MO). To trigger the release of superoxide, 100µl of cells were added to 100µl of a reaction solution containing 0.1mM luminol (Sigma, St. Louis, MO), 0.5mM sodium vanadate (Sigma, St. Louis, MO), and either mAb M22, H22, or 197 and placed in the luminometer at 22° C. Measurements of the spontaneous production of superoxide were made every 30 to 40 seconds starting immediately following the addition of the cells to the reaction solution in the luminometer. To compare the superoxide triggered by crosslinking FcγRI with M22, H22 or 197, each mAb was used at a concentration of 10µg/ml. The production of superoxide in mV/sec was monitored for 20 minutes. MAb M22, M32.2 and 197 were added at various concentrations to establish the dose-responsiveness of superoxide production.

### Results

### Murine Ig V Region Genes

Ig V region cDNAs were prepared from M22 hybridoma RNA using primers specific for murine heavy and kappa constant regions and were amplified by PCR with the additional use of a series of primers based on sequences of known signal and/or 5' sequences of mature V regions. PCR products of the expected sizes for V_{H} and V_{κ} were obtained using the SH2BACK/CG1FOR and VK7BACK/CK2FOR primer combinations. Amplified DNA was digested with appropriate restriction enzymes, cloned into M13 and the sequence in both directions determined from at least 24 independent clones. The deduced amino acid sequences are shown in SEQ. ID Nos. 29 and 30. The 4 N-terminal residues of V_{K} are encoded by the VKBACK primer.

The M22 V_{H} and V_{K} are members of murine heavy chain subgroup IIID and kappa subgroup I, (Kabat, E.A. et al., (1991), Sequences of Proteins of Immunological Interest, 5th Ed., U.S. Department of Health and Human Services), respectively. Apart from the residue at L97, the amino acid sequence of the M22 Vκ is identical to that from the murine anti-IgG mAb A17 (Shlomchik, M. et al., Variable region sequences of murine IgM anti-IgG monoclonal autoantibodies (rheumatoid factors). II Comparison of hybridonias derived bylipopolysaccharide stimulation and secondary protein immunization, J. Exp. Med. 165:970).

### Humanized mAbs and Initial Characterization of their Binding

M22 V_{H} FR showed greater homology (79%) to KOL (human subgroup III) than to NEWM (57%) (human subgroup II). To see how this difference might affect binding, heavy chains were constructed based either on NEWM V_{H} including the murine residues Phe27, Ile28 and Arg71, or on KOL V_{H} with no murine FR amino acids. Both humanized V_{H} were partnered with the same REI-derived humanized light chain.

The affinity of the humanized mAbs was initially assessed by ELISA measuring the binding to FcγRI/IgM heavy chain fusion protein. The data showed that the KOL V_{H}/REI V_{κ} mAb had the same binding as the chimeric mAb whereas the NEWM V_{H}/REI V_{κ} mAb exhibited an approximate 5- fold lower affinity. The low binding of a nonspecific human IgG1 mAb showed that >95% of binding of the humanized mAbs was via the Fv portion rather than through the Fc domain.

While additional changes to the NEWM FR would be expected to restore binding affinity these could create novel epitopes which might provoke an unwanted immunological response. The KOL V_{H}/REI V_{κ} mAb, designated H22, was therefore chosen for further examination of its binding characteristics.

### Functional Characterization of mAbH22

A series of binding experiments were performed to establish the specificity and isotype of the H22 antibody. Peripheral blood leukocytes stained with fluorescein-conjugated M22 or H22 demonstrated specific binding to monocytes with approximately 10⁴ binding sites per cell. In contrast, lymphocytes or unstimulated neutrophils had little or no specific binding (Table 1):

**Table 1: Specific Binding of H22 to Monocytes**

| Antibody | Monocytes | Lymphocytes | PMNs |
|---|---|---|---|
| M22 | 10,000^{a} | <1000 | <1000 |
| H22 | 10,500 | <1000 | <1000 |

| | | | |
|---|---|---|---|
| ^{a}Antibody sites per cell, average of duplicates | | | |

To demonstrate that the H22 binds to FcγRI at the same site as M22 and that it also binds as a ligand at the Fc binding domain, competition experiments with two anti-Fcγ RI murine mAb (M22 and M32.2) and a human IgG1 mAb were performed. Unconjugated H22 and M22 competed equivalently for either the binding of fluoresceinated M22 or fluoresceinated H22 in the presence of excess human IgG which saturated the Fc binding sites on FcγRI. As expected, the anti-FcγRI antibody M32.2 which binds to a different site on FcγRI than M22 (Guyre, P.M. et al., J. Immunol. 143:1650) was also unable to compete with the M22-FITC. In addition, the inhibition of H22-FITC by H22 and not by an irrelevant human IgG 1 mAb confirmed the specificity of FcγRI binding via the V regions of H22.

H22, but not M22, was able to compete for Fc mediated binding to FcγRI by a fluorosceinated human IgG1. This experiment demonstrated that the Fc portion of H22 but not M22 bound to the Fc binding domain of FcγRI. This is consistent with the ability of the Fc portion of human IgG1 antibodies, but not murine IgG1, to bind FcγRI with high affinity.

Since the humanization of M22 was primarily to increase its immunotherapeutic potential, the binding activity of H22 to monocytes and cytokine-activated neutrophlils was determined in the presence of human serum. H22-FITC bound with similar affinity to FcγRI on monocytes in the presence or absence of human serum. In contrast, the Fc-mediated binding of an irrelevant human IgG-FITC was completely inhibited by human serum. Likewise, H22-FITC bound with similar affinity to IFN-γ-treated neutrophils in the absence and in the presence of human serum. Collectively, the data demonstrated that H22 binds both via its V regions to a site distinct from the Fc binding domain and via its Fc region to the ligand binding domain of FcγRI. The former binding activity effectively overcomes antibody blockade of human IgG1.

### Functional Activity of H22 BsAb

The foremost application of anti-FcγRI antibodies for immunotherapy is the development of BsAbs which link FcγRI-bearing effector cells to a tumor cell, a virus, or a virally-infected cell. Such BsAb have been developed with M22; therefore, a comparison was made of the ability of the M22 anti-tumor BsAb (520C9xM22) and a corresponding H22 BsAb (520C9xH22) to mediate cytotoxicity. These BsAbs consisted of H22 or M22 Fab' chemically conjugated to the Fab' of an anti-HER2/neu antibody (520C9), and thus were specific for the effector cell trigger molecule FcγRI and the tumor antigen.

Comparison of M22-derived and H22-derived BsAbs was done by ADCC assays. M22- and H22-derived BsAbs mediated the killing of HER2/*neu* overexpressing SKBR-3 cells. Both the murine and humanized BsAbs exhibited similar levels of lysis of antigen bearing target cells. In addition, both BsAb retained ADCC activity in the presence of human serum, while excess M22 F(ab')₂ resulted in complete inhibition of killing. Taken together these results show that the H22 BsAb-induced lysis is mediated through the M22 epitope and that the ADCC is FcγRI specific.

Finally, the ability of H22 and M22 to stimulate superoxide production by the monocyte-like cell line U937 was evaluated. M22, which binds to the FcγRI only by its V regions, induced a very low level oxygen burst, presumably because it is unable to cross-link the receptor efficiently. However, H22, which can cross-link FcγRI by binding as a ligand via its Fc domain and, additionally, as an antibody via its Fv, induced a more substantial release of superoxide.

### Example 2: Generation of a Functional H22-Epidermal Growth Factor Fusion Protein

### Materials and Methods

### Expression vectors and cloning

Expression vectors for the genomic clones of the heavy (pSVgpt) and light (pSVhyg) chains of H22 are as described in International Patent Application Publication Number: WO 94/10332 entitled, *Humanized Antibodies to Fc Receptors for Immunoglobulin G on Human Mononuclear Phagocytes.* For the Fab-ligand fusion construct, it was unnecessary to alter the light chain. For the heavy chain, however, the CH2 and CH3 domains had to be removed and replaced with the coding sequences of the ligands. The heavy chain vector contains two *Bam*HI sites, one in the intron between VH and CH1, and the other just downstream of CH3. Using the BamHI restriction sites, DNA encoding the constant domains were replaced by a truncated version encoding only CH1 and most of the hinge. To do this, the polymerase chain reaction (PCR) was utilized to engineer the new C-terminus of the heavy chain fragment with the alterations shown in Figure 1.

The construct shown in Figure 1 [C], consisting of a translation termination codon downstream of the cloning restriction sites, *Xho*I and *Not*I*,* and upstream of a *Bam*HI site which was used to clone the new PCR generated CHI fragment downstream of VH, was used to generate the fusion protein constructs. The cloning sites, which are located downstream of most of the hinge in order to retain flexibility between the Fd and ligand domains, was used to insert DNA encoding EGF or other ligands. Also, the single Cys residue has been retained from the previous construct to allow conjugation for the formation of dimeric molecules.

DNA encoding the ligands were amplified by PCR to have a *Xho*I site on the N-terminus and a *Not*I site on the C-terminus of the coding region, and then inserted in the proper reading frame into the same sites of the newly engineered H22 heavy chain truncated fragment described above. cDNA encoding epidermal growth factor (EGF) was obtained from the ATCC (#59957). Only DNA encoding the 53 amino acid residues of mature EGF out of the approximately 1200 residue precursor was cloned beginning with Asn 971 and ending with Arg 1023 (Bell, G.I., Fong, N.M., Stempien, M.M., Wormsted, MA., Caput, D., Ku. L., Urdea, M.S., Rall, L.B. & Sanchez-Pescador, R. Human Epidermal Growth Factor Precurser: cDNA Sequence, Expression In Vitro and Gene Organization. Nucl. Acids Res. 14: 8427-8446,1986.).

### Expression

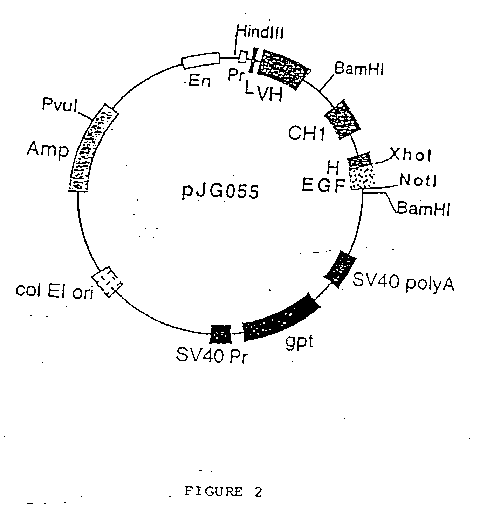

The murine myeloma NSO (ECACC 85110503) is a non-Ig synthesizing line and was used for expression of the fusion proteins. The final expression vector, a pSVgpt construct with DNA encoding H22 Fd fused in frame to EGF (shown in Figure 2) was transfected by electroporation using a BioRad Gene Pulser to NS0 which had been previously transfected with the pSVhyg construct containing DNA encoding H22 light chain. These polypeptides were expressed by an Ig promoter and Ig enhancer present in the vectors, and secreted by the mAb 22 heavy chain signal peptide located on the N-terminus of the constructs. One or two days after transfection, mycophenolic acid and xanthine were added to the media to select for cells that took up the DNA. Individual growing colonies were isolated and subcloned after binding activity was demonstrated by ELISA.

### Purification

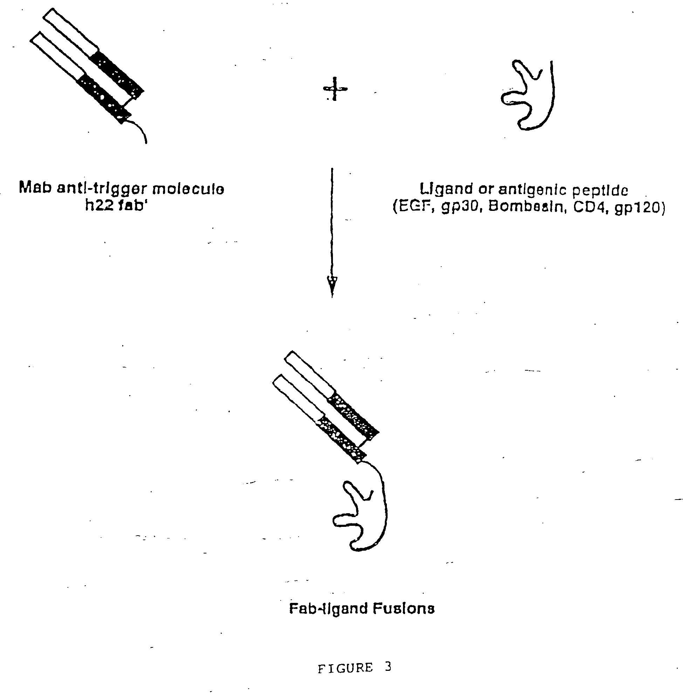

Cells expressing the H22-EGF fusion protein were subcloned and expanded. The fusion protein-expressing clone was expanded and grown in spinner cultures and the supernatant was clarified and concentrated. Small scale purification was performed by affinity chromatography on an anti-human kappa chain affinity column (Sterogene. Carlsbad, CA). The purified protein was analyzed by SDS-PAGE on a 5-15% acrylamide gradient gel under nonreducing conditions. Figure 3 is a schematic representation of the generation of anti-Fc receptor-ligand fusion proteins.

### Bispecific flow cytometry

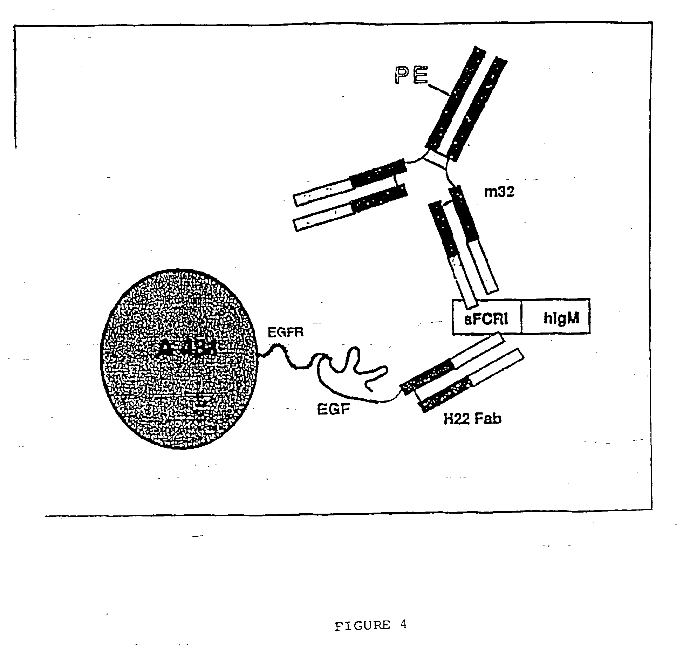

To show that the fusion protein is capable of binding both FcγRI and EGFR simultaneously, a flow cytometric assay has been developed (Figure 4). In this assay different concentrations of H22-EGF fusion protein or the bispecific antibody, BsAb H447 (H22 X H425, a humanized version of the murine monoclonal antibody M425, which binds EGFR at the ligand binding site (E. Merck) was incubated with A431 cells, a cell line which expresses the EGF receptor (EGFR) (ATCC, Rockville, MD). After washing, a supernatant containing a fusion protein consisting of the extracellular domain of FcγRI and the Fc portion of human IgM was added. Finally, a Phycoerythrin (PE)-labeled mAb (32.2), that binds FcγRI at a site that is distinct from that bound by mAb 22, was added. The cells were then analyzed by FACSCAN. Alternatively, binding to EGFR was blocked by excess (100 µg/ml) whole murine mAb 425 (E. Merck), and binding of bsAb or fusion protein was detected by PE-labeled anti-human IgG.

### ADCC

ADCC mediated by the fusion protein was determined using a ⁵¹ Cr killing assay. The EGFR overexpressing cell line, A431, was used as targets for lysis by human monocytes cultured in γ-interferon (IFN-γ) for 24 hours. Targets were labeled with 100 µCi of ⁵¹ Cr for 1 hour prior to combining with effector cells and antibodies in a U-bottom microtiter plate. After incubation for 5 hours at 37°C supernatants were collected and analyzed for radioactivity. Cytotoxicity was calculated by the formula: % lysis = (experimental CPM - target leak CPM/detergent lysis CPM - target leak CPM) X 100%. Specific lysis = % lysis with antibody - % lysis without antibody. The ability of the fusion protein to mediate ADCC was compared with that of the respective BsAb. The assay was also performed in the presence of 25% human serum to demonstrate that IgG or other factors found in human serum will not inhibit fusion protein-mediated ADCC.

### Results

### Purification

NS0 cells expressing the H22 kappa chain were transfected with the H22-EGF heavy chain construct and clones selected for resistance to mycophenolic acid and xanthine were expanded and the fusion protein was affinity-purified from the supernatant on an anti-human kappa column (Sterogene, Carlsbad, CA). The purified protein was analysed by SDS-PAGE. The purified protein migrated at an apparent molecular weight of 50-55 kDa, indicating that the fusion protein is expressed as a monomer, not a disulfide-linked dimer. In addition, a band was seen at an apparent molecular weight of 25kDa and is probably free light chain.

### Binding specificity

To demonstrate that the fusion protein could bind FcγRI and EGFR simultaneously a bispecific FACS assay was devised. Figure 5 shows that both the chemically-linked, fully-humanized BsAb H447 (H22 (anti-FcγRI) x H425), which was made as described in the following Example 3, and the H22-EGF fusion protein bound EGFR on A431 cells and soluble FcγRI simultaneously in a dose-dependent fashion.

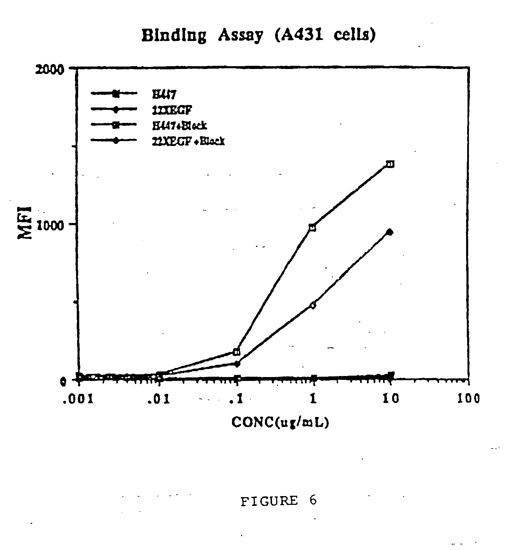

The EGFR-specificity of the fusion protein was demonstrated by the ability of the murine mAb, M425, which binds EGFR at the ligand binding site, to inhibit fusion protein or H22 x H425 binding. Various concentrations of either the BsAb H447, or of the H22-EGF fusion protein were incubated with A431 cells in either the presence or absence of an excess of M425. Figure 6 shows that binding of both the BsAb and the fusion protein were inhibited by M425, demonstrating the specificity of the fusion protein for EGFR.

### ADCC

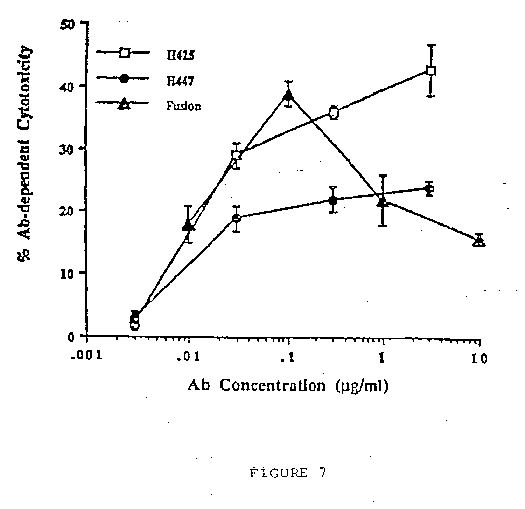

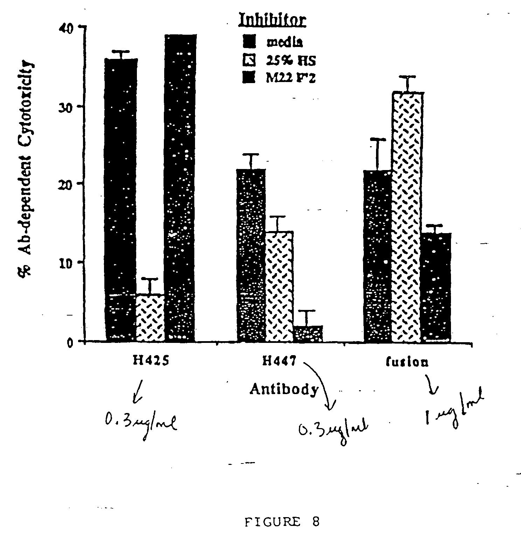

The ability of the fusion protein to mediate ADCC was analyzed using A431 cells as targets. Human monocytes cultured for 24 hours in the presence of IFN-γ were used as effector cells. Figure 7 demonstrates the whole antibody, H425, the BsAb H447 (H22 x H425) and the fusion protein mediated dose-dependent lysis of A431 cells. Figure 8 demonstrates that while ADCC mediated by the whole antibody is inhibited by 25% human serum (25%HS), ADCC mediated by the fusion protein was not inhibited by human serum and, in this particular experiment, fusion protein-mediated ADCC was enhanced by human serum. These results support the clinical utility of these molecules by demonstrating that the fusion protein was capable of killing EGFR-overexpressing cells, even in the presence of FcγRI-expressing effector cells as would be present *in vivo.*

### Growth inhibitory properties of H22-EGF fusion proteins

Although EGF acts to stimulate growth of normal cells that express receptors for it, EGF also can act to inhibit growth of tumor cells that over-express EGF-R (Barnes, D.W. (1982) J. Cell Biol. 93:1, MacLeod, C.L. et al. (1986) J. Cell. Physiol. 127:175). The ability of EGF and the H22-EGF fusion protein to inhibit the growth of A431 cells was examined as follows.

2 x 10⁴ A431 cells were added to six well plates in complete media alone or in media containing various concentration of either EGF, H22-EGF, the Fab fragment of H22, or the F(ab')₂ fragment of H425. Viable cells were counted after seven days using a hemocytometer. The analyses were performed in duplicate and reported as means +/standard deviations.

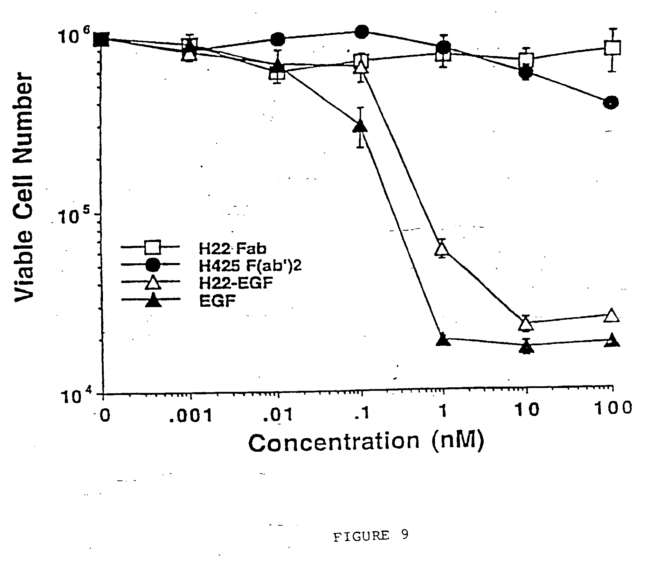

The results are presented in Figure 9. These results indicate that EGF and H22-EGF significantly inhibited cell growth in a dose dependent fashion. On the contrary, the F(ab')2 fragment of H425 which had some inhibitory activity only at high concentrations and the Fab fragment of H22 had no growth inhibiting activity.

Thus, H22-EGF is able to bind to both FcγRI and EGF simultaneously, indicating that the molecule had folded properly and had maintained the flexibility required to bind both receptors at the same time. Furthermore, H22-EGF inhibited proliferation of the EGF-R expressing tumor cell line, A431, indicating that, similar to EGF, the H22-EGF fusion protein is capable of signaling through the EGF-R. H22-EGF also mediates potent killing of A431 cells in the presence of FcγRI expressing effector cells. Thus, H22-EGF mediates both cytotoxic and cytostatic effects on EGF-R expressing cells. Administration of H22-EGF to a subject having a tumor will result in recruitment of the body's natural cytotoxic effector cells to mediate killing of the tumor cells by potentially three different modes - cytotoxicity, growth inhibition, and phagocytosis. Furthermore, in addition to cell mediated cytotoxicity of the tumor cells, the effector cells recruited by H22-EGF may also further augment anti-tumor immunity by secreting inflammatory cytokines and/or by processing and presenting tumor antigens to tumor specific T cells.

### Example 3: H22-Heregulin (H22-gp30) Fusion Protein Mediates Tumor Cell Killing

Heregulin (HRG) is a ligand for the HER3 and HER4 molecules. Both of these receptors may form heterodimers with HER2, a molecule which is overexpressed in some breast cancer cells. The affinity of HRG for HER3 and HER4 increases significantly when these molecules from heterodimers with HER2. This example demonstrates that a bispecific molecule, comprising heregulin and a binding specificity for the FcγRI inhibits growth of a tumor cell line and mediates fusion protein dependent cytotoxicity of these cells in the presence of FcγRI-bearing cytotoxic effector cells.

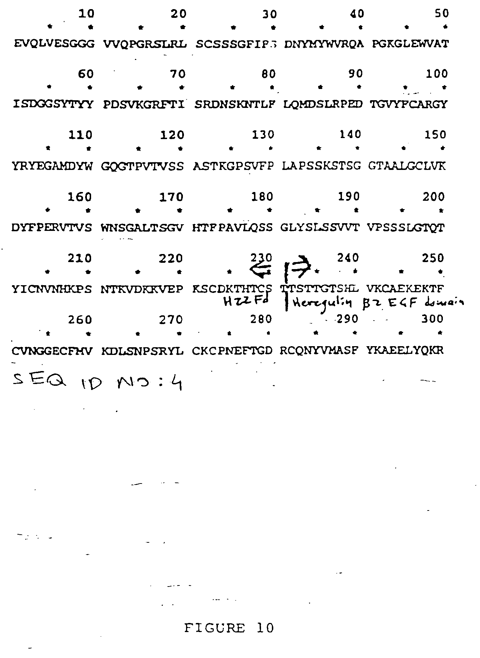

The H22-heregulin fusion protein was constructed in the same manner as the H22-EGF fusion protein described in Example 2. Briefly, genomic DNA encoding the Fd fragment of humanized anti-FcγRI mAb, H22, was fused to cDNA encoding the EGF domain of the ß2 form of HRG. The amino acid sequence of the H22-HRG fusion protein (SEQ_ID NO: 4) is shown in Figure 10. This fusion protein comprises amino acids 171-239 of the heregulin β2 shown in U.S. Patent Number 5,367,060. Other portions of heregulin β2, as well as portions of other heregulin molecules, such as those disclosed in U.S. Patent Number 5,367,060 can also be used. The resulting H22Fd-HRG expressing vector was transfected into a myeloma cell line previously transfected with a vector containing DNA encoding the H22 kappa light chain. The resultant fusion protein was expressed predominantly as a monomer, even though the protein contains a free Cys residue in the hinge region of the H22 Fab component. Flow cytometry showed that this fusion protein was able to bind to the HER2 overexpressing tumor cell line, SKBR-3, as well as to FcγR-expressing cells.

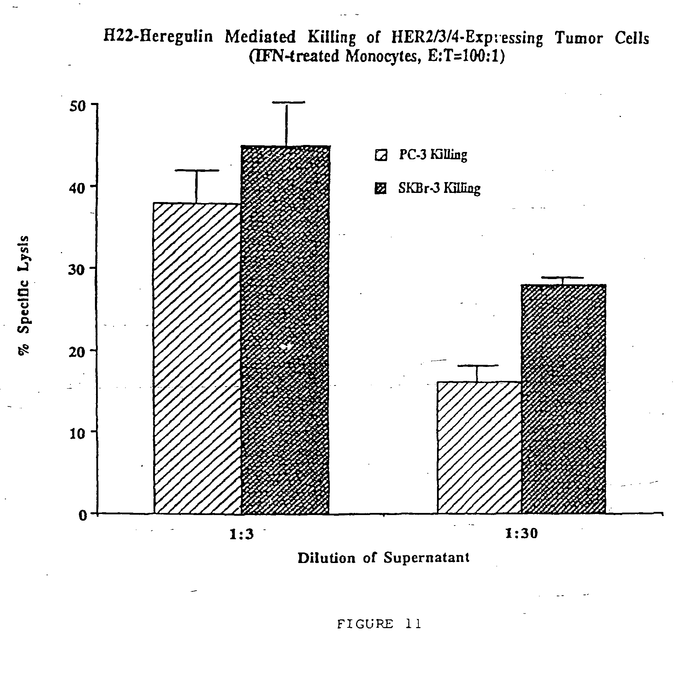

To test the biological activity of the H22Fd-HRG fusion protein, supernatant from the myeloma cells expressing this fusion protein was diluted three fold or thirty fold and added to PC-3 cells or SKBR-3 tumor cells expressing HER2, HER3, and HER4 in the presence of IFN-treated monocytes at a ratio of 100: 1 monocytes to target tumor cells. The monocytes were treated with IFN-γ and the target cells were labeled with ⁵¹Cr as described in Example 2. The % of specicific lysis was calculated as indicated in Example 2. The results are presented in Figure 11. The results indicate that about 45% of SKBR3 cells and up to about 49% of PC-3 cells are lysed upon incubation of the cells with the supernatant diluted 3 fold.

This fusion protein inhibits growth of SKBR-3 tumor cells and mediates fusion protein dependent cytotoxicity of these cells in the presence of FcγRI-bearing cytotoxic effector cells. Thus, the results of this example show that an anti-FcγRI-heregulin fusion protein can mediate anti-tumor cytotoxic activities under physiologic conditions and indicate that such a fusion protein will have therapeutic utility in the treatment of various cancers.

### Example 4: H22-Bombesin Fusion Protein Mediates Tumor Cell Killing

The H22-bombesin fusion protein was constructed similarly to the H22-EGF fusion protein described above. However, since bombesin is a short peptide (14 amino acid residues), instead of amplifying cDNA encoding bombesin using PCR technology, DNA oligomers encoding the sense and anti-sense strands of bombesin were hybridized to create the coding region. The amino acid sequence of the bombesin peptide fused to the carboxyl end of the heavy chain of the H22 antibody is the following:
-Gln-Arg-Leu-Gly-Asn-Gln-Trp-Ala-Val-Gly-His-Leu-Met-Gly (SEQ ID NO: 5)
which corresponds to amino acids 2-14 of bombesin (Anastasi et al. (1971) Experientia 27:166) and contains an additional glycine residue at the carboxyl end of the peptide. The oligomers had overlapping ends that did not hybridize but instead created sticky ends for a *XhoI* site on the N-terminus and a *Not*I site on the C-terminus such that it could be cloned into the H22 heavy chain expression vector described above.

The biological activity of the H22-bombesin fusion protein on tumor cell killing was investigated as described above for the H22-EGF and H22-heregulin fusion proteins. Briefly, PC-3 tumor cells bearing bombesin receptors were labeled with ⁵¹Cr and incubated with monocytes and various concentrations of H22-fusion protein, and fusion protein dependent lysis was determined as described above. The results, shown in Figure 12. indicate that the target cells are lysed and that the level of target cell lysis increases proportionally with the amount of fusion protein added to the assay.

Fusion proteins having H22 as one binding entity and CD4 (AIDS Repository) or gp120 (AIDS Repository) as a second binding entity were also produced.

### Example 5: Production of Bispecific Antibodies From Modified Humanized Antibody Fragments

### Materials and Methods

### Expression vectors and cloning

Expression vectors for the genomic clones of the heavy (pSVgpt) and light (pSVhyg) chains of H22 were as described in International Patent Application Publication Number: WO 94/10332 entitled, *Humanized Antibodies to Fc Receptors for Immunoglobulin Gon Human Mononuclear Phagocytes.* For the Fab' construct, it was unnecessary to alter the light chain. For the heavy chain, however, the CH2 and CH3 domains had to be removed and replaced with a termination codon. The heavy chain vector contains two BamHI sites, one in the intron between VH and CH1, and the other just downstream of CH3. Using the BamHI restriction sites, DNA encoding the constant domains were replaced by a truncated version encoding only CH 1 and most of the hinge. To do this, The polymerase chain reaction (PCR) was utilized to engineer the new C-terminus of the heavy chain fragment with the alterations shown in Figure 1. Figure 1 [B] shows the alterations for generation of a truncated single-sulfhydryl version.

### Expression

The murine myeloma NSO (ECACC 85110503) is a non-Ig synthesizing line and was used for expression of the modified H22 antibody. The final expression vector, a pSVgpt construct with DNA encoding H22 Fd was cotransfected with the pSVhyg construct containing DNA encoding H22 light chain by electroporation using a BioRad Gene Pulser. These polypeptides were expressed by an Ig promoter and Ig enhancer present in the vectors, and secreted by the mAb 22 heavy chain signal peptide located on the N-terminus of the constructs. One or two days after transfection, mycophenolic acid and xanthine were added to the media to select for cells that took up the DNA. Individual growing colonies were isolated and subcloned after FcγRI binding activity was demonstrated.

### Purification

The single sulfhydryl form of the H22 antibody and the whole H425 (anti-EGFR) antibody were produced by *in vitro* cultivation of the respective transfected NSO cells. The H425 was purified by protein A affinity chromatography. The single sulfydryl form of the antibody H22 was purified by ion exchange chromatography using Q-Sepharose followed by SP-Sepharose (Pharmacia, Piscataway, NJ). The purity of the single sulfhydryl form of the H22 antibody was assessed by SDS-PAGE.

### Generation of bispecific antibody (BsAb)

BsAb was constructed using the method of Glennie et al. (Glennie, M.J. et al., (1987), Preparation and performance of bispecific F(ab' gamma)2, antibody containing thioether-linked Fab' gamma fragments, J. Immunol., 139:2367). The F(ab')₂ of H425 was generated by limited pepsin proteolysis in 0.1M citrate buffer, pH 3.5 and the F(ab')₂ purified by ion exchange chromatography. The mAbs were reduced by addition of 20 mM mercaptoethanolamine (MEA) for 30 minutes at 30°C. The Fab' fragments were applied to a Sephadex G-25 column equilibrated in 50mM sodium acetate, 0.5mM EDTA, pH 5.3 (4°C). Ortho-phenylenedimaleimide (o-PDM, 12mM) dissolved in dimethyl formamide and chilled in a methanol/ice bath was added (one half volume) to the H22 Fab' and incubated for 30 minutes on ice. The Fab'-maleimide was then separated from free o-PDM on Sephadex G-25 equilibrated in 50mM Na Acetate, 0.5mM EDTA, pH 5.3 (4°C). For preparation of the BsAbs, the H22 Fab'-maleimide was added to the H425 Fab' at a 1.2:1 molar ratio. The reactants were concentrated under nitrogen to the starting volume using a Diaflo membrane in an Amicon chamber (all at 4°C). After 18 hours the pH was adjusted to 8.0 with 1M Tris-HCI, pH 8.0. The mixture was then reduced with 10mM MEA (30 minutes, 30°C) and alkylated with 25 mM iodoacetamide. The bispecific F(ab')₂ was separated from unreacted Fab's and other products by a Superdex 200 (Pharmacia, Piscataway, NJ) column equilibrated in PBS.

### Bispecific flow cytometry

To show that BsAb generated by the o-PDM method as well as that generated by the DTNB method are capable of binding both FcγRI and EGFR simultaneously, a flow cytometric assay has been developed (Figure 13). In this assay different concentrations of the two BsAbs were incubated with A431 cells, a cell line which expresses the EGF receptor (EGFR). After washing, a supernatant containing a fusion protein consisting of the extracellular domain of FcγRI and the Fc portion of human IgM was incubated with the cells. Finally, the cells were incubated with a FITC-labeled anti-human IgM-specific antibody. The cells were then analyzed by FACSCAN.

### ADCC

BsAb-mediated ADCC was determined using a ⁵¹Cr killing assay. The EGFR overexpressing cell line, A431, was used as targets for lysis by human monocytes cultured in γ-interferon for 24 hours. Targets were labeled with 100 µCi of ⁵¹ Cr for 1 hour prior to combining with effector cells and antibody in a flat-bottomed microtier-plate. After incubation for 16 hours at 37°C supernatants were collected and analyzed for radioactivity. Cytotoxicity was calculated by the formula: % lysis = (experimental CPM - target leak CPM/detergent lysis CPM - target leak CPM) X 100%. Ab-dependent lysis = % lysis with antibody - % lysis without antibody.

### Results

### Purification

NSO cells were cotransfected with the truncated H22 heavy chain construct and the intact kappa chain construct. Clones selected for resistance to mycophenolic acid and xanthine were expanded and the protein was purified from the supernatant by Q-Sepharose followed by SP-Sepharose ion exchange chromatography. The purified protein was analyzed by SDS-PAGE. The purified protein migrated at an apparent molecular weight of 50kDa, indicating that the protein is expressed as a monomer, not a disulfide-linked dimer.

### Construction and characterization of a BsAb composed of single sulfhydryl H22 linked to Fab' of H425 (anti-EGFR)

A BsAb was constructed where the single sulfhydryl form of H22 was linked to the Fab' fragment of H425, a humanized anti-EGFR mAb. The BsAb was generated using o-PDM as a linker by the method of Glennie et al. (Glennie, M.J. et al., (1987), Preparation and performance of bispecific F(ab' gamma)2, antibody containing thioether-linked Fab' gamma fragments, J. Immunol., 139:2367). The activity of this BsAb was compared to one generated by the DTNB method using Fab' fragments made from pepsin digestion and reduction of whole H22. To demonstrate that these BsAbs could bind FcγRI and EGFR simultaneously a bispecific FACS assay was devised. Figure 14 shows that both the o-PDM-linked BsAb and the BsAb made by the DTNB method bound EGFR on A431 cells and soluble FcγRI simultaneously in a dose-dependent fashion.

The ability of the two BsAbs to mediate ADCC was analyzed using A431 cells as targets. Human monocytes cultured for 24 hours in the presence of IFN-γ were used as effector cells. Figure 15 demonstrates the two BsAbs mediated dose-dependent lysis of A431 cells in a comparable fashion. These results demonstrated that BsAb generated from the truncated, single sulfhydryl form of H22 was capable of killing EGFR-overexpressing cells in the presence of FcγRI-expressing effector cells.

### Example 6 : Production of Trivalent Antibodies

### Materials and Methods

### Cell lines and antibodies. M22, 520C9, H425. SKBR3 and A431

M22 and 520C9 were purified from hybridoma supernatant by ion exchange chromatography (Pharmacia, Piscataway, NJ) and 520C9 was further purified by protein A affinity chromatography (Pharmacia, Piscataway, NJ). H425 was purified from hybridoma supernatant by protein A affinity chromatography (Pharmacia, Piscataway, NJ). The M22- and 520C9- producing murine hybridoma were described previously (Guyre et al., (1989) Monoclonal antibodies that bind to distinct epitopes on FcgRI are able to trigger receptor function, J. Immunol. 143:5, 1650-1655; Frankel et al., (1985) Tissue distribution of breast cancer-associated antigens defined by monoclonal antibodies, J. Biol. Response Modifiers, 4:273-286). The murine myeloma NSO (ECACC 85110503) is a non-Ig synthesizing line and was used for the expression of the humanized mAb, H425 (Kettleborough et al., (1991) Humanization of a mouse monoclonal antibody by CDR-grafting: the importante of framework residues on loop conformation, Protein Eng., 4:773). SKBR-3, (ATCC, Rockville, MD) a human breast carcinoma cell line that overexpresses the HER2/neu protooncogene, and A431 (ATCC, Rockville, MD), a human squamous carcinoma cell line that overexpresses EGFR (ATCC, Rockville, MD) were cultivated in Iscove's Modified Dulbecco's Medium (IMDM, Gibco, Grand Island, NY).

### Neutrophil preparation

Neutrophils are separated from mononuclear cells by ficoll hypaque (Pharmacia, Piscataway, NJ) gradient separation. To up-regulate F_{cγ}RI, neutrophils are treated with cytokines. Neutrophils are cultured for 24-48hrs (37°C, 5% C0₂) in AIM V media (Gibco, Grand Island, NY) containing 2.5% normal human serum type AB (Sigma, St. Louis, MO), 50 ng/ml G-CSF (Kindly provided br R. Repp, U. of Erlanger, Germany) and 100 IRU/ml IFN-γ.

### Conjugation method

BsAb were constructed using the method of Glennie et al (Glennie, M.J. et al., (1987), Preparation and performance of bispecific F(ab' gamma)2, antibody containing thioether-linked Fab' gamma fragments, J. Immunol., 139:2367). mAbs M22, 520C9 (anti-HER2/neu, 33), and H425 (anti-EGFR) antibodies were produced by *in vitro* cultivation of the respective hybridoma cells. The F(ab')₂ of each antibody were generated_by limited pepsin proteolysis in 0.1 M citrate buffer, pH 3.5 and the F(ab')₂ purified by ion exchange chromatography. mAbs M22 and H425 were reduced to Fab' by addition of 20 mM mercaptoethanolamine (MEA) for 30 minutes at 30°C. The Fab' fragments were applied to a Sephadex G-25 column equilibrated in 50mM Na Acetate, 0.5mM EDTA, pH 5.3 (4°C). Ortho-phenylenedimaleimide (o-PDM, 12mM) dissolved in dimethyl formamide and chilled in a methanol/ice bath was added (one half volume) to the murine 22 Fab' and incubated for 30 minutes on ice. The Fab'-maleimide was then separated from free o-PDM on Sephadex G-25 equilibrated in 50mM Na Acetate, 0.5mM EDTA, pH 5.3 (4°C). For preparation of the BsAbs, the M22 Fab'-maleimide was added to the H425 Fab' at a 1:1 molar ratio. The reactants were concentrated under nitrogen to the starting volume using a Diaflo membrane in an Amicon chamber (all at 4 °C). After 18 hours the pH was adjusted to 8.0 with 1M Tris-HCI, pH 8.0. The mixture was then reduced with 10mM MEA (30 minutes, 30°C) and alkylated with 25 mM iodoacetamide. The bispecific F(ab')₂ as separated from unreacted Fab's and other products by a Superdex 200 (Pharmacia, Piscataway, NJ) column equilibrated in phosphate buffered saline (PBS). The BsAb M22 x 520C9 was made in a similar fashion except that 520C9 was used instead of H425.

Trispecific antibody composed of M22 x H425 x 520C9 was made in two stages (Figure 16). In the first stage, M22 was linked to H425 as described above to create the M22 x H425 BsAb except that rather than a final reduction and alkylation, the reactants were treated with DTNB to block the remaining free sulfhydryl groups. The bivalent BsAb was purified by gel filtration on a Superdex 200 column, reduced to F(ab')₂(SH) and mixed in a 1: 1 molar ratio with o-PDM-treated 520C9. The resulting trispecific F(ab)3 was purified on a Superdex 200 column. The TsAb was analyzed by HPLC size exclusion chromatography using a TSK 3000 column (ToJo Haas, Japan). Using the same procedure as above another TsAb comprising m22 Fab' x 32.2 Fab' x m22 Fab' has been constructed.

### Bispecific flow cytometry

The TsAb can bind to EGFR and FcγRI simultaneously or to HER2/neu and F_{cγ} RI simultaneously. Either A431 cells (high EGFR-expressing cells) or SKBR-3 cells (high HER2/neu-expressing cells) were incubated with various concentrations of BsAbs (M22 x 520C9 or M22 x H425) or with the TsAb, M22 x H425 x 520C9. The cells were washed and then incubated with the soluble F_{cγ}RI. Soluble F_{cγ}RI binding was detected with mAb 32.2-FITC which binds F_{cγ}RI at a site that is distinct from the 22 binding site. The cells were then analyzed by FACSCAN.

### ADCC

Either SKBR-3 cells or A431 cells were used as targets for lysis by cytokine activated neutrophils. Targets were labeled with 100µCi of ⁵¹Cr for 1 hour prior to combining with neutrophils and antibodies in a U-bottom microtiter plate. After incubation for 16 hours at 37°C supernatants were collected and analyzed for radioactivity. Cytotoxicity was calculated by the formula: % lysis = (experimental CPM - target leak CPM/detergent lysis CPM - target leak CPM) X 100%. Specific lysis = % lysis with antibody - % lysis without antibody. Assays were performed in triplicate.

### FcγRI Modulation Assay

The M22 x 32.2 x M22 BsAb was used for modulation of EcγRI on monocytes in whole blood. The assay procedure is shown in the enclosed flow chart (see Figure 23A). Figure 23B shows that treatment with 10µg/mL of this BsAb decreased the FcγRI expression on monocytes to approximately 50% of the level prior to BsAb treatment.

### Results

### Construction and biochemical characterization of the TsAb

TsAb was made according to the flow chart depicted in Figure 16. In the first stage of the procedure, M22 was coupled to H425, treated with DTNB, and the resulting bispecific F(ab')₂ purified by gel filtration. In the second stage, this bispecific F(ab')₂ was reduced and mixed with o-PDM-treated 520C9 Fab' resulting in the TsAb, M22 x H425 x 520C9. This TsAb is depicted schematically in Figure 17. In this figure, Fab'-A represents M22, Fab'-B represents H425, and Fab'-C represents 520C9.

### Binding (Bs FACS)

To demonstrate that the TsAb, M22 x H425 x 520C9, could bind FcγRI and HER2/neu simultaneously a bispecific FACS assay was devised. This assay is depicted schematically in Figure 18A. Figure 19 shows that both the TsAb bound HER2/neu on SKBR-3 cells and soluble FcγRI simultaneously in a dose-dependent fashion. The BsAb, M22 x H425, generated negligible signal in this assay over a wide range of concentrations. To demonstrate that the TsAb, M22 x H425 x 520C9, could bind FcγRI and EGFR simultaneously a similar assay was devised using the EGFR-overexpressing cell line, A431, in the case. This assay is depicted schematically in Figure 18B. Figure 20 shows that both the TsAb and the BsAb, M22 x H425, bound EGFR on A431 cells and soluble FcγRI simultaneously in a dose-dependent fashion. The BsAb, M22 x 520C9, generated negligible signal in this assay over a wide range of concentrations.

### ADCC

The ability of the TsAb to mediate ADCC was analyzed using either SKBR-3 or A431 cells as targets. Human neutrophils cultured for 24-48 hours in the presence of IFN-γ and G-SF were used as effector cells. Figure 21 demonstrates the both the BsAb, M22 x 520C9, and the TsAb, M22 x H425 x 520C9, mediated lysis of SKBR-3 cells, whereas the BsAb, M22 x H425, did not. On the other hand, Figure 22 demonstrates the BsAb, M22 x H425, and the TsAb, mediated lysis of SKBR-3 cells, whereas the BsAb, M22 x 520C9, did not. These results demonstrated that the TsAb was capable of killing both HER2/neu and EGFR-overexpressing cells in the presence of FcγRI-expressing effector cells.

The trispecific antibody described above included M22, the murine version of the anti-FcγRI mAb. Such a trispecific antibody could be constructed using the single-sulfhydryl form of the humanized anti-FcγRI mAb, H22. The only difference being that single-sulfhydryl form is secreted as a F(ab')₂ fragment of this antibody. The single-sulfhydryl form is purified from culture supernatants utilizing ion exchange chromatography using Q-Sepharose followed by SP-Sepharose (Pharmacia, Piscataway, NJ). Once the single-sulfhydryl form of H22 is purified, the creation of a trispecific antibody using this reagent would be identical to that described above using the F(ab')₂ fragment of M22.

### Example 7: Enhanced Antigen Presentation with H22-antigen Fusion Proteins

This example demonstrates that (a) antigenic peptides genetically grafted onto the constant region of an anti-FcγRI antibody are significantly more efficient in antigen presentation of the antigen and T cell stimulation compared to the antigen alone, and (b) that antagonistic peptides genetically grafted onto the constant region of an anti-FcγRI are significantly more efficient in inhibiting T cell stimulation compared to the antagonistic peptide alone. Thus, such fusion proteins will effectively increase the delivery of peptides to antigen presenting cells (APCs) *in vivo* and will be useful in various therapeutic methods.

### Materials and Methods

### Reagents

AIM V (GIBCO, Grand Island, NY) was used as culture medium. Tetanus Toxoid (TT) was purchased from ACCURACTE CHEMICAL CO. (Westbury, NY). Sterile and low-endotoxin F(ab')₂ fragment of mouse anti-FcγRI mAb 22 and the bispecific Ab, MDXH210 (consisting of Fab' of humanized Ab 22 chemically linked to Fab' of anti-Her2/neu tumor Ag mAb 520C9) were provided by MEDAREX, INC. (Annandale, NJ). The universal Th epitope of TT, TT830-844 (QYIKANSKFIGITEL (SEQ ID NO: 6), termed as TT830 hereafter) (Valmori D. et al. (1994) J. Immunol. 152:2921-29) and the mutant form of this epitope, TT833S (QYISANSKFIGITEL (SEQ ID NO: 9), lysine at position 833 changed into serine) were synthesized and purified to >95% by PEPTIDOGENIC CO. (Livermore, CA). Another universal Th epitope of TT, TT947-967 (FNNFTVSF WLRVPKVSASHLE (SEQ ID NO: 12), referred to as TT947 hereafter), (>80% pure) (Valmori D. *supra*) was used as a control peptide in ths study. Commercially available human IgG for intravenous injection (IVI) was used in blocking experiments.

### Cells

The monocytic cell line, U937, which expresses FcγRI, was obtained from the ATCC. The method of generating CD4⁺, peptide TT830-specific T cells was modified from a previously described protocol for TT-specific T cell lines (Gosselin E.J. (1992) J. Immunol. 149:3477-81). Briefly, mononuclear cells were isolated from peripheral blood using Ficoll Hypaque. 150 x 10⁶ mononuclear cells were stimulated in 50 ml of AIM V medium with 10µM TT830. After three days' incubation at 37°C in a 5% CO₂ incubator, non-attached (mostly non-specific cells) were removed by washing the flask 1X with 10 ml of HEPES-buffered RPMI 1640; specific T cell colonies together with adherent monocytes remained in the flask. Fifty ml of AIM V plus 20 U/ml of human IL-2 (IMMUNEX, Seattle WA), and 1 ml (2%, final concentration) pooled human serum were added back to the flask. After 10-14 days of total incubation time, T cells were harvested and dead cells were pelleted through Ficoll Hypaque, yielding a highly enriched population (95-98%) of viable CD4⁺, Ag-specific T cells. The T cells were confirmed to be specific for TT830 peptide as shown in Fig. 3. Large quantities of monocytes were purified from leukophoresis packs using the cold aggregation method (Mentzer S.J. et al. (1986) Cell. Immunol. 101:132) which resulted in 80-90% purity. Both monocytes and T cells were frozen in aliquots for future use and were shown to function normally after being thawed.

### Ag presentation assay

In proliferation assays, T cells (5 x 10⁴), irradiated monocytes (3000 rad, 10⁵/well), and various concentrations of peptide TT830 fusion protein Fab22-TT830 were incubated together in a final volume of 200 µl/well in flat-bottom 96-well tissue culture plates for 2 days. 10µl (1 µCi/well) ³H-thymidine was then added to each well. After incubating overnight, plates were harvested and counted in a liquid scintillation counter. T cell proliferation was expressed as the mean counts/min (CPM) of three replicates ± SD. Background CPM (T cells and monocytes without Ag) was subtracted from all the data points. Experiments with APL were done according to similar protocols reported by Sette et al. (De Magistris (1992) Cell 68:625). Briefly, for inhibition assays, irradiated monocytes were treated with various concentrations of TT 833S or Fab22-TT833S overnight. 20nM TT830 and T cells were then added. After a further 2 days incubation, T cell proliferation was measured as described above. In "pre-pulsing" experiments, irradiated monocytes were pulsed with 20 nM TT830 4 h prior to the addition of 10 µM TT 833S or 0.1 µM Fab22-TT833S: After overnight incubation, T cells were then added. After a further 2 days incubation, T cells were stimulated with irradiated monocytes and TT833S or Fab22-TT833S for 1 day, recovered after centrifugation over Ficoll Hypaque, and restimulated with monocytes and various concentrations of TT830 for 2 days. T cell proliferation was then measured by the incorporation of ³H-thymidine and the average CPM of three replicates was plotted. In some cases, the percentage of inhibition was calculated by the formula: % inhibition = (CPMₙₒ inhibitor^{-CPM}inhibitor^{)/CPM}no inhibitor^{X} 100. All experiments were repeated at least three times.

### Staining and Flow Cytometry

Staining procedures were adapted from those previously described (Gosselin E.J. et al. (1990) J. Immunol. 144-1817-22). Briefly, to individual wells of a 96-well plate at 4°C, 30 µl of RPMI+ 1mg/ml BSA containing one of the proteins Fab22-TT830, Fab22-TT833S, or the BsAb MDXH210 at varying concentrations. After 1 h incubation at 4° C, plates were centrifuged, the supernatants discarded, and the cells washed three times with PBS/BSA at 4°C. Cells were then incubated for I h with 40 µl/well of FITC-labeled F(ab')₂ goat anti-human IgG (JACKSON IMMUNORESEARCH LABORATORIES, INC. West Grove, PA) followed by three washes with PBS/BSA and resuspended in PBS/BSA containing 1% paraformaldehyde (KODAK, Rochester, NY). Cells were then examined by FACScan (BECTON DICKINSON & CO., Mountain View, CA), and mean fluorescence intensity (MFI) was measured.

### Cytokine measurement

Supernatants were collected from the 96-well plates of Ag presentation assays after 2 days stimulation and frozen until used. The levels of IFN-γ and IL-4 from these samples were measured by specific ELISA. Ab pairs for the IFN-γ and IL-4-specific ELISA were purchased from PHARMINGEN (San Diego, CA). ELISA assays were performed according to the protocol provided by the manufacturer.

### Generation of H22-TT peptide fusion proteins

In order to generate fusion proteins Fab22-TT830 and Fab22-TT833S, synthetic oligonucleotides encoding each peptide were separately engineered into the hinge region in the heavy chain of humanized anti-FcγRI mAb 22 (H22) according to the method set forth below.

### Expression and cloning vectors

mAb 22 has been humanized by grafting its CDR regions into a human IgG1 framework (see above and Graziano R. F. et al. (1995) J. Immunol. 155:4996-5002). The expression vector for the genomic clone of the heavy chain (pSVgpt) of H22 was modified to allow incorporation of the coding sequence for other molecules, in this case, the TT peptides. The BamHI fragment of this vector containing CH1. hinge, and newly engineered XhoI and NotI cloning sites (see Figure 2) was inserted into the BamHI site of pUC 19 to generate the vector pUC19/H22CH1(X+N). This vector was used to clone oligonucleotide sequences encoding TT peptides, as described below.

The oligonucleotide sequences encoding the tetanus toxin (TT) peptides were designed to have a XhoI site on the N-terminus and a NotI site on the C-terminus of the coding region (Figure 24A). These oligonucleotides were synthesized and purified by GENOSYS Biotechnologies (The Woodlands, TX). The synthetic oligonucleotides were then annealed and ligated into the cloning vector pUC 19/H22CH1 (X+N). Clones which had incorporated the coding sequences for TT peptides were screened by restriction mapping. The BamHI fragment containing CH1, hinge, and TT830 or TT833S was then cut out of pUC19 and inserted into the expression vector which already contained VH. The final expression construct of H22 heavy chain fused with TT peptides is shown in Figure 24B.

### Expression of the H22-TT fusion proteins

The murine myeloma NSO (ECACC 851-10503) is a non-Ig synthesizing line and was used for expression of the H22-TT fusion proteins. First, NSO cells were transfected with the pSVhyg vector containing the H22 light chain coding sequence. The H22 light chain expressing NSO cells were then transfected with the expression vector construct containing the H22 H-chain Fd sequence fused in frame to the TT coding sequences (Figure 24B). A BioRad Gene Pulser electroporation apparatus was used to carry out the transfection employing 200 v and 960 µFarad. One or two days after transfection, mycophenolic acid (0.8 µg/ml; SIGMA) and xanthine (2.5 µg/ml; SIGMA) were added to the media to select transfectants which had successfully taken up the expression vectors. Individual colonies were isolated based on the binding activity of the culture supernatants to FcγRI on U937 cells as demonstrated by flow cytometry. The positive colonies were subcloned by limiting dilution.

### Purification of the Fab22-TT fusion proteins

Clone pW5 expressing the Fab22-TT830 fusion protein and clone pM4 expressing the Fab22-TT833S fusion protein were expanded in roller bottle cultures. The supernatants were clarified and concentrated. Small scale purification was performed by affinity chromatography on an anti-H22 affinity column. SDS-PAGE analysis of a 5-10% acrylamide gradient gel under non-reducing conditions showed that fusion proteins were >90% pure and had a molecular weight of 50 kDa as expected. Protein concentration was determined by absorbance at 280 nm using the extinction coefficient of IgG Fab' = 1.53.

### Results

### H22Fd-TT fusion proteins bind to U937 cells

The ability of the H22 fusion proteins, Fab22-TT830 and Fab22-TT833S, to bind to FcγRI was examined first. A previously described bispecific Ab, MDXH210, which contains the same FcγRI-binding component (Fab' of humanized mAb 22) (Valone F. H. et al. (1995) J. Clin. Oncol. 13:2281-92), was used as a positive control. Binding of fusion proteins and MDXH210 to U937 cells, which constitutively express FcγRI, was measured by staining with FITC-labeled goat Ab specific for human IgG and flow cytometry. As indicated in Figures 24A and 24B, fusion proteins Fab22-TT830 and Fab22-TT833S bound to U937 cells in a dose-dependent manner similar to MDXH210. The binding of fusion proteins was completely blocked by murine anti-human FcγRI mAb 22 F(ab')₂, demonstrating the specificity of fusion proteins for FcγRI.

### H22Fd-TT fusion protein enhances presentation of TT peptide by 100-1000 fold.

The fusion protein, Fab22-TT830 was used in Ag presentation assays to determine whether the Th epitope, TT830, when expressed in the constant region of H22, could be effectively presented by monocytes to autologous T cells. As shown in Figure 26, about 1,000-fold less Fab22-TT830 was required than TT830 peptide alone to achieve the same level of T cell proliferation. In addition, Figure 26 shows that the presentation of Fab22-TT830 was about 10,000-fold more efficient than the presentation of the intact TT, suggesting that the enhanced presentation of Fab22-TT830 did not merely result from higher molecular weight nor increased stability of Fab22-TT830 as opposed to TT830 peptide. Another antigenic TT epitope, TT947, failed to stimulate the T cells, confirming that the T cells were specific for TT830 peptide. These results thus provide clear evidence that Th epitopes expressed in the constant region of H22 can be effectively and specifically presented.

### Blockade of FcγR1 on monocytes abrogates the enhancement of Ag presentation by the H22Fd-TT fusion protein

To directly determine whether the enhancement of peptide presentation through the use of the fusion protein is FcγRI-mediated, binding of Fab22-TT830 to FcγRI on Ag-presenting monocytes was blocked by treating monocytes with mAb 22 F(ab')₂ for 1 h prior to the addition of Fab22-TT830 or TT830 peptides. Enhancement of peptide presentation by the fusion protein was abrogated by mAb 22 F(ab')₂, whereas presentation of TT830 was unaffected (Figure 27). The fact that the binding of mAb 22 F(ab')₂ to FcγRI did not lead to an enhancement of the presentation of free peptides implies that binding of mAb 22 to FcγRI alone did not alter the functional state of monocytes in a way that enhanced Ag presentation. Therefore, linkage of the peptide to anti-FcγRI Ab 22 appears to be necessary for the observed enhancing effects on Ag presentation, suggesting that the enhanced presentation is probably a result of efficient Ag capture through FcγRI.

### Enhancement of peptide presentation by H22 is not affected by the presence of human IgG

Under physiological conditions, the ligand-binding domain of FcγRI is saturated by IgG which blocks efficient targeting of AgAb to this receptor. A unique advantage for using derivatives of mAb 22 to trigger FcγRI function is that Ab 22 binds to an epitope outside the ligand binding domain. Therefore, functions triggered by mAb 22, such as ADCC, phagocytosis and Ag presentation are not inhibited by physiological levels of IgG (Gosselin E. J., supra, Guyre P. M. (1989) J. Immunol. 143-1650-55). Similarly, the enhanced presentation of the TT830 peptide using the fusion protein Fab22-TT830 was not inhibited by IgG (Figure 28), suggesting that H22-based fusion proteins is also an effective way to target peptide Ags to FcγRI *in vivo.*

### IFN-γ and IL-4 production is increased following H22Fd-TT fusion protein-enhanced Ag presentation

Upon activation, T cells not only undergo clonal expansion through proliferation, but also produce cytokines such as IFN-γ and IL-4 to exert their effector function of B cell differentiation and monocyte activation (Paul W. E. and Seder, R. A. (1994) Cell 76:241-251). Therefore, the production of IFN-γ and IL-4 following H22 fusion protein-enhanced Ag presentation was examined. As shown in Figures 28A and 28B, both IFN-γ and IL-4 production levels were enhanced by Fab22-TT830, especially at suboptimal Ag concentrations. However, in these experiments, the enhancement for cytokine production (about 20-fold) was less than that for T cell proliferation (about 600-fold).

Thus, Th epitopes expressed in the constant region of anti-FcγRI mAb H22 can be effectively processed and presented by human monocytes, leading to enhanced T cell activation and cytokine production.

### Presentation of APL. TT833S and Fab22-TT833S fails to stimulate T cell proliferation

Peptides containing one or two amino acid changes from native T cell epitopes, termed Altered Peptide Ligands (APL) by Allen and coworkers, have been shown to be agonists, partial agonists, or antagonists for T cell activation (Sette et al. (1994) Ann. Rev. Immunol. 12:413 and Evavold et al. (1993) Immunol. Today 14:602). Recognition of APL by specific T cells through TCR in some cases triggered partial signal transduction and resulted in (i) inhibition of T cell stimulation by superantigen (Evavlold et al. (1994) Proc. Natl. Acad. Sci. U.S.A. 91:2300), T cell anergy (Sloan-Lancaster et al. (1993) Nature 363:156 and Sloan-Lancaster et al. (1994) J. Exp. Med. 185:1195), or (iii) modulation of Th1/Th2 differentiation (Nicholson et al. (1995) Immunity 3:397; Pfeiffer et al. (1995) J. Exp. Med. 181:1569; and Windhagen et al. (1995) Immunity 2:373). Partial agonists have been shown to stimulate some T cell functions such as IL-4 production by T cells, but not others such as T cell proliferation (Evabold et al. (1991) Science 252:1308). Partial agonists also can induce anergy. Certain APL do not trigger any detectable signaling events upon interaction with TCR, but can function as TCR antagonists to inhibit T cell proliferation in response to wildtype peptide antigen and are thus called TCR antagonists (De Magistris et al. (1992) Cell 68:625 and Ruppert et al. (1993) Proc. Natl. Acad. Sci. USA 90:2671.

This example demonstrates that the peptide TT833S, an antagonist peptide for T cell epitope TT830 of tetanus toxin and Fab22-TT833S fail to stimulate T cell proliferation. As shown in Figure 30, even at doses as high as 100 µM for TT833S and I µM for Fab22-TT833S, no significant proliferation of TT830-specific T cells was observed. This indicates that changing lysine to serine at position 833 of the TT830 peptide eliminated T cell reactivity of this T cell epitope. Additionally, when peptides TT830 and TT833S were simultaneously presented to TT830-specific T cells, T cell proliferation in response to TT830 was inhibited by TT833S in a dose-dependent fashion, showing that TT833S can function as an antagonist for TT830-specific T cells (Figure 31).

### Fab22-TT833S is at least 100 times more effective than TT833S in inhibiting T cell activation

This example compares the relative efficiency of TT833S and Fab22-TT833S in inhibiting T cell proliferation in response to TT830. As shown in Figure 32, Fab22-TT833S was about 100 times more effective than TT833S in inhibiting TT830-stimulated T cell proliferation. This suggests that the APL, TT833S, when expressed in the constant region of mAb H22, can be correctly and effectively presented by APC. The increased antagonistic efficacy of fusion protein Fab22-TT833S on T cell proliferation probably reflects more efficient Ag capture mediated by FcγRI as compared to free peptides.

### Inhibition of T cell activation is mediated by competition for T cell receptor binding rather than for MHC Class II binding.

The antagonist effects of APL TT833S and fusion protein Fab22-TT833S might be through competition at the level of MHC-binding or TCR-binding, or both. To gain insight into the mechanisms involved, "pre-pulsing" experiments, first described by Sette and co-workers (DeMagistris, M.T. et al. (1992) Cell 68:625-634), were performed. This experimental setting allows agonist (TT830) to bind to MHC Class II in the absence of competition from the inhibitor (TT833S) and thus, only TCR antagonists but not pure MHC blockers would be effective in inhibiting agonist-stimulated T cell proliferation. Ag-presenting monocytes were pulsed with suboptimal (20 nM) TT830 for 4 h to allow TT830 to bind with MHC Class II in the absence of competition from TT833S. The APL, TT833S or Fab22-TT833S, was then incubated with the pre-pulsed monocytes for an additional 16 h. Responding T cells were added and their proliferation was measured as described above. Even under such conditions where MHC blockade plays a minimal role, T cell proliferation was still inhibited (Figure 33). Thus, the inhibition appears to be a result of competition for T cell receptor rather than for MHC Class II binding.

### Presentation of TT833S and Fab22-TT833S fails to stimulate the production of IL-4 and IFN- γ by T cells

In some circumstances, APL can stimulate T cell cytokine production but not proliferation (Evavold B.D. and Allen P. M. (1991) Science 252:1308-1310). To determine if presentation of TT833S simulates the production of cytokines, the level of IL-4 and IFN-γ in supernatants obtained from Ag presentation assays was determined. As shown in Figure 34, both TT833S and Fab22-TT833S were ineffective in stimulating IFN-γ and IL-4 production by T cells.

### Presentation of TT833S and Fab22-TT833S does not lead to T cell anergy

Allen and co-workers reported that interaction of TCR with some APL-MHC Class II complexes led to T cell anergy (Sloan-Lancaster J. et al. (1993) Nature 363:156-159, Sloan-Lancaster J. et al. (1994) J. Exp. Med. 180:1195-1205). An experimental scheme similar to theirs was used to determine whether presentation of TT833S could also cause T cell anergy. As shown in Figure 35, when T cells were recovered after incubation with APC and TT833S or Fab22-TT833S for 1 day, 2 days, or 4 days, they responded to subsequent antigenic challenge as well as T cells which had been incubated with APC alone. Therefore, the antagonist, presented via the use of either peptide alone or Fab22-TT833S, did not cause T cell anergy. Furthermore, the same percentage of viable T cells (about 50%) was recovered from cultures with no peptides, TT833S or Fab22-TT833S, suggesting that presentation of TT833S also did not increase T cell death.

The observation that immunogenicity is increased by about 1000 fold by targeting antigenic peptides to FcγRI using an anti-FcγRI mAb 22-based fusion protein indicates that such fusion proteins will be useful for peptide-based vaccines for, e.g., infectious diseases and cancer. Engineering peptides into the constant domains of human mAb that are specific for particular APC surface molecules represent a general approach to increase the antigenic potency for peptide-based vaccines.

Moreover, the observation that the FcγRI-targeted antagonistic peptide inhibited proliferation of TT830-specific T cells even when APCs were first pulsed with native peptide, a situation comparable to that which would be encountered *in vivo* when attempting to ameliorate an autoimmune response show that targeted presentation of antagonistic peptides can be used as Ag-specific therapies for disorders, e.g., T cell-mediated autoimmune diseases. APL-based treatment will provide an antigen-specific immunotherapy for T cell mediated autoimmune diseases such as rheumatoid arthritis and multiple sclerosis. Furthermore, the use of a fusion protein having one binding specificity to an FcγRI and a peptide which is a partial agonist of an antigen involved in immune disorders characterized by excessive immune responses will be useful in treating such immune disorders by inducing antigen-specific anergy. Thus, the invention provides methods for treating various immunological disorders, by providing a method allowing for increased antigen presentation of antigens, which either stimulate T cells, block T cell proliferation and/or cytokine secretion, or which induce anergy in the T cells.

### Example 8: Functional Single Chain anti-FcγRI-anti-CEA Bispecific Molecules

This example demonstrates that a recombinant bispecific single chain molecule comprising a humanized anti-FcγRI antibody fused to an anti-carcinoembryonic (anti-CEA) antibody is capable of binding to FcγRI and to CEA.

Figure 37 is a schematic representation of mammalian expression constructs encoding bispecific single chain molecules (constructs 321 and 323) having one binding specificity for the FcγRI and one binding specificity for carcinoembryonic antigen (CEA) that were prepared. The amino acid sequence of the bispecific single chain molecule H22-anti-CEA encoded by construct 321 (SEQ ID NO: 16) and the nucleic acid encoding this fusion protein (SEQ ID NO: 15) are shown in Figure 40. The bispecific single chain molecule H22-anti-CEA encoded by construct 323 differs from the fusion protein encoded by construct 321 only in that the VH and VL chains of H22 were switched.

A mammalian expression construct encoding a single chain antibody having one binding specificity for the FcγRI (construct 225) was also prepared. The amino acid sequence of the single chain antibody H22 encoded by construct 225 (SEQ ID NO: 14) and the nucleic acid encoding this single chain antibody (SEQ ID NO: 13) are shown in Figure 39.

Each of these constructs were cloned into the Hind III and XbaI sites of pcDNA3 (InVitrogen), from which expression is driven from the CMV promoter. Each of these constructs also contain a nucleic acid sequence encoding a peptide from c-myc and a hexa-histidine peptide, which were used for purification of the recombinant protein from the cell culture. The c-myc tag corresponds to amino acids 410 to 420 of human c-myc (Evan et al. (1985) Mol. Cell. Biol. 5:3610). The anti-CEA single chain antibody, termed MFE-23, is further described in Casey et al. (1994) J. Immunol. Methods 179:105 and Chester et al. (1994) Lancet 343:455.

The single chain bispecific molecules H22-anti-CEA and the single chain H22 antibody were used in binding assays, performed as follows. ELISA plates are coated with CEA and the blocked with 5% PBA. Supernatants of the cells transfected with the constructs encoding the single chain molecules (transfectomas) were added to the plates, soluble FcγRI/IgM-µ (supernatant from COS transfected cells, described above) was added and binding was detected by incubation of the plates with alkaline-phosphatase (AP) conjugated goat anti-human IgM, development with PNPP, and reading of the plate at 405-650 nm.

The results are presented in Figure 38. The results indicate that the single chain bispecific H22-anti-CEA molecules encoded by constructs 321 and 323 bind both FcγRI and CEA. On the other hand, the single chain H22 antibody (encoded by construct 225) does not bind both FcγrI and CEA, as expected.

### Example 9: Targeting CD64 (FcγRI) to Induce and/or Enhance Antigen Processing and Presentation

In order to determine whether directing a normally non-immunogenic antigen (e.g., a self antigen) to human CD64 (FcγRI) can overcome immunologic non-responsiveness *in vivo,* several multimer complexes containing such antigens linked to F(ab')2 antibody fragments directed to Fc receptors on antigen presenting cells were prepared and tested as described below. In particular, mice transgenic for human CD64, as well as their nontransgenic littermates, were immunized with the F(ab')2 fragment of the murine anti-human CD64 mAb, M22 (produced by the hybridoma having ATCC Deposit No. HB-12147). The mice were bled seven days after receiving their fourth biweekly immunization. Upon analysis by ELISA, three of the six transgenic mice, but none of the six non-transgenic littermates, exhibited significant anti-M22 Id specific antibody titer. This antisera was M22 specific and did not react with other murine antibodies.

To determine whether multimer complexes of the M22 Fab' would induce more efficient internalization of antigen and, thus, enhance antigen presentation and a stronger anti-M22 Id immune response, multimers of the M22 Fab' molecule were synthesized both chemically and genetically and tested. These multimers comprise antigens which can be chemically linked to 22 F(ab')2 (or other Fc receptor binding agents) and additional 22 Fab' molecules. The final multimer consists of several molecular species that contain, e.g., multiple 22 Fab' arms (e.g., 2 or more) and one or more antigen molecules. These species may be purified by size exclusion or affinity chromatography if desired. Figure 41 shows a schematic representation for the development of such chemically linked multimeric target antigens.

Chemically synthesized multimers were prepared by incubating F(ab')2 of M22 with a 20 molar excess of succinimidyl 4-(N-maleimidomethyl)cyclohexane-1-carboxylate (SMCC) at room temperature for 2 hours. Free SMCC was removed from the resulting complex, F(ab')2-SMCC, by G-25 chromatography. Antigens with free thiol (SH) groups were added to the M22 F(ab')2-SMCC in a 1:1 molar ratio. The resulting mixture was incubated overnight at room temperature. The reaction was stopped by adding an excess of iodoacetamide and the resulting conjugate was purified by size exclusion chromatography.

### M22 F(ab')2+ - antigen multimers enhance/induce an immune response

Figure 42A shows a non-reducing SDS-PAGE gel containing purified M22 F(ab')2 (lane 1) and the chemically-linked M22 Fab' multimer (lane 2). The multimer consisted of several molecular species representing F(ab')3, F(ab')4, F(ab')5, F(ab')6, and higher molecular weight molecules.

To determine the ability of M22 F(ab')2, compared to the multimer, M22 F(ab')3⁺, to mediate internalization of human CD64, various concentrations of either M22 F(ab')2 or M22 F(ab')3⁺ were incubated for two hours with macrophages isolated from human CD64-transgenic mice, human CD64 surface expression was detected by the FITC-labeled anti-human CD64 mAb M32.2 (produced by the hybridoma having ATCC Deposit No. 9469) which binds-to a site on human CD64 which is distinct from the M22 epitope. As shown in Figure 42B, incubation with the M22 F(ab')2 did not result in significant reduction of human CD64 from the surface of the macrophages. However, incubation with the F(ab')3⁺ at 37°C led to up to 50% reduction in human CD64 expression in a dose-dependent fashion. Incubation with the F(ab')3⁺ at 4°C did not lead to a reduction in human CD64 expression, demonstrating the temperature-dependence of this phenomenon.

Further experiments were performed to determine if modulation would occur *in vivo*. Human-CD64 transgenic mice were injected with either M22 F(ab')2 or M22 F(ab')3⁺ and bled one hour later. Surface expression of human CD64 was significantly reduced on circulating monocytes from the M22 F(ab')3⁺, but not the M22 F(ab')2 immunized animals. It was hypothesized that the reduction of surface human CD64 expression represented internalization of the receptor along with the F(ab')3⁺ multimer.

The ability of human CD64-transgenic and nontransgenic littermates to generate an anti-M22 idiotype ("Id") response after three immunizations with the M22 F(ab')3⁺ multimer was next studied. The data shown in Figure 43A demonstrates that high titers of M22-specific antibody were generated in all of the human CD64-transgenic mice (n=12) immunized three times (25 µg/immunization) with the multimer, but no M22-specific antibody was generated in any of the nontransgenic littermates immunized similarly (n=10). The immune response in human CD64-transgenics that was elicited by the M22 F(ab')3⁺ multimer was signficantly -better than that elicited by the M22 F(ab')2. All of the multimer-immunized mice responded and fewer immunizations were required (three versus four) to generate an immune response immunizing with the multimer as compared to the F(ab')2. These results demonstrate that the Fab' multimer complexes lead to better internalization of an antigen via human CD64, resulting in an enhanced antigen-specific immune response. In fact, the data shown in Figure 43B demonstrates that immunizing human CD64-transgenic mice with as little as 0.25 µg of the multimer still leads to a detectable immune response in most of the mice.

It is difficult to observe a potent immune response to many tumor-associated antigens since the immune system is often tolerant to these antigens. In order to determine whether directing such a normally non-immunogenic antigen to human CD64 resulted in an immune response to this antigen, a model antigen, the Fab' fragment of the murine monoclonal antibody, 520C9, was coupled to the M22 multimer.

After immunization of either human CD64 transgenic or their nontransgenic littermates, both anti-520C9 Id titers and anti-M22 Id titers were assessed. As shown in Figure 44A, seven of eight immunized human CD64 transgenic mice developed both strong anti-520C9 and anti-M22 titers. None of the seven immunized nontrangenic mice -developed measurable titers to either 520C9 or to M22. These data demonstrate that by coupling a normally non-immunogenic protein to human CD64 so that it is internalized by human CD64⁺ antigen presenting cells leads to a potent immune response to the antigen. Several groups have shown that developing immunity specific for the idiotype expressed by surface Ig on B cell lymphoma cells can lead to potent tumor cell-specific immunity. The example demonstrates that a specific anti-520C9 Id response can be readily elicited by directing the 520C9 idiotype to human CD64.

The quality of the immune response to a given antigen, particularly a tumor-associated or viral antigen, can be as important as the quantity of the response. T helper cells have been divided into Th1 and Th2 depending on the type of cytokines they secrete and also the type of help that the provide to B cells when stimulated. Th1 cells secrete IL-2 and IFN-γ when stimulated and usually stimulate B cells to secrete antibody of the IgG2a isotype. Th2 cells secrete IL-4 and IL-5, when stimulated, and usually stimulate B cells to secrete antibody of the IgG1 or IgE isotype. In general, Th1 cells are believed to stimulate the cellular arm of the immune response whereas Th2 cells stimulate the humoral arm. The titer of 520C9-specific antibody, of either the IgG1 or the IgG2a isotype, was examined using isotype-specific developing reagents after having immunized human CD64-transgenic mice with 520C9 coupled to the M22 multimer. The data shown in Figure 44B demonstrates that both a strong IgG1 as well as a strong IgG2a Id-specific titer was elicited, demonstrating the ability of this method of immunization to activate both a Th1 and a Th2 response.

### H22sFv-H22sFv-32.2sFv-antigen multimer induces immune response

Figure 45(a) shows the map of a vector encoding a genetically-linked multimeric, targeted antigen. This vector encodes for a protein with two sFv regions from humanized anti- FcγRI antibody, H22 (produced by the hybridoma having ATCC Deposit No. CRL 11177), and one sFv region from antibody M32.2, all linked together to an antigen. The resulting fusion protein, H22sFv-H22sFv-32.2sFv-antigen (shown in Figure 45(b)), is capable of binding to FcγRI at multiple sites, and thereby induce internalization via aggregation of the receptor.

The H22sFv-H22sFv-32.2sFv-antigen multimer was made using murine gp75 (Trp-1 melanoma antigen) as an antigen for model studies in transgenic mice. It was found that this fusion protein binds FcγRI efficiently and induces internalization of the receptor. As shown in Figure 46, chemically-linked M22Fab x M22Fab x M32Fab and genetically-linked H22sFv-H22sFv-32.2sFv-antigen each induced internalization of FcγRI. The study was performed by addition of samples to IFN-γ-treated U-937 cells for 2 hours at 37°C. FcγRI expression was then measured by staining cells with Human IgG1-FITC for 1 hour at 4 °C, and samples were analyzed by FACscan. Percent (%) modulation was calculated by the formula: [1-(MFI of sample/MFI of control)] x 100%, wherein MFI is mean fluorescence intensity.

### H22sFv2-antigen binds FcγRI with affinity approximately equal to H22Fab2-antigen

Figure 47(a) shows a map of a vector encoding a fusion protein made up of two H22 sFv regions genetically linked together to an antigen. The resulting fusion protein, H22sFv-H22sFv-antigen (shown in Figure 47(b)), is capable of binding to two FcγRI molecules. This fusion protein should have greater affinity for FcγRI than fusion proteins consisting of just one H22 sFv. The ability of this multimer to bind to bind two FcγRI molecules may also induce greater internalization of the receptor.

To test this hypotheses, (i.e., whether the H22sFv-H22sFv-antigen fusion binds to FcγRI with greater affinity than a single H22sFv-antigen fusion), a competition experiment was performed. U937 cells, previously treated with IFN-γ to enhance expression of FcγRI, were stained with an antibody H22 and phycoerythrin conjugate. As shown in Figure 48, the conjugate was inhibited by various concentrations of H22 Fab, H22sFv2-EGF fusion protein, or H22 Fab2. As also shown in Figure 48, H22sFv2-EGF binds FcγRI with an affinity approximately equal to that of H22Fab2. It has been previously shown that H22 Fab' and H22 sFv have similar affinity for FcγRI (Goldstein et al. (1997) J. Immunol.*).*

### H22sFv-H22sFv-H22sFv-CEA induces internalization of FcγRI

Figure 49(a) shows a map of a vector which encodes a multimer fusion protein containing three-H22 sFv fragments linked to an antigen. The fusion protein, H22sFv-H22sFv-H22sFv-CEA (shown in Figure 49(b)), contains the tumor antigen CEA linked with the 3 H22sFvs. The fusion protein should bind FcγRI with high affinity (due to the trivalent binding of three sFv molecules), and should also lead to FcγRI internalization by aggregation of three FcγRI molecules.

The ability of the genetically-linked H22sFv-H22sFv-H22sFv-CEA fusion to induce internalization of FcγRI was tested as previously described, and the results are shown in Figure 50. Importantly, the H22Fab-CEA did not mediate internalization at similar concentrations, likely because it binds monovalently to FcγRI. The example was performed by addition of samples to IFN-γ-treated U-937 cells and incubating for either one hour or overnight at 37°C. FcγRI expression was then measured by staining cells with M32.2-FITC for one hour at 4 °C, and samples were analyzed by FACscan.

### Example 10: Human Antibody Fusion Proteins Directed to CD89 (FcαR) and Tumor Antigens(e.g., EGF Receptor) Promote Cell Mediated Cytotoxicity of Tumor Cells

The following studies involve the production and characterization of bispecific fusion constructs which induce effector cell mediated killing of tumor cells expressing EGF Receptor (EGF-R). The constructs include EGF linked to an anti-CD89 (anti-FcaR) antibody (ScFv or Fab'). Therefore, the constructs activate the effector functions of CD89 expressing cells that have been targeted to tumor cells.

CD89 (FcαRI) is a receptor which binds to the Fc portion of IgA, the most abundant Ig in the human body. CD89 is constitutively expressed primarily on cytotoxic immune effector cells including polymorphonuclear leukocytes (PMN), monocytes, macrophages, neutrophils and eosinophils. Both Ag-complexed and monomeric IgA1 and IgA2 bind CD89, suggesting that the receptor may be saturated with monomeric IgA in vivo. Cross-linking CD89 on myeloid effector cells by mAbs specific for epitopes within or outside the ligand binding site stimulates degranulation, superoxide release, secretion of inflammatory cytokines, endocytosis and phagocytosis. CD89 may, therefore, be a clinically relevant trigger receptor on cytotoxic immune effector cells.

Members of the type I growth factor receptor family are tyrosine-kinase linked receptors which have been reported to be over-expressed during the development of various cancers. One member of this family is the receptor for epidermal growth factor (EGF-R, *c-erb*B*-*1 gene product). EGF-R is over-expressed in almost all head and neck tumors, about one third of breast and ovarian tumors and can be over-expressed in cancers of the prostate, kidney, bladder, lung, brain, pancreas, and gastrointestinal system. Despite the existence of the receptor on some normal cells such as keratinocytes and hepatocytes, the over-expression of EGF-R on tumor cells may be useful for directing specific therapies to the tumor. Directed therapies may include a mAb specific for EGF-R, or one of the ligands for the receptor, such as EGF. EGF is a modulator of cell division and differentiation functions of various cell types expressing EGF-R. EGF is a 53 amino acid, unglycosylated polypeptide which contains 3 disulfide bonds, and is proteolytically processed from a 1207 amino acid precursor.

### Generation of Fusion Constructs

To generate bispecific fusion proteins, HuMAb-Mouse™ technology, as previously described herein, was utilized to generate a completely human CD89 mAb. This mAb, 14A8 (also referred to as 14.1), binds to an epitope that is distinct from the Fc binding site while retaining the ability to trigger effector functions. Thus the antibody overcomes the potential blockage of the ligand binding site caused by saturating monomeric IgA. Here the fab' and sFv fragments of mAb 14A8 were fused to EGF. In brief, the 14A8 hybridoma cell line was obtained from HuMAb mice immunized with soluble CD89. The v-regions were cloned using RT=PCR from RNA isolated from the cell line. DNA sequencing analysis revealed that the antibody consists of the 30.3 VH with DN1 D-segment and JH4 J-segment, and the L 18 Vk with JK3 J-segment.

Using DNA encoding the variable regions from the fully human anti-CD89 mAb generated from the HuMAb-Mouse™, antibody fusion proteins which function as bispecific molecules (BMS) were constructed and expressed. As shown in Figure 51, these molecules consisted of an antibody fragment which binds to the receptor for the Fc portion of human IgA (FcαRI, CD89) at an epitope that is distinct from the Fc binding site but still triggers effector cell functions, and an arm which binds to the epidermal growth factor receptor (EGF-R), which is overexpressed on various tumor cell lines. To make the BSM, Fab or sc-Fv fragment of mAb 14A8 (anti-CD89) was fused via a flexible peptide linker to EGF, a ligand for EGF-R. The variable regions of mAb 14A8 were cloned and characterized using RT-PCR and DNA sequencing.

The Fab fusion construct was made by inserting 14A8 VH DNA into an expression vector containing the cDNA of human IgG1 CH1 and hinge regions fused to EGF. 14A8 VL DNA was likewise inserted into an expression vector containing the cDNA of the human CL region. The sc-Fv fusion protein was also expressed as a single chain from one vector. As shown schematically in Figure 51, PJZ906 (14Afd-EGF) and pJZ907 (14A8 L-chain) were cotransfected by electroporation into NSO cells, and selected in media containing both G418 and hygromycin. PJZ909 (14A8sFv-EGF) was similarly transfected and selected in media containing G418. Cell lines expressing fusion protein were subcloned by limiting dilution cloning. To purify the fusion constructs, supernatant of cell lines expressing fusion protein was run over a protein L column, and approximately 2 µg purified protein was loaded onto a 4-15% tris-glycine gel under reducing and non-reducing conditions.

### Characterization of Fusion Construct Binding to Tumor Cells

As shown in Figure 52 (A and B) and Figure 53, flow cytometry experiments confirmed that the antibody fragment and the EGF portion in these BSM bind specifically to their respective cell surface receptors. Figure 52(A) shows 14A8fab'-EGF fusion protein binding to EGF-R on A431 cells. Figure 52(B) shows 14A8sFv-EGF binding to A431 cells (stained with goat anti-human IgG Fab-2 conjugated to phycoerythrin). The fab-2 fragment of 14A8, which does not contain EGF, was used as control. Figure 52(C) shows that the 14A8 fusion proteins and commercially obtained EGF compete for binding to A431 cells with an EGF-FITC conjugate (7nM). Figure 53(A) shows that 14A8fab'- EGF fusion protein binds to CD89 expressing U937 cells (stained with goat anti-human IgG Fab-2 conjugated to phycoerythrin). The fab-2 fragment of humanized mAb H415, which is specific for EGF-R, was used as control. Figure 53(B) shows that 14A8sFv-EGF fusion protein also binds to CD89 expressing U937 cells. The experiment was done in a manner similar as in Figure 53(A), except that various concentrations of 14A8sFv-EGF were assessed for their ability to inhibit the binding of 14A8fab'-EGF fusion (23 nM). H22sFv-EGF fusion protein was again used as control. mAb H22 binds to CD64 (FcγRI), a different Fc receptor on U937 cells.

### Effector Cell-Mediated Lysis of EGF-R Expressing Tumor Cells

Chromium release (cell lysis) assays confirmed that both 14A8fab'-EGF and 14A8sFv-EGF fusion constructs mediated specific lysis of EGF-R expressing tumor cells with purified CD89 expressing polymorphonuclear (PMN) leukocytes, monocytes or whole blood effector cells in a dose-dependent manner.

As shown in Figures 54(A), 54(B) and 54(C) and Figure 55, 14A8 fusion proteins mediated cytotoxicity of A431 cells using monocytes (54A), PMNs (54B), and whole blood (54C). Chromium release assays were done with an incubation period of 16-18 h and using an effector to target ration (for monocytes and PMN) of 100:1. The fab-2 fragment of 14A8 was used as control. As shown in Figure 56, 14A8fab'-EGF fusion (1 µg/ml) (56A) and 14a8Sfv-EGF fusion (1µg/ml) mediated cytotoxicity of A431 cells is inhibited by the fab-2 fragment of 14A8 (40 µg/ml). This demonstrates that the cell lysis by the fusion proteins is specifically mediated by binding to CD89.

Overall, the foregoing studies describe the generation of fusion proteins by genetic means linking EGF to the Fab' or sFv fragments of 14A8, expressing the constructs in mammalian cell culture, and purifying the constructs to near homogeneity using protein L chromatography. These fusion proteins are minimally immunogenic in humans because 14A8 is fully human and EGF is of human origin, which may enhance the therapeutic efficacy of the fusion proteins. The foregoing studies also demonstrate that these fusion proteins, 14Afab'-EGF and 14A8sFv-EGF, are capable of binding to both CD89 and EGF-R expressing cells. Importantly, the studies further demonstrate that, in the presence of CD89-expressing immune effector cells, these fusion proteins mediate killing of EGF-R over-expressing cells in a dose-dependent manner. Tumor cell lysis was also evidenced in the presence of whole blood.

### Example 11: Bispecific and Trispecific Molecules Containing Human Antibody Binding Portions Directed to CD89 (FcαR) and Tumor Antigens (e.g., EGF Receptor and/or HER2) Promote Cell Mediated Cytotoxicity of Tumor Cells

The following studies involve the production and characterization of bispecific and trispecific molecules which induce effector cell mediated killing of tumor cells expressing EGF Receptor (EGF-R) and/or HER2/neu (also referred to as HER2). The bispecific constructs include a human monoclonal anti-CD89 (anti-FcαR) antibody (ScFv or Fab'), referred to as 14.1, linked to a human monoclonal anti-HER2 antibody (ScFv or Fab'), referred to as 3.F2. The trispecific construct included the bispecific anti-CD89 (ScFv) x anti-HER2 (ScFv) construct, fused to EGF. These constructs activate the effector functions of CD89 expressing cells that have been targeted to tumor cells. They also provide the advantage of being fully human.

### Generation of Bispecific and Trispecific Fusion Constructs

Figures 57 and 58 show selected bi- and tri-specific molecules of the invention and their parent human antibodies (HuMabs). Figure 57 shows single-chain (ScFv) bispecific fusion molecules, 931 and 934, which are the same except that 934 includes EGF, whereas 931 does not. The variable light and heavy chain regions of 14.1 (anti-CD89) and 3F2 (anti-HER2) were used to generate the constructs. Figure 58 shows a chemically conjugated Fab' anti-CD89 k-anti-HER2 bispecific molecule. The fab' fragments of 14.1 and 3F2 were linked by disulfide bond using standard chemical cross-linking procedures to generate this bispecific molecule.

The single chain bi- and tri-specific molecules (931 and 934) were produced by transfecting NSO cells with plasmids (e.g., pJZ934) (as shown in Figure 51 for anti-CD89 x EGF fusions), and selected in media containing G418. Cell lines were screened for the expression of fusion protein and subcloned by limited dilution cloning. Finally, fusion protein was purified by running supernatant of cell lines expressing fusion protein over a protein L column and then loading approximately 2 µg of purified protein onto a 4-15% tris-glycine gel under non-reducing and reducing conditions. The 934 construct purified predominantly as a trimer species, which dissociated to monomers under reducing conditions.

### Characterization of Bi- and Tri-Specific Molecule Binding to Tumor Cells

Flow Cytometry was used to characterize binding of the bi- and tri-specific molecules. Figure 59(A) shows binding to tumor cells of fusion proteins in transfectoma supernatants. Transfected (931 & 934) and untransfected (NSO) supernatant was incubated with either SKBR-3 or A431 tumor cell lines. Binding of fusion protein was then detected by staining with goat anti-human IgG fab-2 conjugated to phycoerythrin. As shown in Figure 59(B), supernatants from transfected cells (931 and 934) also mediated cell lysis (ADCC). In these studies, chromium release assays were done with an incubation period of 16-18 h and an effector (monocytes; PMN) to target (SKBR-3, A431) ration of 100:1. Tumor cell killing was detected using transfected (931 & 934) and untransfected (NSO) supernatant to mediate specific lysis.

Figure 60(A) shows binding activity of purified bi- and tri-specific molecules (931 and 934) to U937 cells (through the CD89 receptor). The fab-2 fragment of antibody 425 (anti-EGF-R mAb), which does not bind U937 cells, was used as a negative control. The anti-CD89 (Fab') x anti-HER2 (Fab') chemically coupled bispecific molecule (shown in Figure 58) was used as a positive control. Figure 60(B) is the same as Figure 60(A), except that binding to SKBR-3 cells (through the HER2 receptor) was tested. The fab-2 fragment of anti-CD89 antibody, 14.1, was used as a negative control. Figure 61 shows binding of the 934 trispecific construct to A431 cells. This experiment was done the same way as shown in Figure 60, except A431 tumor cells which over-express EGF-R were used. A fusion protein consisting of the sFv fragment of 14.1 fused to EGF was used as a positive control, and the fab-2 fragment of anti-CD89 antibody, 14.1, was used as a negative control.

### Effector Cell-Mediated Lysis of EGF-R and HER2/neu-Expressing Tumor Cells

Single chain bi- and tri-specific molecules (931 and 934) also were tested for their ability to mediate cell lysis (ADCC) of EGF-R and HER2/neu-expressing tumor cells in the presence of effector cells. Chromium release assays were done with an incubation period of 16-18 h and an effector (monocytes, PMN) to target (SKBR-3, A431) ration of 100:1. Tumor cell killing was detected using transfected (931 & 934) and untransfected (NSO) supernatant to mediate specific lysis. As shown in Figure 59(B), supernatants from transfected cells (931 and 934) mediated cell lysis (ADCC) of monocytes, PMNs and whole blood. In these studies, chromium release assays were done with an incubation period of 16-18 h and an effector (monocytes, PMN) to target (SKBR-3, A431) ration of 100:1. Tumor cell killing was detected using transfected (931 & 934) and untransfected (NSO) supernatant to mediate specific lysis.

Figure 62 and Figure 63 show effector cell-mediated lysis of SKBR-3 cells (Figure 62(A) and Figure 63(A)) and BT474 cells (Figure 62(B) and Figure 63(B)) by purified single chain bispecific construct, 931, in the presence of PMNs and monocytes, respectively. Chromium release assays were done with an incubation period of 16-18 h and an effector to target ration of 100:1. The fab-2 fragment of anti-CD89 antibody, 14.1, was used as a negative control in each experiment.

In addition, purified chemically conjugated bispecific molecule 14.1 × 3.F2 (Figure 58), containing two cross-linked Fab' antibody fragments (as opposed to single chain antibodies as in the 931 and 934 constructs), was tested for ADCC killing with polymorphonuclear (PMN) cells, monocytes and whole blood of ⁵¹Cr labeled SKBR-3 or BT-474 human tumor cells. After overnight incubation at 37 °C, the amount of tumor cell killing was determined by analysis of ⁵¹Cr released into the culture supernatants. Specific lysis was calculated as the amount of tumor cell lysis in the presence of the bispecific molecule - tumor cell lysis with polymorphonuclear cells alone. As shown in Figure 64, bispecific molecule 14.1 × 3.F2 mediated cell killing by PMNs of both SKBR-3 and BT-474 tumor cells expressing HER2/neu in a dose dependent fashion. In addition, as shown in Figure 65, bispecific molecule 14.1 × 3.F2 mediated cell killing by monocytes of both SKBR-3 and BT-474 tumor cells expressing HER2/neu in a dose dependent fashion. In both cases, addition of 10 µg/ml of 14.1 Fab', completely blocked ADCC of the tumor cells by 1 µg/ml of the bispecific molecule 14.1 × 3.F2, demonstrating that targeted cell killing was mediated exclusively by CD89 binding to the effector cells. Figure 66 shows that bispecific molecule 14.1 × 3.F2 also mediated cell killing by whole blood of BT-474 tumor cells expressing HER2/neu in a dose dependent fashion.

Overall, the foregoing studies describe the generation of bi- and trispecific molecules containing human monoclonal antibodies (including single chain and Fab' fragments). In addition, these examples demonstrate that these bi- and trispecific molecules effectively mediate killing of tumor cells expressing HER2/neu and EGF-R in the presence of effector cells.

## Claims

1. A bispecific molecule comprising a human antibody or fragment thereof which binds to an Fcα receptor, linked to EGF, wherein the heavy chain variable region of the human antibody, or fragment thereof, is derived from a human germline 30.3 VH gene and wherein the human light chain variable region of the human antibody, or fragment thereof, is derived from a human germline L18 Vk gene.

2. The bispecific molecule of claim 1, wherein the antibody, or fragment thereof, is a Fab fragment.

3. The bispecific molecule of claim 1, wherein the antibody, or fragment thereof, is a single chain antibody, or fragment thereof.

4. The bispecific molecule of claim 1, wherein the antibody, or fragment thereof, and EGF are genetically fused.

5. The bispecific molecule of claim 1, wherein the antibody, or fragment thereof, and EGF are covalently linked.

6. The bispecific molecule of claim 1, further comprising:
a third binding specificity for a HER2 receptor.

7. An in vitro method of inducing effector cell-mediated cell killing of a tumor cell **characterized by** over expression of EGF receptor (EGFR), comprising contacting the tumor cell with the molecule of any one of the preceding claims.

8. Use of the bispecific molecule of any one of claims 1 to 6 in the manufacture of a medicament for the treatment of cancer.

## Patentansprüche

1. Bispezifisches Molekül, welches einen humanen Antikörper oder ein Fragment davon umfaßt, der/das an einen mit EGF verknüpften Fcα-Rezeptor bindet, worin der variable Bereich der schwere Kette des humanen Antikörpers oder eines Fragments davon abgeleitet ist von einem humanen 30.3 VH-Keimbahngen, und worin der variable Bereich der humanen leichten Kette des humanen Antikörpers oder eines Fragments davon abgeleitet ist von einem humanen L 18 Vk-Keimbahngen.

2. Bispezifisches Molekül nach Anspruch 1, worin der Antikörper oder ein Fragment davon ein Fab-Fragment ist.

3. Bispezifisches Molekül nach Anspruch 1, worin der Antikörper oder ein Fragment davon ein Einzelketten-Antikörper oder ein Fragment davon ist.

4. Bispezifisches Molekül nach Anspruch 1, worin der Antikörper oder ein Fragment davon und EGF genetisch fusioniert sind.

5. Bispezifisches Molekül nach Anspruch 1, worin der Antikörper oder ein Fragment davon und EGF kovalent verknüpft sind.

6. Bispezifisches Molekül nach Anspruch 1, weiter umfassend: eine dritte Bindungsspezifität für einen HER2-Rezeptor.

7. In vitro-Verfahren zum Induzieren eines Effektorzellen-vermittelten Zell-Abtötens einer Tumorzelle, **gekennzeichnet durch** Überexpression von EGF-Rezeptor (EGFR), umfassend, Inkontaktbringen der Tumorzelle mit dem Molekül nach einem der vorhergehenden Ansprüche.

8. Verwendung des bispezifischen Moleküls nach einem der Ansprüche 1 bis 6 zur Herstellung eines Medikaments zur Behandlung von Krebs.

## Revendications

1. Molécule bispécifique comprenant un anticorps humain ou un fragment de ce dernier qui se lie à un récepteur Fcα lié au facteur EGF, dans laquelle la région variable de la chaîne lourde d'un anticorps humain ou d'un fragment de ce dernier dérive d'un gène VH de la lignée germinale humaine 30.3 et dans laquelle la région variable de la chaîne légère humaine de l'anticorps humain ou d'un fragment de ce dernier dérive d'un gène Vk de la lignée germinale humaine L18.

2. Molécule bispécifique selon la revendication 1, dans laquelle l'anticorps ou un fragment de ce dernier est un fragment Fab.

3. Molécule bispécifique selon la revendication 1, dans laquelle l'anticorps ou un fragment de ce dernier est un anticorps monocaténaire ou un fragment de ce dernier.

4. Molécule bispécifique selon la revendication 1, dans laquelle l'anticorps ou un fragment de ce dernier et le facteur EGF sont soumis à une fusion par voie génétique.

5. Molécule bispécifique selon la revendication 1, dans laquelle l'anticorps ou un fragment de ce dernier et le facteur EGF sont liés de manière covalente.

6. Molécule bispécifique selon la revendication 1, comprenant en outre :
une troisième spécificité de liaison pour un récepteur HER2.

7. Procédé in vitro pour la mise à mort cellulaire par l'intermédiaire de cellules effectrices d'une cellule tumorale, **caractérisé par** une surexpression du récepteur du facteur EGF (EGFR), comprenant la mise en contact de la cellule tumorale avec la molécule selon l'une quelconque des revendications précédentes.

8. Utilisation de la molécule bispécifique selon l'une quelconque des revendications 1 à 6, pour la préparation d'un médicament destiné au traitement du cancer.
